# EUROPÄISCHE PATENTSCHRIFT

(11) **EP 2 591 124 B1**
(45) Veröffentlichungstag und Bekanntmachung des Hinweises auf die Patenterteilung: **22.11.2017**
(21) Anmeldenummer: 11734008.3
(22) Anmeldetag: 04.07.2011
(51) Int. Cl.: C12Q 1/68

(54) **NACHWEIS VON MUTATIONEN, INSBESONDERE DELETIONEN ODER INSERTIONEN**
DETECTION OF MUTATIONS, IN PARTICULAR DELETIONS OR INSERTIONS
DÉTECTION DE MUTATIONS, EN PARTICULIER DE DÉLÉTIONS OU D'INSERTIONS

(30) Priorität: 11.12.2010 DE 102010054193; 09.07.2010 DE 102010026736
(43) Veröffentlichungstag der Anmeldung: 15.05.2013
(73) Patentinhaber: Universität Duisburg-Essen, 45141 Essen (DE)
(72) Erfinder: SCHULER, Martin, 45133 Essen (DE); BREITENBÜCHER, Frank, 45468 Mülheim (DE); HOFFARTH, Sandra, 45468 Mülheim (DE); STERGAR, Sarah-Luise, 45894 Gelsenkirchen (DE)
(74) Vertreter: Von Rohr Patentanwälte Partnerschaft mbB
(86) Internationale Anmeldenummer: PCT/EP2011/003297
(87) Internationale Veröffentlichungsnummer: WO 2012/003950

(56) Entgegenhaltungen:
- WO-A2-2010/060046
- WILLMORE-PAYNE CARLYNN ET AL: "The use of EGFR exon 19 and 21 unlabeled DNA probes to screen for activating mutations in non-small cell lung cancer", JOURNAL OF BIOMOLECULAR TECHNIQUES, ASSOCIATION OF BIOMOLECULAR RESOURCE FACILITIES, SANTA FE, NM, US, Bd. 19, Nr. 3, 1. Juli 2008 (2008-07-01), Seiten 217-224, XP002609844, ISSN: 1524-0215
- ERALI M ET AL: "High resolution melting applications for clinical laboratory medicine", EXPERIMENTAL AND MOLECULAR PATHOLOGY, ACADEMIC PRESS, US, Bd. 85, Nr. 1, 1. August 2008 (2008-08-01) , Seiten 50-58, XP022850522, ISSN: 0014-4800, DOI: 10.1016/J.YEXMP.2008.03.012 [gefunden am 2008-04-13]
- SANDRA HOFFARTH ET AL: "Hochsensitiver Nachweis von Mutationen des humanen EGF-Rezeptors bei Patienten mit nicht-kleinzelligem Lungenkarzinom: Individualisierte Therapie", MTA-DIALOG, HOPPENSTEDT, DARMSTADT, DE, Bd. 11, Nr. 1, 1. Januar 2010 (2010-01-01) , Seiten 26-29, XP009141148, ISSN: 1439-071X
- HITOSHI MIYAZAWA ET AL: "Peptide nucleic acid-locked nucleic acid polymerase chain reaction clamp-based detection test for gefitinib-refractory T790M epidermal growth factor receptor mutation", CANCER SCIENCE, Bd. 99, Nr. 3, 1. März 2008 (2008-03-01), Seiten 595-600, XP55007485, ISSN: 1347-9032, DOI: 10.1111/j.1349-7006.2007.00706.x
- TANAKA T ET AL: "Reliability of the peptide nucleic acid-locked nucleic acid polymerase chain reaction clamp-based test for epidermal growth factor receptor mutations integrated into the clinical practice for non-small cell lung cancers", CANCER SCIENCE, JAPANESE CANCER ASSOCIATION, TOKYO, JP, Bd. 98, Nr. 2, 1. Februar 2007 (2007-02-01), Seiten 246-252, XP002586235, ISSN: 1347-9032, DOI: 10.1111/J.1349-7006.2006.00377.X [gefunden am 2006-12-11]

## Beschreibung

Die vorliegende Erfindung betrifft den Bereich des Nachweises bzw. der Detektion von Genveränderungen, insbesondere Mutationen, in genomischer DNA, wobei die Genveränderung bzw. Mutation insbesondere im Zusammenhang mit einer Tumor- und/oder Krebserkrankung, wie einem Bronchialkarzinom, in Verbindung stehen kann. Auf Basis des Nachweises bzw. der Detektion der Genveränderung können Therapieansätze bzw. -maßnahmen zur gezielten Behandlung der Krebs- bzw. Tumorerkrankung zweckgerichtet optimiert werden. Wie nachfolgend geschildert, handelt es sich bei der Genveränderung insbesondere um eine Leserastermutation, vorzugsweise um eine Deletion bzw. Insertion.

Insbesondere betrifft die vorliegende Erfindung ein Verfahren zum Nachweis bzw. zur Detektion mindestens einer Genveränderung, insbesondere Mutation, in einem Gen, vorzugsweise in einem Gen, welches für ein mit einer Tumor- und/oder Krebserkrankung in Verbindung stehendes Protein kodiert, nach Anspruch 1.

Krebs- bzw. Tumorerkrankungen sind nach den Erkrankungen des Herz/Kreislauf-Systems die zweithäufigste Todesursache in Deutschland. Sofern eine rechtzeitige Therapie begonnen wird oder die Tumorerkrankung erst im hohen Lebensalter auftritt und mitunter nur langsam voranschreitet, ist nicht jede Krebserkrankung tödlich. Die derzeitige Heilungsrate bei allen Krebserkrankungen beträgt durchschnittlich 30 bis 40 %, wobei sich diesbezüglich jedoch in Abhängigkeit von der konkreten Krebserkrankung deutliche Unterschiede ergeben. So zählen zum Beispiel Krebserkrankungen der Atemwege, insbesondere der Lunge, zu den nur schlecht therapierbaren Krebserkrankungen.

Bei Krebserkrankungen bzw. bei Tumorzellen ist die Abstimmung von Wachstum, Teilung und Zerstörung bzw. Apoptose im Zellverband gestört bzw. außer Kraft gesetzt. Oftmals werden körpereigene regulierende Signale nicht erkannt oder nicht bzw. fehlerhaft ausgeführt, was oftmals ursächlich auf genetische Defekte bzw. Genveränderungen, wie Mutationen, zurückzuführen ist. So können genetische Veränderungen, wie Mutationen, zu Veränderungen in der Struktur und in der Physiologie von durch die betroffenen Gene kodierten Proteinen führen, was ein Tumorwachstum induzieren bzw. fördern kann. So kann die Krebsentstehung bzw. Karzinogenese, insbesondere das primäre Krankheitsereignis, auf Veränderungen des Erbgutes beruhen, welche durch körpereigene Überwachungs- und Korrektursysteme nicht kompensiert werden und folglich beispielsweise im Rahmen von Zellteilungsprozessen auf nachfolgende Zellen übertragen werden können, was mitunter zu der Entstehung eines Primärtumors führt.

Lungenkarzinome, welche synonym auch als Bronchialkarzinome, bronchogene Karzinome bzw. Lungenkrebs bezeichnet werden, stellen eine bösartige Tumorerkrankung auf Basis entarteter Zellen, insbesondere der Bronchien oder der Bronchiolen, dar. Das Bronchial- bzw. Lungenkarzinom ist eine der häufigsten bösartigen Krebserkrankungen des Menschen und stellt eine der häufigsten durch Krebs bedingten Todesursachen in der westlichen Welt dar. So liegt die Zahl der Neuerkrankungen an Lungenkrebs in Deutschland bei etwa 50.000 Personen pro Jahr. Hauptursache für Lungenkrebserkrankungen ist das inhalative Rauchen. Daneben gibt es einige toxische Substanzen, wie Asbest oder Chrom, welche gleichermaßen Lungenkarzinome induzieren können. Aufgrund der in frühen Erkrankungsphasen mitunter ganz fehlenden oder nur unspezifischen Symptome werden die meisten Erstdiagnosen eines Lungenkarzinoms erst in späteren Stadien der Erkrankung gestellt, so dass eine der erfolgversprechendsten Therapieoptionen - eine vollständige operative Entfernung des Tumors - oftmals nicht mehr realisierbar ist, insbesondere auch aufgrund einer bereits voranschreitenden Metastasierung. Die Heilungsrate des Bronchialkarzinoms ist im Allgemeinen sehr schlecht und liegt bei einer Fünfjahresüberlebensrate von unter 10 %; die Überlebenswahrscheinlichkeit nach zwei Jahren liegt unter 20 %.

Etwa ein Viertel aller bösartigen Tumore bzw. Malignome sind Bronchialkarzinome. Beim Mann ist das Bronchialkarzinom weltweit die häufigste Tumorerkrankung, in Deutschland ist sie die dritthäufigste nach dem Prostatakarzinom und dem kolorektalen Karzinom, jedoch liegt das Bronchialkarzinom als Ursache von Krebssterbefällen auf dem ersten Platz.

Aufgrund ihrer Histologie und des Krankheitsverlaufes werden Lungenkarzinome im Allgemeinen in zwei Gruppen unterteilt, nämlich in ein kleinzelliges Lungenkarzinom (*Small Cell Lung Cancer* bzw. SCLC) einerseits und ein nichtkleinzelliges Lungenkarzinom (*Non Small Cell Lung Cancer* bzw. NSCLC) andererseits. Das nichtkleinzellige Lungenkarzinom bzw. NSCLC stellt mit einer Häufigkeit von 85 % der Lungenkrebserkrankungen die größte Gruppe der Bronchialkarzinome dar. In Abhängigkeit von dem histologischen Befund kann das nichtkleinzellige Karzinom bzw. NSCLC in ein mitunter spindelzellig ausgebildetes Plattenepithelkarzinom, ein Adenokarzinom sowie in ein großzelliges Karzinom bzw. Riesenzellkarzinom differenziert werden.

Was die im Stand der Technik bekannten therapeutischen Ansätze zur Therapie des Lungenkarzinoms, insbesondere des kleinzelligen Lungenkarzinoms, anbelangt, so fokussieren diese maßgeblich auf einen therapeutischen Ansatz auf Basis einer Chemo- bzw. Radiotherapie. Derartige Therapien sind jedoch mit starken Nebenwirkungen verbunden und führen oftmals nicht zum gewünschten therapeutischen Erfolg. Auch platinbasierte Kombinationstherapien erreichen in Studien nur eine mediale Überlebensverlängerung von lediglich zehn bis zwölf Monaten. Seit kurzer Zeit werden für Patienten mit einem diagnostizierten kleinzelligen Lungenkarzinom bzw. NSCLC Therapiealternativen zu der üblichen Behandlung mit Chemotherapeutika bereitgestellt. So werden Medikamente eingesetzt, die - im Gegensatz zu Zytostatika - spezifisch auf Tumorzellen wirken und, damit verbunden, auch deutlich weniger Nebenwirkungen aufweisen. Hierzu zählen insbesondere die unter den internationalen Freinamen Gefitinib, Erlotinib sowie Cetuximab erhältlichen Substanzen, welche spezifisch an den häufig in Lungenkrebserkrankungen involvierten Rezeptor des Proliferationsfaktors EGF (*Epidermal Growth Factor*)*,* nämlich den so genannten EGF-Rezeptor bzw. EGFR, binden bzw. diesen inaktivieren.

Der EGF-Rezeptor (*Epidermal Growth Factor-*Rezeptor) ist ein Mitglied der ErbB-Familie mit einer Unterfamilie von vier engverwandten Rezeptor-Tyrosinkinasen. Der EGF-Rezeptor wird oftmals synonym auch als HER1, EGFR1 bzw. ErbB-1 bezeichnet.

Bei dem EGF-Rezeptor handelt es sich um einen Transmembranrezeptor mit intrinsischer Tyrosinkinase-Aktivität, der in allen Zellarten vorkommt. Der Rezeptor weist einen Membrandurchgang und im zytoplasmatischen Teil eine Kinase-Domäne mit ATP-Bindungsstelle auf. Der EGF-Rezeptor gehört zu den Rezeptoren für Wachstumsfaktoren.

In nichtmalignen Zellen wird der Rezeptor nach Bindung seines Liganden (EGF) durch Dimerisierung und Phosphorylierung aktiviert und vermittelt in der Folge Wachstums- und Überlebenssignale ins Zellinnere. Die Aktivierung des Rezeptors führt letztendlich zu einer Stimulation des Zellwachstums und zu einer Verhinderung von Apoptose bzw. des programmierten Zelltodes. Der EGF-Rezeptor unterstützt die Proliferation und das zelluläre Überleben.

Eine Überexpression und bestimmte Mutationen im EGF-Rezeptor - wie sie mitunter in Tumorzellen beobachtet werden können - vermitteln jedoch eine dauerhafte bzw. übermäßige Aktivierung des Rezeptors, was mit einem unerwünscht hohen Zellwachstum, einer übermäßigen Zellteilung und somit einer Tumorentstehung bzw. einem Tumorwachstum einhergeht. Für maligne Zellen ist eine stete Vermittlung von Wachstumssignalen von Vorteil, da sie die beschleunigte Proliferation und das Überleben der malignen Zellen bewirken bzw. unterstützen. Tumorzellen, die eine Überexpression bzw. aktivierende Mutationen in Bezug auf den EGF-Rezeptor aufweisen, sind sogar von der dauerhaften bzw. übermäßigen Aktivierung des EGF-Rezeptors bezüglich ihrer Proliferation und ihres Überlebens abhängig. Der EGF-Rezeptor wird somit in verschiedenen Tumorarten hochreguliert bzw. in mutierter Form vorgefunden, was dazu führt, dass die betreffenden Tumorzellen unkontrolliert wachsen und sich vermehren. Die zuvor genannten Wirksubstanzen zielen darauf ab, das onkogene Signal des EGF-Rezeptors zu blockieren und somit das Tumorwachstum zu unterbinden bzw. zu verlangsamen.

Denn der EGF-Rezeptor kann im unmittelbaren Zusammenhang mit einer Tumor- bzw. Krebserkrankung, insbesondere einem Lungen- bzw. Bronchialkarzinom, wie dem kleinzelligen Lungenkarzinom, stehen, insbesondere da der EGF-Rezeptor in seiner mutierten Form zu einem unkontrollierten Wachstum und einer Vermehrung von Tumorzellen führt. Eine spezifische Blockierung bzw. Inaktivierung des insbesondere mutierten EGF-Rezeptors kann somit zu einer Eingrenzung bzw. Unterbindung des Wachstums von Tumorzellen führen.

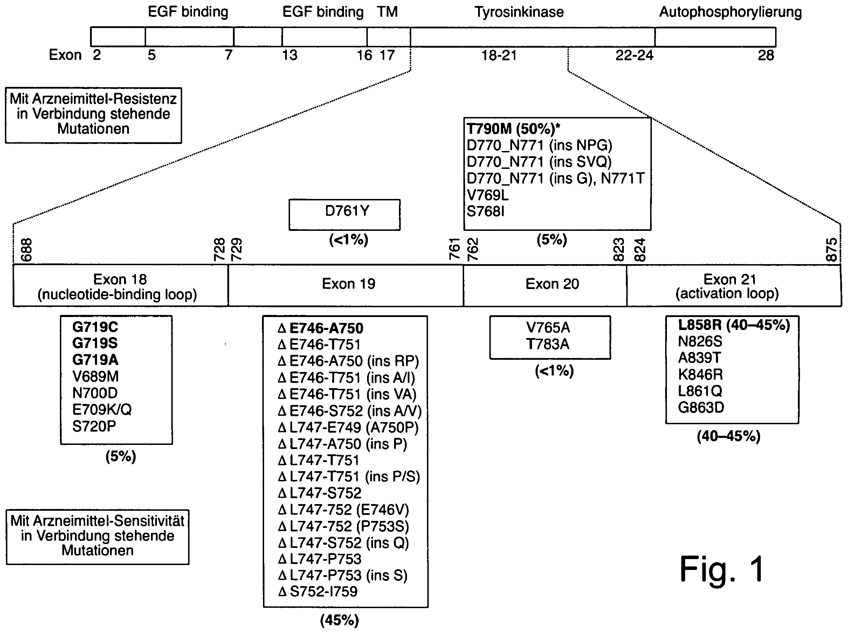

Im Rahmen der vorliegenden Erfindung ist es von Bedeutung, dass durch die zweckgerichtete Inhibition des EGF-Rezeptors die Aktivierung des Rezeptors vermindert bzw. gehemmt werden kann. Ein großer Teil der Mutationen des EGF-Rezeptors bei Patienten mit kleinzelligem Lungenkarzinom bzw. NSCLC basieren auf verschiedenen Deletionen in Exon 19 des EGF-Rezeptors, wie z. B. der Deletion ΔE746-A750 (d. h. Wegfall der Aminosäuren an Position 746 (Glutaminsäure) bis Position 750 (Alanin) in der Aminosäuresequenz des EGF-Rezeptors), sowie auf einer Punktmutation in Exon 21, nämlich der L858R-Mutation (d. h. Austausch der Aminosäure Leucin L an Position 858 in der Aminosäuresequenz des EGF-Rezeptors durch die Aminosäure Arginin R). In Bezug auf Exon 19 des EGF-Rezeptors existiert insgesamt eine große Anzahl an Deletionen, welche für sich zwar gemein haben, dass sie im Bereich der Aminosäureposition 746 bzw. 747 in der Aminosäuresequenz des EGF-Rezeptors liegen, sich jedoch hinsichtlich ihrer konkreten Ausbildung, insbesondere im Hinblick auf die konkrete Veränderung der Aminosäuresequenz, unterscheiden (vgl. auch Übersichtsdarstellung gemäß Fig. 1). Von daher können derartige Mutationen auch nur schwer bzw. unter großem verfahrenstechnischen Aufwand nachgewiesen werden.

Patienten mit einem Lungentumor, die eine dieser Veränderungen aufweisen, eignen sich besonders für eine Therapie mit EGF-Rezeptor-Inhibitoren. Insbesondere verfügen die Medikamente bzw. Substanzen Gefitinib und Erlotinib über eine hohe Wirkspezifität in Bezug auf EGF-Rezeptoren, welche derartige Mutationen aufweisen. Die Therapie mit spezifischen Inhibitoren des EGF-Rezeptors, insbesondere in Bezug auf seine mutierte Form, ist im Allgemeinen gut verträglich und weist auch eine gewisse Wirksamkeit auf. Aufgrund der hohen Spezifität werden selektiv die mutationstragenden Rezeptoren inhibiert, was Nebenwirkungen verringert und den Therapieeffekt vergrößert.

Die meisten Patienten entwickeln nach einer gewissen Zeit eine Sekundärmutation, die zusätzlich zu der bereits vorhandenen Mutation auftritt und mitunter zu einer Resistenz gegenüber Erlotinib und Gefitinib führt. In etwa 65 % dieser Fälle findet man eine Mutation in Exon 20 des EGF-Rezeptors vor, wobei es sich hierbei um eine T790M-Mutation (d. h. Austausch der Aminosäure Threonin T durch Methionin M an Position 790 des EGF-Rezeptors) handelt. Für solche Patienten stehen Medikamente zur Verfügung, deren Wirkmechanismus und Spezifität sich von denjenigen Medikamenten der ersten Generation, wie Erlotinib und Gefitinib, unterscheiden. Die Inhibitoren der zweiten Generation binden insbesondere irreversibel an den Rezeptor und nicht reversibel, wie es bei den in Rede stehenden Medikamenten der ersten Generation der Fall ist. Patienten mit einem kleinzelligen Lungenkarzinom, die aufgrund der Sekundärmutation, insbesondere der T790M-Mutation, nicht mehr auf Medikamente der ersten Generation ansprechen, können folglich mit einem EGF-Rezeptor-Inhibitor der zweiten Generation weiterbehandelt werden. Diese Inhibitoren sind ebenfalls hochspezifisch und wirksam, so dass das Wachstum und Überleben der Tumorzellen verlangsamt oder verhindert werden kann.

Vor diesem technischen bzw. medizinischen Hintergrund kommt somit einer schnellen, einfach zu handhabenden und zu exakten Ergebnissen führenden Mutationsanalyse in Bezug auf den EGF-Rezeptor bei Patienten mit einem Lungenkarzinom eine herausragende Bedeutung zu, insbesondere auch vor dem Hintergrund, den Therapieablauf im Hinblick auf den spezifischen Mutationsbefund abzustimmen bzw. zu optimieren.

Insbesondere ist es zur Gewährleistung eines optimalen therapeutischen Ansatzes mittels einer hocheffektiven und individualisierten Medizin notwendig, das Tumorgewebe auf den Status des EGF-Rezeptors, insbesondere im Hinblick auf etwaige vorliegende Mutationen, insbesondere wie zuvor angeführt, zu untersuchen. Auf dieser Basis kann bei den jeweiligen Patienten in Abhängigkeit von ihrem Mutationsstatus eine Behandlung mit den entsprechenden EGF-Rezeptor-Inhibitoren durchgeführt werden.

Auf Basis einer aussagekräftigen Mutationsanalyse in Bezug auf den EGF-Rezeptor kann somit eine diesbezügliche zielgerichtete Therapie mit den jeweiligen Medikamenten realisiert werden.

Im Stand der Technik stehen zur Detektion von Mutationen in genomischer DNA aus Tumorgewebe verschiedene molekularbiologische Methoden bzw. Ansätze zur Verfügung. Standardmäßig wird zum Beispiel die Sequenzierung nach Sanger eingesetzt. Dieses Verfahren weist jedoch den Nachteil auf, dass Mutationen nur nachgewiesen werden können, wenn die sie tragende DNA mindestens mit einem Anteil von 20 % bis 25 % in der zu analysierenden Probe, bezogen auf den Gesamt-DNA-Gehalt der Probe, vorliegt. Zudem ist der Zeitaufwand für die Durchführung und Auswertung relativ hoch, da die Versuchsdurchführung mehrere Stunden betragen kann.

Ein weiteres Verfahren des Standes der Technik zur Analyse von Mutationen besteht in der Polymerase-Kettenreaktion bzw. *Polymerase Chain Reaction* bzw. PCR, wie beispielsweise der *Real-Time-PCR* bzw. RT-PCR. Durch den Einsatz dieser Methode kann die Analysedauer verringert werden. Zudem ist die Durchführung relativ kostengünstig und die Sensitivität gegenüber der zu detektierenden bzw. zu analysierenden Mutation bereits erhöht. Dennoch sind die mittels herkömmlicher PCR erhaltenen Ergebnisse nicht immer zufriedenstellend, insbesondere sofern die Probe nur äußerst geringe Mengen an Mutationsmaterial aufweist. Die herkömmliche PCR weist im Ergebnis nur eine geringe Sensitivität auf.

Weiterhin ist es bei den Verfahren des Standes der Technik von Nachteil, dass die zu untersuchenden Mutationen als solche bekannt sein müssen. Im Stand der Technik wird das grundlegende Prinzip realisiert, wonach für die jeweils zu untersuchende bzw. nachzuweisende Mutation spezifische Sonden eingesetzt werden, anhand derer ausschließlich eine konkrete Mutation, beispielsweise in Form einer speziellen Punktmutation, nachgewiesen werden kann. Der Einsatz mutationsspezifischer Sonden ist insbesondere im Hinblick auf die Erfassung von Deletionen bzw. Insertionen nicht immer optimal, insbesondere sofern diese an vergleichbarer Position und somit gewissermaßen nach Art einer Gruppe in einem Protein vorkommen und unterschiedliche Auswirkungen auf die Aminosäuresequenz zeigen.

So betrifft die WO 2010/060046 A2 ein Verfahren zur Detektion, Identifizierung und zum Screening von Einzelnukleotid-Polymorphismen, Insertionen, Deletionen und Mikrosatelliten-DNA. Bei dem Verfahren wird zunächst die auf die Mutation zu untersuchende einzelsträngige Nukleinsäuresequenz generiert, welcher zum einen ein unspezifisch in doppelsträngige DNA interkalierender Farbstoff als Donor und zum anderen eine mit dem entsprechend komplementären Fluorophor markierte und zu dem zu untersuchenden Nukleinsäuremolekül zumindest im Wesentlichen komplementäre Sonde zugesetzt wird, so dass ein doppelsträngiges Nukleinsäuremolekül auf Basis von Probe und Sonde resultiert. Anschließend wird die doppelsträngige DNA mit Licht mit einer spezifischen Wellenlänge bestrahlt, um ein FRET-Signal zu erzeugen, wobei die Fluoreszenzemission gemessen wird. Abschließend wird eine Schmelzkurvenanalyse durchgeführt, um die zuvor ausgeführten Genveränderungen nachzuweisen.

Weiterhin betrifft die Publikation von Willmore-Payne et al., "The Use of EGFR Exon 19 and 21 Unlabeled DNA Probe to Screen for Activating Mutations in Non-Small Cell Lung Cancer", Journal of Biomolecular Techniques, Volume 19, Seiten 217 bis 224 ein Verfahren, mit welchem Mutationen in Exon 19 bzw. Exon 21 des für den *epidermal growth factor-Rezeptor* codierenden Gens detektiert werden sollen. Dazu werden unmarkierte DNA-Proben aus Tumorzellen von Lungenkrebspatienten einer Polymerasekettenreaktion zur Amplifizierung des zu untersuchenden DNA-Abschnitts von Exon 19 bzw. Exon 21 des EGFR-Gens unterzogen und mit einer Sonde hybridisiert. Zur Detektion der Mutationen wird anschließend eine Schmelzkurvenanalyse durchgeführt.

In der wissenschaftlichen Publikation Erali et al., "High revolution melting applications for clinical laboratory medicine", Experimental and Molecular Pathology, 2008, Volume 85, Seiten 50 bis 58*,* wird die molekularbiologische Standardmethode der Schmelzkurvenanalyse von doppelsträngiger DNA beschrieben.

Die wissenschaftliche Publikation gemäß Hoffarth et al., "Hochsensitiver Nachweis von Mutationen des humanen EGF-Rezeptors bei Patienten mit nicht-kleinzelligem Lungenkarzinom - Individualisierte Therapie ", MTA Dialog, 2010 fokussiert auf den Nachweis von Mutationen in dem für den EGF-Rezeptor codierenden Gen. Dabei wird zu Nachweiszwecken eine für den mutierten DNA-Strang spezifische Sensorsonde eingesetzt. Die beschriebene Nachweismethode erfordert somit, zur Detektion verschiedener Mutationen auch verschiedene bzw. jeweils spezielle, auf die zu detektierende Mutation abgestimmte Sensorsonden einzusetzen.

Vor diesem technischen Hintergrund besteht nunmehr die Aufgabe der vorliegenden Erfindung darin, ein Verfahren zum Nachweis bzw. zur Detektion von Genveränderungen, insbesondere Mutationen, bereitzustellen, welches die zuvor geschilderten Nachteile des Standes der Technik zumindest teilweise vermeidet oder aber wenigstens abschwächt, wobei das erfindungsgemäße Verfahren vorrangig auf die Erfassung spezieller Mutationen in Form von Deletionen bzw. Insertionen fokussieren soll.

Dabei soll insbesondere ein Verfahren bereitgestellt werden, mit welchem in einfacher Weise eine Vielzahl verschiedenartiger Genveränderungen unter Durchführung möglichst weniger Verfahrensschritte bzw. -Wiederholungen nachgewiesen werden kann.

Insbesondere soll zudem ein Verfahren bereitgestellt werden, welches eine sehr hohe Sensitivität aufweist, d. h. bereits bei sehr geringen Mengen an Mutationsmaterial in einer Probe bzw. einem zu analysierenden Material zu aussagekräftigen Ergebnissen führt.

Zudem soll das erfindungsgemäße Verfahren an einer Vielzahl verschiedenartiger Proben bzw. Patientenmaterialien, wie beispielsweise Blutproben, Lymphflüssigkeit, Zellen, aufgereinigter DNA oder dergleichen, eingesetzt werden können.

Das erfindungsgemäße Verfahren soll zudem eine fundierte Aussage im Hinblick auf einen sozusagen mutationsabhängigen bzw. -spezifischen therapeutischen Ansatz ermöglichen, um auf diese Weise das zugrundeliegende Behandlungsschema, insbesondere im Hinblick auf die Auswahl spezieller Medikamente, zu optimieren.

Insbesondere soll das erfindungsgemäß vorgeschlagene Verfahren zur Detektion bzw. Analyse von Mutationen in Proteinen, insbesondere dem EGF-Rezeptor, geeignet sein, wobei die Mutation bzw. das Protein mit der Mutation im Zusammenhang mit der Entwicklung bzw. dem Auftreten von Lungenkarzinomen, insbesondere des kleinzelligen Lungenkarzinoms bzw. NSCLC, steht.

Zur Lösung der zuvor geschilderten Aufgabenstellung schlägt die vorliegende Erfindung gemäß dem ersten und einzigen Erfindungsaspekt ein Verfahren zum Nachweis mindestens einer Mutation in einem Gen, vorzugsweise in einem Gen, welches für ein mit einer Tumor- und/oder Krebserkrankung in Verbindung stehendes Protein kodiert, insbesondere wobei das die Genveränderung aufweisende Gen (Mutationsgen) zusammen mit weiteren, für das Protein kodierenden, jedoch keine Genveränderung aufweisenden Genen (Wildtypgenen) vorliegen kann, gemäß Anspruch 1 vor. Weitere, vorteilhafte Ausgestaltungen dieses Erfindungsaspekts sind Gegenstand der diesbezüglichen Unteransprüche.

Es versteht sich von selbst, dass Ausgestaltungen, Ausführungsformen, Vorteile und dergleichen, welche nachfolgend zu Zwecken der Vermeidung von Wiederholungen nur zu einem Erfindungsaspekt aufgeführt sind, selbstverständlich auch in Bezug auf die übrigen Erfindungsaspekte entsprechend gelten.

Ein erster Gegenstand der vorliegenden Erfindung - gemäß einem **ersten** Aspekt der vorliegenden Erfindung - ist somit ein Verfahren zur Detektion mindestens einer Mutation in einem Gen, vorzugsweise in einem Gen, welches für ein mit einer Tumor- und/oder Krebserkrankung in Verbindung stehendes Protein kodiert, wobei das die Mutation aufweisende Gen (Mutationsgen) zusammen mit weiteren, für das Protein kodierenden, jedoch keine Mutation aufweisenden Genen (Wildtypgenen) vorliegt,
wobei das Verfahren mittels einer asymmetrischen Polymerase-Kettenreaktion (*Polymerase Chain Reaction* bzw. PCR) unter Verwendung mindestens einer detektierbaren Hybridisierungssonde (Sensorsonde), welche an dem keine Mutation aufweisenden Gen (Wildtypgen) und dem die Mutation aufweisenden Gen (Mutationsgen) zu binden imstande ist, mindestens einer hiervon verschiedenen zweiten Hybridisierungssonde (Ankersonde) und mindestens eines die Anbindung der Sensorsonde an die Wildtypgene inhibierenden wildtypspezifischen Blockierungsmittels durchgeführt wird und wobei zum Nachweis und/oder zur Detektion der Mutation im Anschluss an die Polymerase-Kettenreaktion eine Schmelzkurve aufgenommen wird und/oder eine Schmelzkurvenanalyse durchgeführt wird, wobei die Abspaltung und/oder Dehybridisierung der Sensorsonde von dem jeweiligen DNA-Einzelstrang des Mutationsgens und/oder des Wildtypgens erfasst wird und wobei über den oder die Schmelzpunkte und/oder Schmelzpunktbereiche der Schmelzkurve auf das Vorliegen einer Mutation geschlossen wird und im Falle des Vorliegens einer Mutation die Art der Mutation bestimmt wird,
wobei die Hybridisierungssonde (Sensorsonde) eine höhere Spezifität und/oder Bindungsaffinität und/oder Selektivität zu dem keine Mutation aufweisenden Gen (Wildtypgen) als zu dem die Mutation aufweisenden Gen (Mutationsgen) aufweist, wobei die Hybridisierungssonde (Sensorsonde) vollständig an dem DNA-Einzelstrang (wt-Sondenstrang) des Wildtypgens zu binden imstande ist und wobei die Hybridisierungssonde (Sensorsonde) an der Position der Mutation an dem die Mutation aufweisenden DNA-Einzelstrang (mt-Sondenstrang) des Mutationsgens nicht zu binden imstande ist,
wobei die zweite Hybridisierungssonde (Ankersonde) auf demselben DNA-Einzelstrang wie die Hybridisierungssonde (Sensorsonde) zu binden imstande ist und wobei die Hybridisierungssonde (Sensorsonde) einerseits und die zweite Hybridisierungssonde (Ankersonde) andererseits ein FRET-Paar auszubilden imstande sind,
wobei als Blockierungsmittel ein Nukleinsäure-Analogon in Form einer *Locked Nucleic Acid* (LNA) eingesetzt wird und wobei das Blockierungsmittel ausgewählt wird derart, dass das Blockierungsmittel eine höhere Spezifität und/oder Bindungsaffinität und/oder Selektivität in Bezug auf den Bereich des DNA-Einzelstrang des Wildtypgens, welcher zu dem die Mutation aufweisenden Genabschnitt des Mutationsgens korrespondiert, gegenüber dem entsprechenden Mutationsgen aufweist, und
wobei die asymmetrische Polymerase-Kettenreaktion in Gegenwart von Primern durchgeführt wird, wobei ein erster Primer spezifisch an dem DNA-Einzelstrang des Mutationsgens (mt-Sondenstrang) bindet, an welchen die Sensorsonde bindet, und wobei ein zweiter Primer spezifisch an dem zum Sondenstrang komplementären DNA-Einzelstrang des Mutationsgens (mt-Komplementärstrang) bindet, und wobei die Konzentration des ersten Primers größer ist als die Konzentration des zweiten Primers.

Die vorliegende Erfindung basiert somit auf dem Prinzip, wonach als Hybridisierungssonde bzw. Sensorsonde eine wildtypspezifische Hybridisierungssonde eingesetzt wird, welche eine verringerte Spezifität bzw. Affinität, insbesondere Bindungsaffinität, zu dem die Genveränderung aufweisenden Gen bzw. Mutationsgen aufweist, und zwar bedingt durch eine Nichtbindung der Sensorsonde an der Position bzw. Stelle der Mutation. Erfindungsgemäß wird somit eine spezielle Sensorsonde auf Basis einer Nukleotidsequenz eingesetzt, welche stärker mit dem entsprechenden DNA-Einzelstrang des Wildtypgens hybridisiert als mit dem entsprechenden DNA-Einzelstrang des Mutationsgens, und zwar insbesondere durch eine Bindung der Sensorsonde auch in dem zu der Mutation korrespondierenden Stelle im DNA-Einzelstrang des Wildtypgens (d. h. auch an den Nukleotiden bzw. Basen der Wildtypsequenz, welche sich an der entsprechenden Stelle bzw. Position befinden wie die zu detektierende Mutation). Dabei ist die Sensorsonde, wie nachfolgend noch angeführt, erfindungsgemäß insbesondere derart konzipiert, dass diese hinsichtlich ihrer Länge bzw. Anzahl an Nukleotiden bzw. Basen den Bereich der Mutation und einen diesbezüglich angrenzenden Nukleotidbereich im Gen umfasst.

Durch dieses der vorliegenden Erfindung zugrundeliegende Prinzip ist es möglich, eine Vielzahl an in vergleichbarer Position bzw. Stelle im Gen vorkommenden Genveränderungen mit ein und derselben Sensorsonde zu detektieren, was eine enorme verfahrenstechnische Vereinfachung darstellt, wobei das erfindungsgemäße Verfahren insbesondere zu einer einfachen Detektion von Mutationen in Form von Deletionen bzw. Insertionen und somit von Leserastermutationen an bestimmten Stellen im Gen führt. So ist es z. B. und in nicht beschränkender Weise auf Basis des erfindungsgemäßen Verfahrens unter Einsatz eines einzigen Sensorsondentyps möglich, die beispielhaft in Fig. 1 dargestellten Deletionen in Exon 19 des EGF-Rezeptors zu detektieren. Somit ist es erfindungsgemäß möglich, unter Verwendung eines einzigen Sensorsondentyps verschiedene Deletionen in Exon 19 des humanen EGF-Rezeptors zu erfassen, wobei die Genveränderung bzw. Deletionen bzw. Insertionen unterschiedliche Größen bzw. leicht unterschiedliche Positionierungen innerhalb des Gens aufweisen können.

In diesem Zusammenhang können erfindungsgemäß beispielsweise solche Deletionen erfasst werden, welche zum Wegfall von einer, zwei, drei oder mehr Aminosäuren in der Aminosäuresequenz des entsprechenden Proteins führen. In Bezug auf die detektierbaren Mutationen, insbesondere Deletionen, können somit auch solche Veränderungen erfasst werden, die den Wegfall bzw. die Deletion von mehreren Aminosäuren zur Folge haben können. In diesem Zusammenhang können beispielsweise und im Allgemeinen im Rahmen der vorliegenden Erfindung Deletionen erfasst werden, welche auf den Wegfall von drei bis fünfzehn oder mehr Nukleotiden in der für ein Protein kodierenden Gensequenz aufweisen. Dabei kann der Wegfall vollständiger Codons oder einzelner Nukleotide bzw. Basen (z. B. von einer oder zwei Basen) detektiert werden. Zudem können solche Genveränderungen, insbesondere Deletionen, mit ein und demselben Sensortyp erfasst werden, welche an zwei oder mehr aufeinanderfolgenden Positionen hinsichtlich der Aminosäuresequenz des betreffenden Proteins vorliegen. Dazu zählen zum Beispiel solche Deletionen, welche an der Aminosäureposition 746 und/oder 747 des EGF-Rezeptors auftreten bzw. beginnen.

Dabei kann die erfindungsgemäß einsetzte Sensorsonde derart sein, dass die Sonde in dem Bereich vor und/oder nach der eigentlichen Mutation mit dem Gen bzw. mit dem betreffenden DNA-Einzelstrang interagiert bzw. hybridisiert, während im Bereich der Mutation keine Hybridisierung in Bezug auf den DNA-Einzelstrang mit der Mutation vorliegt. Demgegenüber kann der erfindungsgemäß eingesetzte Sensorsondentyp, wie nachfolgend noch geschildert, zumindest im Wesentlichen vollständig mit dem nichtmutierten Gen bzw. dem betreffenden DNA-Einzelstrang ohne Mutation hybridisieren. Als Folge weisen die jeweiligen Komplexe unterschiedliche Ablöse- bzw. Schmelztemperaturen auf, wobei sich die nicht vollständig an den DNA-Einzelstrang mit der Mutation hybridisierte Sonde bei niedrigeren Temperaturen als die zumindest im wesentlichen vollständig an den DNA-Einzelstrang des Wildtypgens hybridisierte Sensorsonde löst, was über eine Veränderung eine sondenpezifischen detektierbaren Signals messtechnisch erfasst werden kann, wie nachfolgend noch geschildert.

Darüber hinaus eignet sich das erfindungsgemäße Verfahren auch zur Detektion von Punktmutationen, wie z. B. der zuvor beschriebenen T790M-Mutation in Exon 20 des EGF-Rezeptors, so dass erfindungsgemäß gewissermaßen durch die jeweilige Maßschneiderung der Sensorsonde ein universell einsetzbares Verfahren zur Erfassung von Mutationen als solchen bereitgestellt wird.

Wie im Nachfolgenden noch ausführlich angeführt, wird das erfindungsgemäße Verfahren unter Durchführung einer asymmetrischen Polymerase-Kettenreaktion in kombinierter Verwendung mit einem speziellen Blockierungsmittel, welches die Anbindung der erfindungsgemäß eingesetzten Sensorsonde an den DNA-Einzelstrang des Wildtyps verhindert bzw. zumindest verringert, durchgeführt, was zu einer deutlichen Verbesserung der Sensitivität des erfindungsgemäßen Verfahrens führt.

Auf dieser Basis ist eine weitere grundlegende Idee der vorliegenden Erfindung somit darin zu sehen, dass aufgrund der spezifischen Konzeption des erfindungsgemäßen Verfahrens Genveränderungen bzw. Mutationen in einer Probe bzw. einem Ensemble von Genen mit sehr hoher Sensitivität erfasst werden können, so dass bereits kleinste Mengen an mutierter DNA, insbesondere in einem Gemisch bzw. Ensemble mit Wildtyp-DNA bzw. keine Mutation aufweisender DNA, analysiert werden können. Im Rahmen der vorliegenden Erfindung ist es somit gelungen, bereits geringe Mengen bzw. Gehalte an DNA, welche die Genveränderung bzw. Mutation aufweisen, in einer Probe mit hoher Zuverlässigkeit zu analysieren und zwar insbesondere auch insofern, als eine Vielzahl verschiedenartiger Mutationen erfasst werden kann.

In diesem Zusammenhang ist es erfindungsgemäß möglich, bereits geringe Mengen an mutierter DNA ab einem Gehalt von etwa 0,0025 % in einem Gemisch mit sonstiger DNA bzw. nichtmutierter DNA bzw. Wildtyp-DNA, bezogen auf den DNA-Gehalt, zu detektieren.

In diesem Zusammenhang besteht somit eine weitere grundlegende Idee der vorliegenden Erfindung darin, die Effektivität bzw. Sensitivität des Verfahrens nach der Erfindung dadurch gezielt zu erhöhen bzw. zu verbessern, dass in zweckgerichteter Weise eine asymmetrische Polymerase-Kettenreaktion durchgeführt wird. Dabei erfolgt vorrangig bzw. selektiv eine Vermehrung bzw. Amplifizierung des DNA-Einzelstranges insbesondere auch des Mutationsgens, welcher die Mutation aufweist und an welchem die zur eigentlichen Detektion im Sinne einer messtechnischen Erfassung dienende Sensorsonde mit geringerer Affinität als zu dem Einzelstrang des Wildtypgens bindet. Dies kann beispielsweise, wie nachfolgend noch ausführlich angeführt, dadurch realisiert werden, dass unterschiedliche Mengen an Primer, welche synonym auch als (PCR-)Startermoleküle bezeichnet werden, eingesetzt werden, wobei im Rahmen der vorliegenden Erfindung insbesondere die Menge desjenigen Primers erhöht wird, welcher an dem so genannten Sondenstrang insbesondere mit der Mutation bindet. Hierdurch wird erfindungsgemäß erreicht, dass insbesondere die Menge an DNA-Einzelsträngen mit der Mutation im Vergleich zu den übrigen DNA-Einzelsträngen erhöht wird, so dass in der Probe der Sondenstrang an sich und insbesondere der DNA-Strang mit der Mutation gewissermaßen überrepräsentiert ist und somit aufgrund der statistisch häufigeren Wechselwirkung mit der Sensorsonde ein verstärktes Sensorsignal resultieren kann.

Zudem ist es im Rahmen der vorliegenden Erfindung in völlig überraschender Weise gelungen, die Sensitivität noch weiter dadurch zu erhöhen, dass die asymmetrische Polymerase-Kettenreaktion zudem in Gegenwart eines nachfolgend noch angeführten speziellen Blockierungsmittels bzw. Blockers in Form von LNA-Molekülen (*locked nucleic acid)* bzw. *Clamp*-Molekülen durchgeführt wird, welches bzw. welcher spezifisch, d. h. mit erhöhter Affinität, an den DNA-Einzelstrang ohne Mutation bzw. den wildtypischen DNA-Einzelstrang an dem zum Mutationsbereich korrespondierenden Abschnitt bindet und die Anbindung der Sensorsonde an diesen Bereich der nichtmutierten DNA verhindert bzw. verringert. Was das eingesetzte Blockierungsmittel bzw. die LNA-Sonde, synonym auch als *Clamp-Sonde* bzw. Kompetitor bezeichnet, so ist diese erfindungsgemäß derart konzipiert, dass das Blockierungsmittel gleichermaßen wie die eingesetzte Sensorsonde an die Wildtypsequenz bindet, und zwar an einer Stelle, an welcher auch die Sensorsonde zu binden imstande ist. Hierdurch wird sowohl die Amplifikation des entsprechenden DNA-Einzelstranges, an welchem das Blockierungsmittel bindet, als auch die Bindung der Sensorsonde an die entsprechende Wildtypsequenz minimiert bzw. unterdrückt.

Auf Basis der erfindungsgemäßen Kombination von Maßnahmen ist es im Rahmen der vorliegenden Erfindung in völlig überraschender Weise gelungen, ein Verfahren zur Detektion von Mutationen bzw. Genveränderungen bereitzustellen, welches zudem zu einer deutlichen Verstärkung speziell des auf die zu untersuchende Mutation zurückgehenden Messsignals im Sinne einer Diskriminierung bzw. Verstärkung gegenüber den sonstigen Signalen in der Probe führt und bei welchem eine Vielzahl an Mutationen mit ein und derselben Sensorsonde erfasst werden können. Auf Basis des erfindungsgemäßen Verfahrens können im Ergebnis bereits sehr geringe Mengen an mutierter DNA in einer Probe bzw. Ausgangsmaterial analysiert werden. Aufgrund des hochsensitiven Verfahrens ist es im Rahmen der vorliegenden Erfindung möglich, auf Proben zurückzugreifen, welche nur geringe Mengen an auf Tumorzellen zurückgehende DNA aufweisen. Somit können im Rahmen der vorliegenden Erfindung bereits kleinste Anteile mutierter DNA nachgewiesen werden, ohne dass hierzu aufwändige und aus medizinischer Sicht mitunter problematische Biopsien durchgeführt werden müssen. Grundsätzlich können erfindungsgemäß aber auch Zellproben bzw. Zellmaterial, z. B. Tumorzellen als solche dem erfindungsgemäßen Verfahren zugrundegelegt werden.

Auf Basis des erfindungsgemäßen Verfahrens mit der aussagekräftigen Detektion bzw. Analyse einer Vielzahl verschiedenartiger Mutationen, welche hinsichtlich einer speziellen Erkrankung von Relevanz sind, ist es weiterhin auch möglich, in gezielter Weise eine Aussage über den Krankheitszustand bzw. eine Verlaufskontrolle, insbesondere in Bezug auf Arzneimittelwirkungen oder dergleichen, zu ermöglichen, wobei zudem auf Basis der konkreten Mutationsanalyse diesbezüglich optimierte Tumortherapien, beispielsweise unter Verwendung mutationsspezifischer Inhibitoren, durchgeführt werden können.

Zusammenfassend ist es somit im Rahmen der vorliegenden Erfindung erstmalig möglich, durch die Verwendung spezieller Hybridisierungssonden in vorteilhafter Kombination mit einer asymmetrischen Polymerase-Kettenreaktion und der Verwendung von speziellen Blockierungsmitteln Deletionen unterschiedlicher Größe und in leicht unterschiedlicher Lage nachzuweisen - und dies auch bei geringem Gehalt an mutierter DNA in einer zu analysierenden Probe.

Das der vorliegenden Erfindung zugrundeliegende Prinzip liegt somit in einem hochsensitiven Nachweis verschiedener Genveränderungen bzw. Deletionen mit Hilfe einer einzigen Sensorsonde. Im Gegensatz dazu war im Stand der Technik bislang lediglich der Nachweis einer einzigen, spezifischen Punktmutation mit Hilfe einzelner spezifischer Sensorsonden möglich. In Bezug auf den EGF-Rezeptor wird dies - rein veranschaulichend - insbesondere durch eine spezifische Bindung der Sensorsonde an die Wildtypsequenz im Bereich von Exon 19 des EGF-Rezeptors möglich. Erfindungsgemäß ist die eingesetzte Sensorsonde somit insbesondere derart konzipiert, dass eine unspezifische Bindung der Sensorsonde im Bereich einer potentiellen Deletion (Mutation) erfolgt, wobei an der konkreten Stelle der Mutation in vorteilhafter Weise zumindest im wesentlichen keine Interaktion bzw. Bindung vorliegt. Auf dieser Basis können erfindungsgemäß die weitaus häufigsten und bekannten Mutationen insbesondere in Exon 19 des EGF-Rezeptors, beispielsweise beginnend an der Aminosäureposition 746 oder 747, detektiert werden.

Die Funktionsweise der erfindungsgemäß eingesetzten Sensorsonde kann nachfolgend rein beispielhaft und die vorliegende Erfindung nicht beschränkend an einer Deletion in Position 747 in Exon 19 des EGF-Rezeptors veranschaulicht werden: Liegt eine beliebige Deletion in diesem Bereich vor, ist die erfindungsgemäß eingesetzte Sensorsonde so konzipiert, dass sie komplementär zur Wildtypsequenz bindet und an Position 747 mit der letzten Base bzw. dem letzten Nukleotid abschließt. Liegt nun eine Deletion in einem der Bereiche vor, kann die Sonde nicht oder nur unzureichend an Position 747 oder 746 und 747 binden. Auf diese Weise wird jedwede potentielle Mutation bzw. Deletion in diesem Bereich mit einer einzigen Sondenkombination detektiert, insbesondere da die Sensorsonde auch derart konzipiert ist, dass sie hinsichtlich ihrer Länge bzw. der Anzahl an Nukleotiden bzw. Basen größer ist als der zu erfassende Deletionsbereich. Somit werden auch die an die Mutation angrenzenden Randbereiche gewissermaßen umfasst, wobei diesbezüglich ein zur Mutation randständiger bzw. benachbarter Bereich bevorzugt ist, mit welchem die eingesetzte Sensorsonde auch im Fall des Mutationsgens interagiert, da dieser Bereich dem der Wildtypsequenz entspricht. Im Falle der zuvor angeführten Deletion an der Aminosäureposition 746 bzw. 747 ist das weiterhin eingesetzte Blockierungsmittel insbesondere derart konzipiert, dass auch dieses mit der letzten Base des Codons 747 abschließt. Auf diese Weise wird der Wildtyp inhibiert und die Deletion kann dann mit einer hohen Sensitivität mit der Sensorsonde nachgewiesen werden.

Die vorliegende Erfindung ist nicht auf die Erfassung von Genveränderungen im EGF-Rezeptor beschränkt, vielmehr ist es im Rahmen der vorliegenden Erfindung möglich, insbesondere Insertionen, Deletionen und Translokationen als solche in jeweils unterschiedlichen Genen mit hoher Sensitivität zu detektieren. Dabei kommt im Allgemeinen erfindungsgemäß insbesondere die Detektion von Mutationen an für das Auftreten von chromosomalen Veränderungen bekannten und häufig wiederkehrenden Stellen in Frage. Klinisch relevante Beispiele für derartige Mutationen sind neben dem EGF-Rezeptor beispielsweise Insertionen in Form der so genannten *"Internal Tandem Duplications (ITD)"* in der Rezeptortyrosinkinase FLT3, die häufig bei akuter myeloischer Leukämie (etwa 30 %) zu finden ist. Ein weiteres Beispiel stellen Translokationen im Fusionsgen bzw. Fusionsprotein EML4-ALK dar.

Wie zuvor angeführt, ist es im Rahmen der vorliegenden Erfindung möglich, dass das Mutationsgen bzw. die Mutationsgene gewissermaßen in einem Ensemble bzw. einer Probe gemeinsam mit einem Wildtypgen bzw. mit Wildtypgenen vorliegt bzw. vorliegen, was beispielsweise dann der Fall ist, wenn in bereitgestellter (Ausgangs-)Probe sowohl Tumorzellen als auch nichtentartete Zellen vorliegen.

Im Rahmen der vorliegenden Erfindung ist es auch möglich, dass die entsprechenden Gene Mutationsallele und Wildtypallele aufweisen bzw. aus diesen bestehen. So kann beispielsweise das Mutationsgen im Sinne einer homozygoten Ausprägung zwei Mutationsallele aufweisen, d. h. beide Allele des Gens sind Träger der entsprechenden Genveränderung bzw. Mutation. Gleichermaßen ist es möglich, dass das Mutationsgen heterozygot ausgebildet ist, wobei in diesem Fall ein Allel, nämlich das Mutationsallel, mit der Genveränderung bzw. Mutation und - hierzu korrespondierend - ein Wildtypallel ohne Mutation in Bezug auf das Mutationsgen vorliegt. In Bezug auf das Wildtypgen, welches insbesondere aus gesunden bzw. nichtmalignen Zellen stammt, liegen beide Allele insbesondere in der Wildtyp-Form bzw. als Wildtypallele vor. Insofern können im Nachfolgenden die verwendeten Begriffe "Mutationsgen" bzw. "Wildtypgen" sich insbesondere auch auf die entsprechenden Allele, wie zuvor definiert, beziehen. Das erfindungsgemäße Verfahren eignet sich somit auch zum Nachweis bzw. zur Detektion von Mutationen in den jeweiligen Allelen eines Gens.

Gemäß einer erfindungsgemäß bevorzugten Ausführungsform kann es sich bei der Genveränderung, insbesondere Mutation, um eine Leserastermutation, insbesondere um eine Deletion und/oder Insertion, handeln. Gleichermaßen kann es sich erfindungsgemäß um eine Punktmutation handeln. Wie zuvor angeführt, ist es erfindungsgemäß bevorzugt, wenn es sich bei der Genveränderung um eine Deletion und/oder Insertion handelt. Gleichermaßen können auf Basis des erfindungsgemäßen Verfahrens auch Translokations-Mutationen nachgewiesen werden.

Erfindungsgemäß ist es vorgesehen, dass die Sensorsonde derart ausgewählt wird, dass die Sensorsonde an dem DNA-Einzelstrang (wt-Sondenstrang) des Wildtypgens und dem hierzu entsprechenden und die Genveränderung aufweisenden DNA-Einzelstrang (mt-Sondenstrang) des Mutationsgens zu binden imstande ist, wobei die Sensorsonde eine höhere Spezifität und/oder Bindungsaffinität und/oder Selektivität zu dem DNA-Einzelstrang (wt-Sondenstrang) des Wildtypgens als zu dem die Genveränderung aufweisenden DNA-Einzelstrang (mt-Sondenstrang) des Mutationsgens, insbesondere an der Position und/oder Stelle der Genveränderung, aufweist.

Die erfindungsgemäß eingesetzte Sensorsonde ist derart ausgewählt, dass die Sensorsonde eine geringere Spezifität und/oder Bindungsaffinität und/oder Selektivität zu dem DNA-Einzelstrang (mt-Sondenstrang) des Mutationsgens an der Position und/oder Stelle der Genveränderung als zu dem entsprechenden DNA-Einzelstrang (wt-Sondenstrang) des Wildtypgens aufweist. Der Begriff "entsprechender DNA-Einzelstrang des Wildtypgens" bezieht sich insbesondere auf den zum mutierten DNA-Einzelstrang korrespondierenden Bereich des DNA-Einzelstrangs ohne Genveränderung.

Die erfindungsgemäß eingesetzte Sensorsonde ist erfindungsgemäß derart ausgewählt, dass die Sensorsonde mit dem DNA-Einzelstrang (wt-Sondenstrang) des Wildtypgens eine spezifische Bindung einzugehen imstande ist und hiermit vollständig hybridisiert.

Was die erfindungsgemäß eingesetzte Sensorsonde weiterhin anbelangt, so ist diese ausgewählt derart, dass die Sensorsonde vollständig bzw. über ihre vollständige Nukleotidsequenz an dem DNA-Einzelstrang (wt-Sondenstrang) des Wildtypgens zu binden imstande ist.

Diesbezüglich sollte die Länge der Sensorsonde bzw. die Anzahl der die Sensorsonde ausbildenden Nukleotide derart ausgewählt werden, dass diese zum einen die Anzahl der die Genveränderung ausbildenden Nukleotide zumindest im wesentlichen umfasst sowie zum anderen weitere Nukleotide aufweist, die gewissermaßen einen Randbereich der Sensorsonde ausbilden bzw. an einen Randbereich zu der die Genveränderung ausbildenden Nukleotidsequenz binden können.

Weiterhin ist die Sensorsonde vollständig bzw. über ihre vollständige bzw. gesamte Nukleotidsequenz an dem Abschnitt des DNA-Einzelstrangs (wt-Sondenstrang) des Wildtypgens, welcher zu dem Abschnitt der Genveränderung des die Genveränderung aufweisenden DNA-Einzelstrangs (wt-Sondenstrang) des Mutationsgens korrespondiert, zu binden imstande.

Weiterhin ist die Sensorsonde ausgewählt derart, dass die Sensorsonde zumindest eine komplementäre Nukleotidsequenz zu dem DNA-Einzelstrang (wt-Sondenstrang) des Wildtypgens aufweist. Hierdurch wird vollständige Bindung bzw. Hybridisierung der Sensorsonde an die Wildtyp-DNA gewährleistet. Dabei ist die Sensorsonde komplementär zu dem Bereich des Wildtypgens, welcher zu dem Mutationsbereich korrespondiert, und einem sich hieran anschließenden Randbereich.

Was die erfindungsgemäß eingesetzte Sensorsonde weiterhin anbelangt, so ist es erfindungsgemäß vorgesehen, dass die Sensorsonde mit dem DNA-Einzelstrang (mt-Sondenstrang) des Mutationsgens eine unspezifische und/oder unvollständige und/oder abschnittsweise Bindung einzugehen imstande ist, insbesondere hiermit nur unvollständig und/oder nur abschnittsweise hybridisiert.

In diesem Zusammenhang ist die Sensorsonde derart ausgewählt, dass die Sensorsonde an der Position und/oder Stelle der Genveränderung keine Bindung und/oder Wechselwirkung mit dem DNA-Einzelstrang (mt-Sondenstrang) des Mutationsgens einzugehen imstande ist und an einer Position und/oder Stelle der Genveränderung nicht hybridisiert.

Zudem ist die erfindungsgemäß eingesetzte Sonde derart ausgewählt, dass die Sensorsonde an der Position und/oder Stelle der Genveränderung mit dem die Genveränderung aufweisenden DNA-Einzelstrang (mt-Sondenstrang) des Mutationsgens nicht zu interagieren und/oder nicht wechselzuwirken bzw. nicht hieran zu binden imstande ist. Mit anderen Worten ist die Sensorsonde erfindungsgemäß derart konzipiert, dass diese nicht an der Stelle bzw. Position der Mutation an sich in dem Mutationsgen mit dem Mutationsgen interagieren bzw. wechselwirken, insbesondere hieran zu binden, imstande ist.

Weiterhin ist es erfindungsgemäß vorgesehen, dass die Sensorsonde derart ausgewählt wird, dass die Sensorsonde nur abschnittsweise mit dem DNA-Einzelstrang (mt-Sondenstrang) des Mutationsgens zu interagieren und/oder wechselzuwirken, insbesondere hieran zu binden, imstande ist. Insbesondere handelt es sich bei dem Abschnitt, mit welchem die Sensorsonde mit dem mt-Sondenstrang zu interagieren imstande ist, um einen Randabschnitt bzw. randständigen Bereich und/oder um einen Endabschnitt bzw. endständigen Bereich der Nukleotidsequenz der Sensorsonde. Mit anderen Worten kann es erfindungsgemäß in vorteilhafter Weise vorgesehen sein, dass in Bezug auf die Sensorsonde sozusagen das eine Ende mit einer bestimmten Anzahl an Nukleotiden bzw. Basen mit dem mt-Sondenstrang zu interagieren imstande ist, während das andere Ende mit einer definierten Anzahl an Nukleotiden bzw. Basen nicht mit dem mt-Sondenstrang interagiert bzw. hybridisiert.

Aufgrund dieser erfindungsgemäßen Konzeption der Sensorsonde handelt es sich diesbezüglich gewissermaßen um eine "Flattersonde" mit einer lediglich randständigen bzw. abschnittsweisen Interaktion mit dem mt-Sondenstrang. Erfindungsgemäß handelt es sich somit bei der Sensorsonde in vorteilhafter Weise um eine derartige Sonde, welche nur mit einem Teil und/oder Abschnitt ihrer Nukleotidsequenz mit dem Mutationsgen zu interagieren und/oder wechselzuwirken, insbesondere hieran zu binden, imstande ist, wobei es sich diesbezüglich insbesondere um einen Rand- bzw. Endabschnitt der Sensorsonde handelt.

Was die Sensorsonde weiterhin anbelangt, so sollte diese derart ausgewählt werden, dass die Sensorsonde mit dem der Position und/oder Stelle der Genveränderung anschließenden Abschnitt des die Genveränderung aufweisenden DNA-Einzelstrangs (mt-Sondenstrang) des Mutationsgens zu interagieren und/oder wechselzuwirken, insbesondere hieran zu binden, imstande ist.

Weiterhin kann es erfindungsgemäß vorgesehen sein, dass die Sensorsonde derart ausgewählt wird, dass die Sensorsonde, bezogen auf den die Genveränderung aufweisenden DNA-Einzelstrang (mt-Sondenstrang) des Mutationsgens, mindestens einen Bindungsbereich und/oder -abschnitt, vorzugsweise einen einzigen Bindungsbereich und/oder -abschnitt, und mindestens einen Nichtbindungsbereich und/oder -abschnitt, vorzugsweise einen einzigen Nichtbindungsbereich und/oder -abschnitt, aufweist. Da die Sensorsonde, wie zuvor angeführt, zumindest im Wesentlichen vollständig an den wt-Sondenstrang hybridisiert, weist die Sensorsonde in Bezug auf den wildtypischen DNA-Einzelstrang (wt-Sondenstrang) demnach nur einen einzigen, sozusagen durchgehenden Bindungsbereich auf, welcher zumindest im Wesentlichen durch die vollständige Nukleotidsequenz der Sensorsonde ausgebildet wird. Insbesondere bezieht sich der Nichtbindungsbereich der Sensorsonde auf den Mutationsbereich des wt-Sondenstrangs, während sich der Bindungsbereich auf einen sich vorzugsweise unmittelbar an den Bereich der Genveränderung angrenzenden Bereich des wt-Sondenstrangs bezieht, insbesondere wobei dieser Bereich des wt-Sondenstrangs, an dem die Sensorsonde binden kann, die wildtypische Nukleotidsequenz aufweist.

Im Allgemeinen kann die Anzahl der Nukleotide des Nichtbindungsbereichs der Sensorsonde zumindest im Wesentlichen der Anzahl der die Genveränderung ausbildenden Nukleotide entsprechen. Beispielsweise kann der Nichtbindungsbereich 3, 6, 9 oder mehr Nukleotide umfassen, wobei diesbezüglich auch ganze Zahlen zwischen den vorgenannten Werten möglich sind, wie beispielsweise 1, 2, 4 oder 5 Nukleotide. Erfindungsgemäß kann es insbesondere bei größeren Deletionen, bei denen zwei oder mehr Codons deletiert sind, auch vorgesehen sein, dass der Nichtbindungsbereich kleiner ist als die Genveränderung als solche.

Mit andere Worten kann es vorgesehen sein, dass die Anzahl der Nukleotide der Sensorsonde, welche nicht mit dem die Genveränderung aufweisenden DNA-Einzelstrang (mt-Sondenstrang) des Mutationsgens zu interagieren und/oder wechselzuwirken, insbesondere nicht hieran zu binden, imstande ist, im Bereich von 1 bis 15, insbesondere 2 bis 12, vorzugsweise 3 bis 9, bevorzugt 3 bis 6, liegt. Da, wie zuvor angeführt, die Sensorsonde zumindest im Wesentlichen vollständig an den wt-Sondenstrang bindet, stellen die vorgenannten Werte gewissermaßen die Differenz aus bindenden Nukleotiden bzw. Basen der Sensorsonde an den wt-Sondenstrang zu den bindenden Nukleotiden bzw. Basen der Sensorsonde an den mt-Sondenstrang dar.

Gleichermaßen kann die Anzahl der Nukleotide bzw. Basen des Bindungsbereichs im Bereich von 1 bis 30, insbesondere 3 bis 21, vorzugsweise 6 bis 12 liegen. Auch diesbezüglich sind in Bezug auf die Anzahl der den Bindungsbereich ausbildenden Nukleotide auch ganze Zahlen zwischen den Codons bildenden Triplettwerten möglich, wie 4 oder 5 Nukleotide.

Insbesondere kann die Sensorsonde ausgewählt werden derart, dass die Sensorsonde mindestens 3, insbesondere mindestens 6, vorzugsweise mindestens 9 Nukleotide aufweist und/oder dass die Anzahl der Nukleotide der Sensorsonde im Bereich von 3 bis 60, insbesondere 6 bis 36, vorzugsweise 9 bis 21, beträgt. Auch diesbezüglich sind ganze Zahlen zwischen den Codons bildenden Triplettwerten möglich, wie 4 oder 5 Nukleotide. Die zuvor angegebene Größe bezieht sich auf den Nukleotidabschnitt der Sensorsonde.

Das Verhältnis von bindenden Nukleotiden zu nichtbindenden Nukleotiden der Sensorsonde, bezogen auf den die Genveränderung aufweisenden DNA-Einzelstrang (mt-Sondenstrang) des Mutationsgens, kann im Bereich von 10 : 1 bis 1 : 10, insbesondere 6 : 1 bis 1 : 6, vorzugsweise 4 : 1 bis 1 : 4, liegen. Mit anderen Worten kann das Verhältnis von Bindungsbereich zu Nichtbindungsbereich der erfindungsgemäß eingesetzten Sensorsonde hinsichtlich der jeweiligen Anzahl an Nukleotiden bzw. Basen auf Basis der oben angeführten Verhältnisse ausgebildet werden.

Im Rahmen des erfindungsgemäßen Verfahrens kann es weiterhin vorgesehen sein, dass die Sensorsonde derart ausgewählt wird, dass die Sensorsonde mit höchstens 60 %, insbesondere höchstens 50 %, vorzugsweise höchstens 40 %, der die Sensorsonde ausbildenden Nukleotide bzw. Basen mit dem die Genveränderung aufweisenden DNA-Einzelstrang (mt-Sondenstrang) des Mutationsgens nicht zu interagieren und/oder nicht wechselzuwirken, insbesondere nicht hieran zu binden, imstande ist, bezogen auf die Gesamtzahl der Nukleotide bzw. Basen der Sensorsonde. Dementsprechend kann es erfindungsgemäß vorgesehen sein, dass die Sensorsonde mit mindestens 40 %, insbesondere mindestens 50 %, vorzugsweise mindestens 60 %, der die Sensorsonde ausbildenden Nukleotide bzw. Basen mit dem die Genveränderung aufweisenden DNA-Einzelstrang (mt-Sondenstrang) des Mutationsgens zu interagieren bzw. hieran zu binden imstande ist. Erfindungsgemäß führt das definierte Verhältnis zwischen bindenden zu nichtbindenden Nukleotiden, bezogen auf den mt-Sondenstrang, zu einer Differenzierung zu der vollständig an dem wt-Sondenstrang des Wildtypgens bindenden Sensorsonde, insbesondere was die Ausbildung bzw. Maßschneiderung von unterschiedlichen Ablöse- bzw. Schmelztemperaturen hinsichtlich der hybridisierten Sensorsonden anbelangt. Auf diese Weise kann eine differenzierte Aussage über das Vorliegen einer Mutation getroffen werden, welches sich nämlich insbesondere an dem Vorhandensein einer niedrigeren Schmelztemperatur, bedingt durch die lediglich partielle Hybridisierung der Sensorsonde an den mt-Sondenstrang, im Vergleich zu der höheren Schmelztemperatur der Sensorsonde, welche an den wt-Sondenstrang bindet, äußert.

Im Rahmen der vorliegenden Erfindung kann in diesem Zusammenhang vorgesehen sein, dass die Sensorsonde derart ausgebildet wird, dass eine wärmeinduzierte Ablösung der Sensorsonde von dem DNA-Einzelstrang des Mutationsgens (mt-Sondenstrang) bei niedrigeren Temperaturen erfolgt als von dem entsprechenden DNA-Einzelstrang des Wildtypgens (wt-Sondenstrang). Dies wird, wie zuvor angeführt, insbesondere durch die geringere Anzahl der an dem mt-Sondenstrang bindenden Nukleotide bzw. Basen gewährleistet bzw. hervorgerufen.

Erfindungsgemäß kann die Sensorsonde die Nukleotidsequenz TAATT-CCTTGATAGCGACGGG aufweisen oder hieraus bestehen, bezogen auf deren Nukleotidsequenz. Das vorgenannte Beispiel ist nicht beschränkend. Vielmehr ist der Fachmann jeder Zeit in der Lage, spezifische Sensorsonden hinsichtlich ihrer Nukleotidsequenz bzw. ihres Nukleotidabschnitts derart auszuwählen, dass eine erhöhte Spezifität zu dem Wildtypgen im Sinne der vorliegenden Erfindung und im Hinblick auf die zu erfassenden Mutationen gewährleistet ist.

Was die Bindung der Sensorsonde an den mt-Sondenstrang anbelangt, so kann diese veranschaulichend so beschrieben werden, dass die Sensorsonde an dem unmittelbar an die Genveränderung angrenzenden Bereich des mt-Sondenstrangs bindet, während der Mutationsbereich nicht in die Bindung einbezogen ist, sondern die Sensorsonde den Mutationsbereich, gewissermaßen ohne eine Bindung einzugehen, überlappt.

Was die Sensorsonde (Reportersonde) als solche anbelangt, so handelt es sich hierbei um eine Hybridisierungssonde, welche insbesondere im Rahmen der PCR und den diesbezüglichen Amplifizierungsschritten mit den jeweiligen DNA-Einzelsträngen des Wildtypgens bzw. Wildtypallels und/oder des Mutationsgens bzw. Mutationsallels im Bereich der zu analysierenden bzw. zu untersuchenden Mutation zu binden bzw. zu hybridisieren imstande ist, insbesondere wie zuvor beschrieben.

Dabei besteht ein der Erfindung zugrundeliegendes Prinzip darin, dass die in Form einer Hybridisierungssonde eingesetzte Sensorsonde eine erhöhte Selektivität bzw. Affinität zu dem DNA-Einzelstrang des Wildtypgens bzw. Wildtypallels gegenüber dem korrespondierenden Genabschnitt bzw. Nukleotidbereich, welcher die zu untersuchende Mutation aufweist, besitzt. Somit ist die Selektivität bzw. Affinität der Sensorsonde zu dem Mutationsbereich des Mutationsgens bzw. des Mutationsallels bzw. des diesbezüglichen Einzelstrangs verringert. Die Selektivität bzw. Spezifität in Bezug auf den Wildtypbereich kann in dem Fachmann an sich bekannter Weise durch eine spezielle Auswahl der Nukleotidsequenz der Hybridisierungssonde gewährleistet werden. Diesbezüglich sollte die Nukleotidsequenz bzw. Basenabfolge derart ausgewählt werden, dass die entsprechende Nukleotidsequenz zumindest im Wesentlichen komplementär zu den entsprechenden Nukleotiden des Wildtyps ist. Auf diese Weise wird die erhöhte Sensitivität bzw. Affinität bzw. Bindungsstärke der Sensorsonde in Bezug auf den zu dem Mutationsbereich korrespondierenden Bereich des Wildtypgens bzw. -allels insofern gewährleistet, als die Anzahl der Basenpaarungen zwischen Sensorsonde und Wildtyp größer ist als in Bezug auf eine insofern unspezifische Interaktion der Sensorsonde mit dem korrespondierenden Genabschnitt des Mutationsgens. Ohne sich auf diese Theorie festlegen zu wollen, führt die höhere Bindungsaffinität, insbesondere auf Basis der höheren Anzahl an Basenpaarungen bei Interaktion der Sensorsonde mit dem entsprechenden Genabschnitt bzw. DNA-Einzelstrang, zu einer "festeren" Bindung bzw. Interaktion, was im Vergleich zu der Interaktion der Sensorsonde mit dem korrespondierenden Bereich des Mutationsgens zu einem höheren Schmelzpunkt, d. h. insbesondere zu einer Ablösung bzw. Dehybridisierung der Sensorsonde von dem jeweiligen DNA-Einzelstrang erst bei höheren Temperaturen führt.

Weiterhin ist es erfindungsgemäß vorgesehen, dass die Sensorsonde derart ausgewählt wird, dass die Sensorsonde im Falle der Wechselwirkung und/oder Interaktion und/oder Bindung an den DNA-Einzelstrang des Mutationsgens (mt-Sondenstrang) und/oder an den entsprechenden DNA-Einzelstrang des Wildtypgens (wt-Sondenstrang) ein Fluoreszenzsignal abzugeben imstande ist. Bei dem Signal handelt es sich, wie nachfolgend noch angeführt, um ein FRET-Signal.

Weiterhin kann es im Rahmen der vorliegenden Erfindung vorgesehen sein, dass die Sensorsonde derart ausgebildet ist, dass die Sensorsonde im Falle einer insbesondere wärmeinduzierten Ablösung von dem DNA-Einzelstrang des Mutationsgens (mt-Sondenstrang) und/oder von dem korrespondierenden DNA-Einzelstrang des Wildtypgens (wt-Sondenstrang) kein oder aber zumindest ein vermindertes detektierbares und/oder messbares Signal, insbesondere kein oder aber zumindest ein vermindertes Fluoreszenzsignal, abzugeben imstande ist.

Im Rahmen der vorliegenden Erfindung ist es bevorzugt, wenn die Sensorsonde eine vorzugsweise mit einer detektierbaren Substanz (Markierung), vorzugsweise mit einer bei Interaktion bzw. Bindung der Sensorsonde mit dem DNA-Einzelstrang des Mutationsgens detektierbaren Substanz, insbesondere einem Farbstoff, vorzugsweise einem Fluoreszenzfarbstoff, ausgerüstetes bzw. markiertes Nukleotidmolekül, insbesondere ein markiertes Oligo- oder Polynukleotid, ist.

Mit anderen Worten sollte die Sensorsonde ausgebildet werden derart, dass diese zumindest im Wesentlichen nur bei Interaktion bzw. Bindung bzw. Hybridisierung an dem jeweiligen DNA-Einzelstrang unter Anregung, beispielsweise unter Anregung mit elektromagnetischer Strahlung mit einer speziellen Wellenlänge bzw. mit einem speziellen Wellenlängenbereich, ein messbares Signal, insbesondere Fluoreszenzsignal, abgibt bzw. aussendet. Entsprechend sollte die nichtgebundene Sensorsonde auch unter Anregung kein Signal als solches abgeben können.

Im Rahmen der vorliegenden Erfindung werden - in Verbindung mit der nachfolgend angeführten Ankersonde - so genannte FRET-Sonden (*Fluorescence Resonance Energy Transfer-*Sonden) eingesetzt. Diesbezüglich können beispielsweise unterschiedliche Farbstoffe, insbesondere Fluoreszenzfarbstoffe bzw. Fluorochrome, zum Einsatz kommen, welche an die Sensorsonde, einerseits und an ein weiteres Molekül bzw. eine weitere Sonde, wie die Ankersonde, andererseits, gebunden sind. Eine Signalgebung kann in diesem Fall in Abhängigkeit von der Beabstandung der entsprechenden Fluoreszenzfarbstoffe zueinander und in Abhängigkeit von der Bindung an den jeweiligen DNA-Einzelstrang ausgebildet werden. Die diesbezüglichen Farbstoffe bzw. Farbstoffsysteme sind dem Fachmann als solchen wohlbekannt.

Erfindungsgemäß ist es zu Zwecken der Ausbildung eines FRET-Signals bei Interaktion bzw. Anbindung der Sensorsonde an den jeweiligen Sondenstrang vorgesehen, dass zusätzlich zu der Sensorsonde mindestens eine hiervon verschiedene zweite Hybridisierungssonde (Ankersonde) eingesetzt wird, wobei die Ankersonde einen zu dem detektierbaren Stoff der Sensorsonde kompatiblen Stoff zu Zwecken der Ausbildung eines FRET-Signals aufweist. Dabei sind die Sensorsonde einerseits und die Ankersonde andererseits bzw. der detektierbare Stoff der Sensorsonde einerseits und der detektierbare Stoff der Ankersonde andererseits ein FRET-Paar auszubilden imstande, wenn sowohl die Sensorsonde als auch die Ankersonde an den entsprechenden Sondenstrang gebunden sind und die Ankersonde ausgewählt ist derart, dass die Ankersonde auf demselben DNA-Einzelstrang wie die Sensorsonde zu binden imstande ist, insbesondere wobei die Ankersonde in einem Abstand von 1 bis 5 bp (Basenpaare) zu der Sensorsonde zu binden imstande ist. Diesbezüglich sollten im Falle einer Bindung der Sensorsonde und der Ankersonde an den entsprechenden Sondenstrang die detektierbaren Stoffe zueinander gewandt sein. Die Ankersonde sollte eine zum Wildtyp komplementäre Nukleotid- bzw. Basensequenz aufweisen.

Die das FRET-Paar ausbildenden Stoffe bzw. Substanzen können in dem Fachmann an sich bekannter Art und Weise an die entsprechenden Sonden gekoppelt werden, beispielsweise durch eine kovalente Bindung. Erfindungsgemäß wird zusätzlich zu der Sensorsonde mindestens eine hiervon verschiedene zweite Hybridisierungssonde (Ankersonde) eingesetzt.

Diesbezüglich ist es im Rahmen der vorliegenden Erfindung vorgesehen, dass die Ankersonde ein mit mindestens einer zu der Sensorsonde komplementären bzw. kompatiblen detektierbaren Substanz (Markierung in Form eines Fluoreszenzfarbstoffs ausgerüstetes bzw. markiertes Nukleotidmolekül ist. Die Ankersonde sollte ein Oligo- bzw. Polynukleotid sein bzw. ein solches Molekül bzw. einen solchen Abschnitt aufweisen. Der Nukleotidabschnitt der Ankersonde sollte eine Größe von 3 bis 50 bp, insbesondere 5 bis 45 bp, vorzugsweise 10 bis 40 bp, aufweisen. Die Nukleotidsequenz der Ankersonde ist dabei ausgewählt derart, dass die Ankersonde auf demselben DNA-Einzelstrang wie die Sensorsonde zu binden imstande ist, wobei die Bindung der Ankersonde in einem benachbarten Bereich zu der zu analysierenden Mutation und somit nicht im unmittelbaren Mutationsbereich bzw. der hierzu korrespondierenden Stelle des wildtypischen DNA-Einzelstrangs erfolgen sollte. Erfindungsgemäß erfolgt die Bindung der Ankersonde an dem Sondenstrang derart, dass die zu detektierenden Substanzen, insbesondere Farbstoffe, der Ankersonde einerseits und der Sensorsonde andererseits imstande sind, miteinander eine Wechselwirkung zur Bildung eines detektierbaren Signals nach Art eines FRET-Paares auszubilden.

In diesem Zusammenhang ist es erfindungsgemäß vorgesehen, dass die Ankersonde auf demselben DNA-Einzelstrang wie die Sensorsonde zu binden imstande ist, insbesondere wobei die Ankersonde in einem Abstand von 1 bis 5 bp zu der Sensorsonde zu binden imstande sein sollte. Auf diese Weise wird eine Wechselwirkung der detektierbaren Substanzen, insbesondere der Fluoreszenzfarbstoffe, ermöglicht. Der zuvor angeführte Abstand von 1 bis 5 bp entspricht einer räumlichen Beabstandung von etwa 1 bis 10 nm. Wie zuvor angeführt, ist im Rahmen der vorliegenden Erfindung die Ankersonde ausgebildet derart, dass diese sowohl an dem DNA-Einzelstrang mit der Mutation (Sondenstrang) als auch an dem korrespondierenden wildtypischen DNA-Einzelstrang zu binden imstande ist. Dies ergibt sich auch dadurch, dass die jeweiligen Bindungsbereiche in Bezug sowohl auf den Sondenstrang als auch auf den wildtypischen DNA-Einzelstrang im Bereich der Bindungsstelle der Ankersonde eine zumindest im Wesentlichen identische Nukleotidsequenz bzw. Basenabfolge aufweisen. Vor diesem Hintergrund kann die Ankersonde an den Sondenstrang einerseits und den wildtypischen DNA-Einzelstrang andererseits mit vergleichbarer Spezifität binden, während die Sensorsonde an den Sondenstrang im Vergleich zu dem wildtypischen DNA-Einzelstrang mit erhöhter Affinität bzw. Bindungsstärke bindet. Im Rahmen der vorliegenden Erfindung ist es vorteilhaft, wenn die Sensorsonde einerseits und die Ankersonde andererseits, insbesondere die detektierbare Substanz bzw. Markierung der Sensorsonde einerseits und die detektierbare Substanz bzw. Markierung der Ankersonde andererseits, bei Bindung der Sonden ein FRET-Paar auszubilden imstande sind.

In diesem Zusammenhang besteht eine Möglichkeit der Nutzung des FRET zur Quantifizierung der zugrundeliegenden Genveränderung bzw. Mutation in der Verwendung von so genannten LightCycler®-Sonden, welche spezielle Hydrolysesonden darstellen, wobei verschiedene, jeweils mit einem Donor bzw. Akzeptor markierte Oligonukleotide (Sensorsonde und Ankersonde), die nebeneinander an der Zielsequenz (Wildtyp- bzw. Mutationsbereich in Bezug auf die Sensorsonde einerseits und diesbezüglich benachbarter Bereich in Bezug auf die Ankersonde) des zu untersuchenden Genabschnitts bzw. der entsprechenden DNA-Einzelstränge binden und damit den Donor und den Akzeptor in eine für den FRET ausreichende Nähe bringen. Derartige Sondenpaare können somit zur Quantifizierung der zugrundeliegenden Mutation im Rahmen des erfindungsgemäßen Verfahrens und somit sozusagen zur Quantifizierung von PCR-Produkten eingesetzt werden. Dabei kann es im Rahmen der vorliegenden Erfindung vorgesehen sein, dass beispielsweise die Sensorsonde mit einem detektierbaren Stoff, insbesondere Fluoreszenzfarbstoff, in Form eines Donors und die Ankersonde entsprechend mit einem detektierbaren Stoff, insbesondere Fluoreszenzfarbstoff, in Form eines Akzeptors ausgerüstet bzw. markiert wird. Auch eine umgekehrte Ausrüstung bzw. Markierung, d. h. Ausrüstung der Sensorsonde mit einem Akzeptor und der Ankersonde mit einem Donor, ist im Rahmen des erfindungsgemäßen Verfahrens möglich.

Bei Bindung der Sensorsonde einerseits und der Ankersonde andererseits an den jeweiligen DNA-Strang werden - ohne sich auf diese Theorie beschränken zu wollen - Donor und Akzeptor somit in räumliche Nähe zueinander gebracht, um bei entsprechender Anregung ein FRET hervorzurufen, was zu einem detektierbaren Signal führt. Bei Ablösung der Sensorsonde bzw. der Ankersonde mit einhergehender räumlicher Beabstandung tritt auch bei Anregung kein FRET auf, so dass in diesem Fall auch kein FRET-Signal detektierbar ist. Das zugrundeliegende Prinzip des FRET bzw. der Signalausbildung mit dem spezifischen Einsatz von Donor- und Akzeptormolekülen kann somit als Maß für die Bindung von Sensor- bzw. Ankersonde an einen jeweiligen DNA-Abschnitt herangezogen werden. Dabei steigt die Fluoreszenz proportional mit der Konzentration komplementärer DNA an, d. h. je mehr Sensorsonden bzw. Ankersonden an die DNA des PCR-Ansatzes gebunden sind, desto stärker ist das Fluoreszenzsignal.

Gemäß einer ganz besonders bevorzugten Ausführungsform des einzusetzenden Sondenpaares kann es sich bei der Sensorsonde um eine LightCycler®-Sonde mit der zuvor beschriebenen Nukleotidsequenz TAATTCCTT-GATAGCGACGGG handeln, an die ein Fluoreszenzfarbstoff als Teil eines FRET-Paares gekoppelt ist (vlg. Sequenzprotokoll II, Sequence Listing, SEQ ID NO. 4). Bei der Ankersonde kann es sich ebenfalls um eine LightCycler-Sonde handeln, welche die Nukleotidsequenz ATTTTAACTTTCTCACCTT-CTGGGATCCAG aufweist und ebenfalls mit einem Fluoreszenzfarbstoff ausgerüstet ist (vlg. Sequenzprotokoll II, Sequence Listing, SEQ ID NO. 5).

Diese Sensorsonde ist aufgrund des gebundenen Fluoreszenzfarbstoffs insbesondere in der Lage, mit einer Ankersonde im Falle der Hybridisierung an den DNA-Einzelstrang des Wildtypgens bzw. Wildtypallels (wt-Sondenstrang) und/oder im Fall der insbesondere unspezifischen Hybridisierung an den korrespondierenden DNA-Einzelstrang des Mutationsgens (mt-Sondenstrang) ein detektierbares und/oder messbares Signal abzugeben.

Eine Messung des zu detektierenden Signals, welches bei Interaktion mit dem entsprechenden DNA-Einzelstrang auftritt, kann beispielsweise am Ende einer *Annealing-*Phase eines PCR-Zyklus stattfinden. Zudem kann die Fluoreszenz, wie nachfolgend geschildert, im Rahmen einer Schmelzkurvenanalyse ermittelt bzw. erfasst werden.

Im Falle der Detektion bzw. Analyse der genannten Deletionen in Exon 19 des EGF-Rezeptors, insbesondere an den Positionen 746 bzw. 747 der Aminosäuresequenz des EGF-Rezeptors, kann die Ankersonde in nicht beschränkender Weise die Nukleotid- bzw. Basensequenz ATTTTAACTTTCCACCTTC-TGGGATCCAG aufweisen oder hieraus bestehen.

Im Falle der Detektion bzw. Analyse der Mutation T790M kann die Ankersonde insbesondere die Nukleotid- bzw. Basensequenz CACGGTGGAGGT-GAGGCAGATGC aufweisen bzw. hieraus bestehen. Im Falle der Analyse bzw. Detektion der Mutation L858R umfasst die Nukleotid- bzw. Basensequenz der Ankersonde die Nukleotid- bzw. Basensequenz GCATGGTATT-CTTTCTCTTCCGCACCCAGC oder besteht hieraus.

Der Fachmann ist jederzeit in der Lage, die für die Detektion der jeweiligen Mutation speziell auszubildende bzw. einzusetzende Ankersonde entsprechend auszuwählen bzw. maßzuschneidern.

Was die vorliegende Erfindung weiter anbelangt, so kann es sich bei der Tumor- oder Krebserkrankung um ein Lungenkarzinom, insbesondere um ein nichtkleinzelliges Lungenkarzinom (*Non Small Cell Lung Cancer* bzw. NSCLC) und/oder um ein kleinzelliges Lungenkarzinom (*Small Cell Lung Cancer* bzw. SCLC), vorzugsweise um ein nichtkleinzelliges Lungenkarzinom (NSCLC), handeln.

Mit anderen Worten fokussiert die vorliegende Erfindung darauf, Mutationen nachzuweisen bzw. zu detektieren, die im Zusammenhang mit den vorgenannten Krebserkrankungen, insbesondere im Zusammenhang mit einem nichtkleinzelligen Lungenkarzinom, stehen bzw. diese Erkrankungen, beispielsweise durch eine abnorme Funktion des resultierenden Genproduktes des Mutationsgens, hervorrufen.

Die vorliegende Erfindung ist jedoch nicht auf die vorgenannten Erkrankungen beschränkt. Vielmehr kann das erfindungsgemäße Verfahren sozusagen universell zur Analyse bzw. Detektion von insbesondere bekannten Mutationen bzw. Genveränderungen, die im Zusammenhang mit Erkrankungen des Organismus als solchen stehen, eingesetzt werden. Dies kann beispielsweise über eine spezifische Anpassung der Hybridisierungssonden bzw. der Sensorsonden, der Primer und/oder des Blockierungsmittels realisiert werden, so dass das erfindungsgemäße Verfahren gewissermaßen für eine Vielzahl spezieller Erkrankungen maßgeschneidert werden kann.

Bei dem in Rede stehenden Protein, dessen Veränderung über die Erfassung bzw. Analyse der Mutation des für das Protein kodierenden Gens bestimmt werden kann, handelt es sich gemäß einer bevorzugten erfindungsgemäßen Ausführungsform um ein insbesondere humanes und/oder ein das Zellwachstum und/oder die Proliferation von Zellen regulierendes und/oder induzierendes Protein. Bei dem Protein kann es sich zudem um einen insbesondere transmembranen Rezeptor für Wachstumsfaktoren, insbesondere mit intrinsischer Tyrosinkinaseaktivität, handeln. Bevorzugt handelt es sich bei dem in Rede stehenden Protein, welches im Zusammenhang mit einer Tumor- oder Krebserkrankung steht, um den Epidermalen-Wachstumsfaktor-Rezeptor (*Epidermal-Growth-Factor-*Rezeptor bzw. EGF-Rezeptor), insbesondere wie zuvor beschrieben. Insbesondere handelt es sich im Rahmen der vorliegenden Erfindung um den humanen EGF-Rezeptor.

Die vorliegende Erfindung umfasst gleichermaßen auch strukturidentische bzw. strukturähnliche Proteine mit Wirkidentität bzw. Wirkähnlichkeit zu dem zuvor genannten EGF-Rezeptor. Der Begriff "EGF-Rezeptor", wie er im Rahmen der vorliegenden Erfindung verwendet wird, umfasst insbesondere auch funktionsidentische bzw. funktionsähnliche Abwandlungen und auch nicht unmittelbar mit der Krebs- bzw. Tumorerkrankung in Verbindung stehende Mutationsformen des EGF-Rezeptors an sich. Insbesondere umfasst der Begriff "EGF-Rezeptor" auch insbesondere zumindest im Wesentlichen funktionsähnliche bzw. funktionsidentische Isoformen und/oder Vorläufer bzw. Präkursoren. Gemäß einer erfindungsgemäß bevorzugten Ausführungsform handelt es sich bei dem in Rede stehenden Protein um den EGF-Rezeptor gemäß dem Locus bzw. gemäß der Referenznummer NP_005219.2 in Sequenzprotokoll I und/oder der in Sequenzprotokoll II (Sequence Listing) unter der SEQ ID NO. 1 gelisteten Aminosäuresequenz .

Im Allgemeinen umfasst die vorliegende Erfindung auch solche Formen des EGF-Rezeptors bzw. Proteine im Allgemeinen, welche eine Übereinstimmung in der Aminosäuresequenz von mindestens 90 %, insbesondere mindestens 95 %, vorzugsweise mindestens 98 %, bevorzugt mindestens 99 %, bezogen auf die Aminosäuresequenz des EGF-Rezeptors gemäß der Referenznummer NP_005219.2 in Sequenzprotokoll I und/oder gemäß der SEQ ID NO. 1 in Sequenzprotokoll II, aufweisen.

Dabei ist das Sequenzprotokoll I zu dem Sequenzprotokoll II in Bezug auf die unter NP_005219.2 bzw. SEQ ID NO. 1 aufgelisteten Aminosäuresequenz synonym bzw. inhaltsgleich. Der wesentliche Unterschied zwischen den Sequenzprotokollen besteht darin, dass das Sequenzprotokoll I auf Basis einer wissenschaftlich standardisierten Angabe bzw. Darstellung beruht, während das Sequenzprotokoll II auf Basis einer patentrechtlich standardisierten Angabe bzw. Darstellung unter Verwendung der Software PatentIn Version 3.3 erstellt wurde. Hierbei handelt es sich also in Bezug auf die Aminosäuresequenz um einen rein formal darstellungsgemäßen, nicht aber um einen inhaltlichen Unterschied.

Die im Rahmen des erfindungsgemäßen Verfahrens zu untersuchende bzw. zu analysierende Genveränderung, insbesondere Mutation, kann somit zu einer erhöhten und/oder übermäßigen Aktivität des von dem entsprechenden Gen kodierten Proteins führen und/oder hiermit im Zusammenhang stehen. Somit kann die Genveränderung bzw. Mutation zu einem erhöhten bzw. übermäßigen Zellwachstum und/oder zu einer erhöhten bzw. übermäßigen Zellproliferation führen bzw. hiermit im Zusammenhang stehen. Die zu untersuchende Genveränderung kann zu einer pathologischen bzw. von der physiologischen Norm abweichenden Aktivität des von dem entsprechenden Gen kodierten Proteins, insbesondere des EGF-Rezeptors, führen. Mit anderen Worten bewirkt die Genveränderung, insbesondere Mutation, eine Änderung in dem entsprechenden Genprodukt, welche im Zusammenhang mit der Tumor- bzw. Krebserkrankung steht. Bei der Erkrankung, welche im Zusammenhang mit der Genveränderung steht, handelt es sich insbesondere, wie zuvor angeführt, um ein Bronchialkarzinom, insbesondere um NSCLC.

Bei der Genveränderung kann es sich insbesondere um eine Genveränderung in Exon 18, Exon 19, Exon 20 oder Exon 21, vorzugsweise in Exon 19, des Epidermalen-Wachstumsfaktor-Rezeptors (*Epidermal-Growth-Factor-*Rezeptor bzw. EGF-Rezeptor) handeln.

Insbesondere kann es sich bei der Genveränderung um eine Genveränderung, insbesondere Deletion, in Exon 19 und/oder im Bereich der Aminosäurereposition 746 und/oder 747 des Epidermalen-Wachstumsfaktor-Rezeptors (*Epidermal-Growth-Factor-Rezeptor* bzw. EGF-Rezeptor) handeln. Wie zuvor angeführt, kann es sich bei der Deletion um den Wegfall vollständiger Codons auf Basis von drei Nukleotiden bzw. Basen handeln. Gleichermaßen ist aber auch der Wegfall einzelner Basen, beispielsweise von einer Base oder von zwei Basen möglich.

Weiterhin kann es sich bei der in Rede stehenden Genveränderung um eine Deletion, insbesondere in Exon 19 des Epidermalen-Wachstumsfaktor-Rezeptors (*Epidermal-Growth-Factor-Rezeptor* bzw. EGF-Rezeptor), handeln, wobei die Deletion ausgewählt werden kann aus der Gruppe der Deletionen ΔE746-A750, ΔE746-T751, ΔE746-A750 (ins RP), ΔE746-T751 (ins A/I), ΔE746-T751 (ins VA), ΔE746-S752 (ins A/V), ΔL747-E749 (A750P), ΔL747-A750 (ins P), ΔL747-T751, ΔL747-T751 (ins P/S), ΔL747-S752, ΔL747-S752 (E746V), ΔL747-S752 (P746V), ΔL747-S752 (ins Q), ΔL747-P753, ΔL747-P753 (ins S) und ΔS752-I759.

Erfindungsgemäß können somit Genveränderungen, die zu einer Deletion, insbesondere in Exon 19 des EGF-Rezeptors, führen und/oder hiermit im Zusammenhang stehen, detektiert werden. Gleichermaßen ist die Detektion von solchen Genveränderungen möglich, die zu einem Austausch mindestens einer Aminosäure in Exon 20 und/oder Exon 21 des EGF-Rezeptors führen bzw. hiermit im Zusammenhang stehen. In diesem Zusammenhang können beispielsweise solche Genveränderungen detektiert werden, welche zu einem Austausch von Serin an Position 768 des EGF-Rezeptors, insbesondere durch Isoleucin (S768I), führen bzw. hiermit im Zusammenhang stehen. Gleichermaßen können auch Genveränderungen detektiert werden, die zu einem Austausch von Threonin an Position 790 des EGF-Rezeptors, insbesondere durch Methionin (T790M), führen bzw. hiermit im Zusammenhang stehen. Zu dem können auch solche Genveränderungen detektiert werden, die zu einem Austausch von Leucin an Position 858 des EGF-Rezeptors, insbesondere durch Arginin (L858R), führen bzw. hiermit im Zusammenhang stehen.

Wie zuvor angeführt, ist die vorliegende Erfindung nicht auf die Detektion bzw. Erfassung von Mutationen in dem vorgenannten EGF-Rezeptor beschränkt. Gleichermaßen können Genveränderungen, insbesondere in Form von Deletionen bzw. Insertionen, für eine Vielzahl weiterer Proteine, welche insbesondere im Zusammenhang mit Krebs- bzw. Tumorerkrankungen stehen, detektiert werden.

In diesem Zusammenhang kann die Tumor- und/oder Krebserkrankung im Zusammenhang mit einer Genveränderung, insbesondere Translokation, in dem für das Fusionsprotein EML4-ALK kodierenden Gen stehen Somit kann die Genveränderung eine Genveränderung, insbesondere Translokation, in dem für das Fusionsprotein EML4-ALK kodierenden Gen darstellen.

Darüber hinaus kann es sich im Rahmen der vorliegenden Erfindung bei der Tumor- bzw. Krebserkrankung auch um eine Leukämie, insbesondere eine akute myeloische Leukämie (AML), handeln. Diesbezüglich kann die Tumor- bzw. Krebserkrankung im Zusammenhang mit einer Genveränderung, insbesondere Insertion, in dem für die Rezeptortyrosinkinase FLT3 kodierenden Gen stehen. Demnach kann die Genveränderung eine Genveränderung, insbesondere Insertion, in dem für die Rezeptortyrosinkinase FLT3 kodierenden Gen darstellen. Hierbei handelt es sich insbesondere um die zuvor angeführten *Internat Tandem Duplication (ITD).*

Somit stehen neben der bereits erwähnten Deletion, insbesondere im Falle des nichtkleinzelligen Bronchialkarzinoms, auch klinisch verwandte Beispiele weiterer Genveränderungen, die mit Krebserkrankungen assoziiert sind, im Fokus der vorliegenden Erfindung. Ein Beispiel für relevante Translokationen stellt das zuvor beschriebene Fusionsgen bzw. Fusionsprotein EML4-ALK dar, dessen Auftreten ebenfalls mit dem nichtzelligen Bronchialkarzinom assoziiert ist. Neben den Karzinomen der Lunge sind auch weitere maligne Erkrankungen mit dem Auftreten von Genveränderungen assoziiert, welche im Rahmen des erfindungsgemäßen Verfahrens detektiert werden können, insbesondere solche hämatologischer Art. Ein Beispiel hierfür ist die zuvor angeführte akute myeloische Leukämie (AML), welche eine maligne Erkrankung des blutbildenden Systems - also der Hämatopoese - insbesondere der Myelopoese ist. Die Myelopoese bezeichnet den Teil der Hämatopoese, welcher die Differenzierung von pluripotenten Zellen zu Granulozyten, Erythrozyten, Thrombozyten, Makrophagen und Mastzellen umfasst. Die akute myeloische Leukämie (AML) führt zu einer massiven Vermehrung unreifer Vorstufen - also von nicht vollständig differenzierten Formen der bereits erwähnten Zelltypen - im Knochenmark und häufig auch im Blut. Ähnlich dem Zusammenhang zwischen Bronchialkarzinomen und Genveränderungen des für den EGF-Rezeptor kodierenden Gens, kann auch im Fall von AML eine Assoziation des Auftretens der Krankheit mit der Genveränderung des FLT3-Gens, welches für eine Fns-ähnliche Tyrosinkinase aus der Familie der Klasse III-Tyrosinkinase-Rezeptorproteine kodiert, nachgewiesen werden. So zeigen etwa 30 % bis 40 % der Erwachsenen AML-Patienten Veränderungen des FLT3-Gens. Bei den für AML-relevanten Genveränderungen, welche im Rahmen des erfindungsgemäßen Verfahrens detektiert werden können, kann es sich sowohl um *Internal Tandem Duplications (ITD)* des für die Juxtamembran-Domäne kodierenden Genabschnitts als auch um ITD handeln, die sich in Bereichen außerhalb der Juxtamembran-Domäne befinden.

Was das erfindungsgemäße Verfahren weiterhin anbelangt, so ist es vorgesehen, dass das Verfahren nach der Erfindung mittels einer asymmetrischen Polymerase-Kettenreaktion (*Polymerase Chain Reaction* bzw. PCR) unter Verwendung mindestens einer Sensorsonde und mindestens einer Ankersonde und mindestens eines die Anbindung der Sensorsonde an die Wildtypgene inhibierenden wildtypspezifischen Blockierungsmittels durchgeführt wird. Dabei kann im Rahmen der vorliegenden Erfindung insbesondere derart vorgegangen werden, dass eine selektive Vermehrung bzw. Amplifizierung derjenigen DNA-Einzelstränge auch des Mutationsgens (mt-Sondenstränge) und des Wildtypgens (wt-Sondenstränge), an welchen die Sensorsonde zu interagieren bzw. wechselzuwirken, insbesondere hieran zu binden, imstande ist, stattfindet.

Im Rahmen der vorliegenden Erfindung ist es zudem vorgesehen, dass die asymmetrische PCR in Gegenwart von Primern, insbesondere in Form von Oligonukleotiden, durchgeführt wird.

In diesem Zusammenhang werden die Primer ausgewählt derart, dass im Rahmen der PCR eine Amplifizierung des die Genveränderung aufweisenden Genabschnitts des Mutationsgens bzw. Mutationsallels erfolgt bzw. dass eine Amplifizierung des zu dem die Genveränderung aufweisenden Genabschnitt des Mutationsgens korrespondierenden Genabschnitts der Wildtypgene bzw. der Wildtypallels erfolgt. Der Begriff "der zu dem die Genveränderung aufweisende Genabschnitt des Mutationsgens korrespondierende Genabschnitt des Wildtypgens" ist dabei insbesondere so zu verstehen, dass der zu dem Abschnitt mit der Mutation entsprechende Abschnitt des Wildtypgens bzw. Wildtypallele, bei welchem als solchen keine Mutation vorliegt, gemeint ist.

Durch die Auswahl der Primer ist eine gezielte Amplifizierung bzw. Vermehrung des für die Untersuchung bzw. die Genanalyse relevanten Genabschnitts bzw. des hierzu korrespondierenden Genabschnitts des Wildtypgens und somit eine Diskriminierung gegenüber den übrigen Genen bzw. der übrigen DNA in der Probe möglich. Insbesondere handelt es sich bei der zu amplifizierenden DNA um das Gen des zuvor beschriebenen EGF-Rezeptors bzw. vorzugsweise um einen Genabschnitt des für das EGF-Protein kodierenden Gens, und zwar insbesondere um denjenigen Genabschnitt, in welchem die Mutation vorliegen kann. Die konkrete Auswahl der diesbezüglich einzusetzenden Primer stellt den Fachmann vor keine Schwierigkeiten, und der Fachmann ist jederzeit in der Lage, die entsprechenden Primer auszuwählen und einzusetzen.

Die im Rahmen der vorliegenden Erfindung eingesetzten bzw. verwendeten Primer können ausgewählt werden derart, dass eine Amplifizierung des die Deletion insbesondere in Exon 19 des EGF-Rezeptors aufweisenden Genabschnitts des Mutationsgens, insbesondere an Position 746 und/oder 747, erfolgt. Gleichermaßen kann es im Rahmen der vorliegenden Erfindung vorgesehen sein, dass die Primer ausgewählt werden derart, dass eine Amplifizierung des den Austausch von Serin an Position 768 des EGF-Rezeptors insbesondere durch Isoleucin (S768I), aufweisenden Genabschnitts des Mutationsgens erfolgt. Gemäß einer weiteren erfindungsgemäßen Ausführungsform können die Primer ausgewählt werden derart, dass eine Amplifizierung des den Austausch von Threonin an Position 790 des EGF-Rezeptors, insbesondere durch Methionin (T790M), aufweisenden Genabschnitts des Mutationsgens erfolgt und/oder dass eine Amplifizierung des den Austausch von Leucin an Position 858 des EGF-Rezeptors, insbesondere durch Arginin (L858R), aufweisenden Genabschnitts des Mutationsgens erfolgt.

Zudem kann es diesbezüglich gegebenenfalls möglich sein, dass gleichzeitig eine Amplifizierung des zu dem die Genveränderung aufweisenden Genabschnitt des Mutationsgens korrespondierenden Genabschnitts der Wildtypgene erfolgen kann, insbesondere aufgrund der Eigenschaften der Primer als solche.

Was die zu amplifizierenden Genabschnitte anbelangt, so wird die diesbezügliche Größe bzw. die Anzahl an Basenpaaren ausgewählt derart, dass die Sensorsonde sowie die gleichermaßen an den Genabschnitt bindende Ankersonde an den entsprechenden DNA-Einzelsträngen der jeweiligen Genabschnitte zu binden imstande sind.

Im Rahmen der vorliegenden Erfindung ist es vorgesehen, dass ein erster Primer und ein hiervon verschiedener zweiter Primer eingesetzt werden. In diesem Zusammenhang bindet der erste Primer spezifisch an den DNA-Einzelstrang des Mutationsgens (wt-Sondenstrang), an welchem die Sensorsonde zu binden bzw. zu hybridisieren imstande ist bzw. mit welchem die Sensorsonde zu interagieren imstande ist. Gleichermaßen kann der erste Primer aufgrund der Natur der Primer auch an dem hierzu korrespondierenden DNA-Einzelstrang des Wildtypgens binden. Bei dem ersten Primer handelt es sich somit gewissermaßen um einen *sense-*Primer*.* Was den zweiten Primer anbelangt, so bindet dieser spezifisch an dem zum Sondenstrang komplementären DNA-Einzelstrang des Mutationsgens (mt-Komplementärstrang). Bei dem zweiten Primer handelt es sich gewissermaßen um einen *antisense-*Primer. Der zweite Primer kann zudem an dem diesbezüglich korrespondierenden DNA-Einzelstrang des Wildtypgens binden.

Im Falle der speziellen Detektion bzw. Analyse der angeführten Deletionen in Exon 19 des EGF-Rezeptors sollten der erste und zweite Primer derart ausgewählt werden, dass spezifisch der die Mutation aufweisende Genabschnitt des Mutationsgens bzw. des korrespondierenden Wildtypgens im Rahmen der PCR amplifiziert wird; entsprechendes gilt für die Analyse bzw. Detektion der Mutation Mutation T790M bzw. L858R.

Im Falle der Analyse bzw. Detektion von Deletionen im Bereich der Aminosäureposition 746 bzw. 747 in Exon 19 des EGF-Rezeptors kann der erste Primer in nicht beschränkender Weise insbesondere die Basensequenz bzw. Nukleotidsequenz GGGCCTGAGGTTCAGAGC (vgl. Sequenzprotokoll II, Sequence Listing, SEQ ID NO. 3) oder CACACAGCAAAGCAGAAACTCA aufweisen bzw. aus dieser Nukleotidsequenz bestehen. Im Falle des zweiten Primers kann dieser in nicht beschränkender Weise die Nukleotidsequenz GTCTTCCTTCTCTCTCTGTCATAGGG (vgl. Sequenzprotokoll II, Sequence Listing, SEQ ID NO. 2) oder ATCTCACAATT-GCCAGTTAACGTCT aufweisen bzw. hieraus bestehen.

Im Fall der Analyse bzw. Detektion der Mutation T790M kann der erste Primer in nicht beschränkender Weise insbesondere die Basenabfolge bzw. Nukleotidsequenz GACTCCGACTCCTCCTTTATCCAATG aufweisen bzw. aus dieser Nukleotidsequenz bestehen. Im Falle des zweiten Primers kann dieser in nicht beschränkender Weise die Nukleotidsequenz CACACACCAGTT-GAGCAGGTA aufweisen bzw. hieraus bestehen.

Im Falle der Analyse bzw. Detektion der Mutation L858R kann der erste Primer die Nukleotidsequenz GCTCAGAGCCTGGCATGAA aufweisen bzw. hieraus bestehen; der zweite Primer kann die Nukleotidsequenz CATCCTCCCCTGCATGTGT aufweisen bzw. hieraus bestehen.

Die jeweiligen Primer umfassen insbesondere auch solche Primer, welche eine vergleichbare Spezifität bzw. Selektivität in Bezug auf die jeweiligen Genabschnitte aufweisen. Dies ist dem Fachmann aber an sich bekannt, und der Fachmann ist jederzeit in der Lage, vor dem Hintergrund der Amplifizierung der entsprechenden Genabschnitte die jeweils spezifischen Primer auszuwählen. Die Primer sollten ausgewählt werden derart, dass diese selbst außerhalb des Bereichs der zu analysierenden Mutation des Mutationsgens bzw. der hierzu korrespondierenden Abschnitte des Wildtypgens bzw. den diesbezüglichen DNA-Einzelsträngen binden.

Im Rahmen der vorliegenden Erfindung ist es vorgesehen, dass die asymmetrische PCR durchgeführt wird derart, dass der DNA-Einzelstrang, an welchem die Sensorsonde (Sondenstrang) zu binden imstande ist, stärker bzw. häufiger amplifiziert wird als der hierzu komplementäre DNA-Einzelstrang (Komplementärstrang), so dass nach Amplifizierung im PCR-Ansatz mehr Kopien des Sondenstranges im Vergleich zu dem Komplementärstrang vorliegen. Dies erfolgt im Rahmen der Erfindung dadurch, dass der erste Primer und der zweite Primer ausgewählt werden derart, dass die Menge und/oder die Konzentration des ersten Primers, insbesondere bezogen auf den PCR-Ansatz, größer ist als die Menge und/oder Konzentration des zweiten Primers, so dass eine erhöhte und/oder verstärkte Amplifikation des Sondenstranges gegenüber dem Komplementärstrang erfolgt. Auf diese Weise kommt es zu einer verstärkten Amplifizierung des Sondenstranges gegenüber dem komplementären DNA-Einzelstrang, was dazu führt, dass der DNA-Einzelstrang, an welchem die Sensorsonde zu binden imstande ist, in einer höheren Kopienanzahl vorliegt, so dass aufgrund der größeren Anzahl an Ereignissen in Bezug auf die Bindung zwischen Sensorsonde einerseits und entsprechendem DNA-Einzelstrang andererseits eine Verstärkung bzw. Amplifizierung des mutationsspezifischen Sensorsondensignals erfolgen kann.

In diesem Zusammenhang ist es im Rahmen der vorliegenden Erfindung bevorzugt, wenn das mengenmäßige Verhältnis des ersten Primers zu dem zweiten Primer (erster Primer : zweiter Primer), insbesondere in dem PCR-Ansatz, im Bereich von 1.000 : 1 bis 1,05 : 1, insbesondere 100 : 1 bis 1,5 : 1, vorzugsweise 10 : 1 bis 2 : 1, liegt.

Dementsprechend ist es bevorzugt, wenn nach erfolgter Amplifizierung die Anzahl bzw. Konzentration des Sondenstranges gegenüber dem Komplementärstrang um einen Faktor von mindestens 1,1, insbesondere 1,5, vorzugsweise 2, bevorzugt 10, besonders bevorzugt 100, erhöht ist.

Wie zuvor angeführt, wird durch das spezielle Verhältnis der Primer mit der Bevorzugung des ersten Primers erreicht, dass im Rahmen der PCR-basierten Amplifikation vorrangig der DNA-Einzelstrang des Mutationsgens bzw. des Mutationsallels amplifiziert bzw. vermehrt wird. Auf diese Weise wird das mutationsspezifische Signal zusätzlich verstärkt.

Die Anmelderin hat weiterhin in völlig überraschender Weise gefunden, dass die Nachweisgrenze bzw. Sensitivität des erfindungsgemäßen Verfahrens zur Detektion bzw. Analyse bestimmter Mutationen dadurch weiter signifikant erhöht werden kann, dass die asymmetrische PCR sowie die diesbezügliche Verwendung wildtypspezifischer Hybridisierungssonden in spezieller Kombination mit dem Einsatz mindestens eines Blockierungsmittels durchgeführt wird. In völlig überraschender Weise kann hierdurch eine weitere Erhöhung der Sensitivität erreicht werden, wobei die Gesamtheit der erfindungsgemäß vorgesehenen Maßnahmen - Einsatz spezifischer Sensorsonden, asymmetrische PCR sowie Verwendung spezieller Blockierungsmittel - über die Wirkung der Einzelmaßnahmen hinausgeht, so dass die erfindungsgemäß realisierten Maßnahmen in völlig überraschender Weise synergistisch im Hinblick auf die Verbesserung der Sensitivität des erfindungsgemäßen Verfahrens wirken.

Im Rahmen der vorliegenden Erfindung ist es vorgesehen, dass das Blockierungsmittel ausgewählt wird derart, dass das Blockierungsmittel eine höhere Spezifität bzw. Bindungsaffinität bzw. Selektivität in Bezug auf den DNA-Einzelstrang des Wildtypgens bzw. Wildtypallels - insbesondere in Bezug auf den Bereich des DNA-Einzelstrangs des Wildtypgens bzw. Wildtypallels, welcher zu dem die Genveränderung aufweisenden Genabschnitt des Mutationsgens bzw. Mutationsallels korrespondiert (wildtypischer DNA-Strang) - gegenüber dem entsprechenden Mutationsgen bzw. dem diesbezüglichen DNA-Einzelstrang mit der Genveränderung bzw. Mutation (mutierter DNA-Strang) aufweist. Mit anderen Worten wird das Blockierungsmittel derart ausgewählt, dass es mit höherer Spezifität bzw. Selektivität an den wildtypischen DNA-Strang und insbesondere an der zu dem mutierten DNA-Einzelstrang korrespondierenden Position bzw. Stelle bindet, um auf diese Weise das Anbinden der Sensorsonde an den wildtypischen DNA-Strang zu verringern bzw. zu verhindern. Blockierungsmittel einerseits und Sensorsonde andererseits verhalten sich somit hinsichtlich der Bindungsstellen kompetitiv, wobei die Bindung der Sensorsonde an den wildtypischen DNA-Strang verringert bzw. unterbunden wird. Auf diese Weise kann das zu detektierende Signal der Sensorsonde in Bezug auf die zu erfassende Mutation weiter diskriminiert bzw. verstärkt werden, da weniger Sensorsonden an den wildtypischen DNA-Strang binden, sofern das Blockierungsmittel in dem PCR-Ansatz vorhanden ist. In Bezug auf den mutierten DNA-Strang weist die Sensorsonde eine höhere Affinität auf als das Blockierungsmittel, so dass die Bindung der Sensorsonde an den mutierten DNA-Strang zumindest im Wesentlichen nicht von dem Blockierungsmittel beeinflusst bzw. unterbunden wird.

In diesem Zusammenhang weist die Bindung und/oder der Komplex des Blockierungsmittels mit dem DNA-Einzelstrang des Wildtypgens eine höhere Stabilität, insbesondere einen höheren Schmelzpunkt, auf als die Bindung bzw. der Komplex der Sensorsonde mit dem Einzelstrang des Mutationsgens. Dies wird im Rahmen der vorliegenden Erfindung dadurch gewährleistet, dass die Nukleotidsequenz des Blockierungsmittels komplementär zu dem dem Mutationsbereich des mutierten DNA-Einzelstrangs entsprechenden nichtmutierten Bereich des wildtypischen DNA-Einzelstrangs ist.

In diesem Zusammenhang wird das Blockierungsmittel gemäß einer erfindungsgemäßen bevorzugten Ausführungsform ausgewählt derart, dass es ein Nukleotidmolekül, insbesondere ein Oligo- oder Polynukleotid, in Form einer blockierten Nukleinsäure *(Locked Nucleic Acid* bzw. LNA) aufweist oder hieraus besteht. Dabei sollte das Blockierungsmittel eine Größe von 3 bis 30 bp, insbesondere 5 bis 25 bp, vorzugsweise 10 bis 20 bp, aufweisen.

Wie zuvor angeführt, wird das Blockierungsmittel erfindungsgemäß ausgewählt derart, dass das Blockierungsmittel komplementär zu dem Bereich des DNA-Einzelstrangs des Wildtypgens bzw. Wildtypallels ist, welcher mit dem die Genveränderung aufweisenden Genabschnitt des Mutationsgens bzw. Mutationsallels korrespondiert. Der Bereich des wildtypischen DNA-Einzelstranges, welcher zu dem die Genveränderung aufweisenden Genabschnitt des Mutationsgens bzw. zu dem die Mutation aufweisenden Abschnitt des DNA-Einzelstranges korrespondiert, stellt somit gewissermaßen den zu dem Mutationsbereich analogen Abschnitt des wildtypischen DNA-Stranges des Wildtypgens dar, an welchem das Blockierungsmittel bzw. der Blocker spezifisch zu binden imstande ist.

Durch den Einsatz des spezifischen Blockierungsmittels wird somit im Ergebnis die Bindung der Sensorsonde an den Wildtyp-Strang inhibiert bzw. vermindert, so dass nur bzw. vorrangig der mutierte DNA-Strang im Rahmen der PCR vervielfältigt wird und die eingesetzten Sonden konkurrenzlos an die mutierte DNA binden können. Das Blockierungsmittel verhindert bzw. vermindert somit einerseits die Amplifizierung des wildtypischen DNA-Stranges sowie andererseits die Bindung der Sensorsonde an den wildtypischen DNA-Strang, insbesondere im Sinne einer kompetitiven bzw. konkurrierenden Bindung, was das Messergebnis weiter verbessert.

Das Blockierungsmittel sollte somit zu diesem Zweck im Allgemeinen so beschaffen sein, dass es eine sehr hohe Bindungsstärke gegenüber dem Wildtyp-Einzelstrang aufweist und erst bei sehr hohen Temperaturen abschmilzt. In diesem Zusammenhang sollte das Blockierungsmittel ausgewählt werden derart, dass das Blockierungsmittel verbrückte Nukleinsäuren aufweist, in welchen der Zuckeranteil, insbesondere der Riboseanteil, chemisch modifiziert ist, insbesondere wobei der Riboseanteil eine Sauerstoff/Methylen-Brücke, vorzugsweise am C₂- und C₄-Atom des Riboseanteils, aufweist.

Erfindungsgemäß ist das Blockierungsmittel ausgewählt derart, dass das Blockierungsmittel ein Nukleinsäure-Analogon in Form einer blockierten Nukleinsäure *(Locked Nucleic Acid* bzw. LNA) ist. Bei derartig blockierten Nukleinsäuren handelt es sich um insbesondere strukturell weniger flexible und somit um drehsteife Moleküle, welche an den DNA-Strang unter Ausbildung eines erhöhten Schmelzpunktes spezifisch binden bzw. hybridisieren.

Im Falle der Detektion bzw. Analyse der genannten Deletionen in Exon 19 des EGF-Rezeptors, insbesondere an den Positionen 746 bzw. 747 der Aminosäuresequenz des EGF-Rezeptors kann das Blockierungsmittel in Form eines LNA die Nukleotidsequenz TAATTCCTTGATA aufweisen oder hieraus bestehen (vgl. Sequenzprotokoll II, Sequence Listing, SEQ ID NO. 6).

In diesem Zusammenhang sollte in Bezug auf die Detektion bzw. Analyse der Mutation T790M das Blockierungsmittel in Form eines LNA die Nukleotidsequenz TGAGCTGCGTGATG aufweisen oder hieraus bestehen; hinsichtlich der Detektion bzw. Analyse der Mutation L858R sollte das Blockierungsmittel, insbesondere in Form eines LNA, die Nukleotidsequenz GCCAGCCCAAAATCT aufweisen oder aus dieser Nukleotidsequenz bestehen.

Zum Nachweis bzw. zur Deletion der Genveränderung wird im Anschluss an die Polymerase-Kettenreaktion eine Schmelzkurve aufgenommen bzw. eine Schmelzkurvenanalyse durchgeführt. In diesem Zusammenhang kann die Abspaltung bzw. Dehybridisierung, insbesondere der Sensorsonde, von dem jeweiligen DNA-Einzelstrang des Mutationsgens bzw. des Wildtypgens erfasst werden, insbesondere wobei die Erfassung photometrisch durch Messung der Fluoreszenz erfolgen kann. Gleichermaßen kann über die Schmelzpunkte und/oder den Schmelzpunktbereich der Schmelzkurve auf das Vorliegen einer Mutation geschlossen werden. Eine Ablösung der Sensorsonde führt dabei zu einer Abnahme bzw. Verringerung des detektierbaren Signals, beispielsweise im Falle eines FRET-Paares unter Verwendung einer Ankersonde durch Beabstandung der das FRET-Paar ausbildenden detektierbaren Stoffe der Sensor-bzw. Ankersonde. Wie zuvor angeführt, geschieht dies im Falle der Mutation im Vergleich zum Wildtyp bei niedrigeren Temperaturen.

Das erfindungsgemäße Verfahren wird somit derart durchgeführt, dass zum Nachweis bzw. zur Detektion der Mutation im Anschluss an die Polymerase-Kettenreaktion eine Schmelzkurve aufgenommen bzw. eine Schmelzkurvenanalyse durchgeführt wird. In diesem Zusammenhang können die Reaktionsansätze langsam erhitzt werden, beispielsweise bis auf 95 °C. An dem Punkt bzw. bei der Temperatur, an dem sich 50 % der Sensorsonden von den entstandenen PCR-Produkten gelöst haben, fällt die Fluoreszenz deutlich ab. Sofern die Sensorsonde spezifisch an den Wildtyp- DNA-Einzelstrang gebunden hat, erfolgt die Dehybridisierung der Sensorsonde bei einer höheren Temperatur im Vergleich zu einer Bindung an der entsprechenden Mutationsbereich bzw. dem entsprechenden Mutations-DNA-Einzelstrang. Die entsprechende Analyse der Schmelzkurven lässt somit Rückschlüsse auf das Vorhandensein einer spezifischen Mutation zu.

Diesbezüglich kann die Abspaltung bzw. Dehybridisierung, insbesondere der Sensorsonde, von dem jeweiligen DNA-Einzelstrang des Mutationsgens bzw. des Wildtypgens erfasst werden, insbesondere wobei die Erfassung photometrisch, insbesondere durch Messung der Fluoreszenz, erfolgt. Wie zuvor dargestellt, führt die Hybridisierung bzw. die Abspaltung der Sensorsonde von dem jeweiligen DNA-Einzelstrang zu einer Abnahme der Fluoreszenz, wobei der diesbezügliche Schmelzpunkt aufgrund der höheren Bindungsstärke bzw. Affinität in Bezug auf den Wildtyp-DNA-Einzelstrang höher ausfällt als in Bezug auf den DNA-Einzelstrang mit der entsprechenden Mutation. Durch den Einsatz der Sensorsonden kann demnach festgestellt werden, ob in einer Probe mutierte Genformen vorliegen. Zur besseren Analyse der Schmelzkurven können diese in Form der ersten mathematischen Ableitung dargestellt werden; insbesondere stellen die Maxima der betreffenden ersten mathematischen Ableitung die jeweiligen Schmelzpunkte dar.

Im Rahmen des erfindungsgemäßen Verfahrens kann somit derart vorgegangen werden, dass anhand der Schmelzpunkte und/oder Schmelzpunktbereiche der Schmelzkurve auf das Vorliegen einer Genveränderung, insbesondere Mutation, geschlossen wird. Mit anderen Worten kann auf Basis der Analyse bzw. Zuordnung (d. h. Zuordnung über eine Referenz, wie nachfolgend noch beschrieben) der Schmelzpunkte bzw. Schmelzbereiche festgestellt werden, ob und bejahendenfalls welche konkrete Mutation in der Probe vorliegt.

In diesem Zusammenhang kann beispielsweise derart vorgegangen werden, dass im Rahmen der Schmelzkurvenanalyse parallele Vergleichs- bzw. Referenzansätze mitgeführt bzw. analysiert werden oder aber alternativ diese Vergleichs- bzw. Referenzansätze vorab und/oder unabhängig vermessen worden sind (d. h. mit anderen Worten das Verfahren über eine Referenz standardisiert wird). So kann z. B. mindestens ein Referenzansatz auf Basis einer wildtypischen DNA bzw. eines Wildtypgens bzw. -allels in einem insbesondere parallelen Ansatz mitgeführt bzw. vorab vermessen bzw. standardisiert werden. Der Referenzansatz kann ohne Blockierungsmittel mitgeführt bzw. vorgelegt werden, d. h. es kann ein weiterer Referenzansatz des Wildtypgens ohne Blockierungsmittel mitgeführt werden. Gleichermaßen kann es vorgesehen sein, dass mindestens ein Referenzansatz auf Basis einer zu analysierenden Genveränderung bzw. Mutation und/oder einer definierten bzw. vorbekannten Genveränderung bzw. Mutation in einem insbesondere parallelen Ansatz mitgeführt wird bzw. vorab vermessen bzw. standardisiert wird; hierbei kann es sich insbesondere um solche Referenzansätze handeln, welche Mutationsgene bzw. Mutationsallele auf Basis der bereits angeführten Deletionen in Exon 19 des EGF-Rezeptors enthalten.

Ein Vergleich der anhand der zu analysierenden Proben einerseits, und der anhand der Referenzansätze bzw. -messungen erhaltenen Schmelzkurven andererseits, ermöglicht dann eine Aussage über das Vorliegen einer speziellen Mutation in der Probe (d. h. Prüfung auf Vorliegen einer Genveränderung bzw. Mutation und bejahendenfalls der Art der Genveränderung bzw. Mutation). So kann im Allgemeinen dann von dem Vorliegen einer Genveränderung bzw. Mutation ausgegangen werden, wenn sich in Bezug auf den Referenzansatz mit dem Wildtyp unterschiedliche, insbesondere bei niedrigeren Temperaturen auftretende Schmelzpunkte bzw. Schmelzbereiche für die zu untersuchende Probe ergeben. Das Vorliegen einer Genveränderung bzw. Mutation kann auch anhand eines Vergleichs der Schmelzkurve mit derjenigen Probe des jeweiligen Referenzansatzes mit dem betreffenden Mutationsgen erfolgen, wobei das Vorliegen von zumindest im Wesentlichen gleichen Schmelzpunkten bzw. -bereichen als Anzeichen für das Vorhandensein gleicher Genveränderungen bzw. Mutationen herangezogen werden kann.

Grundsätzlich ist es im Rahmen der vorliegenden Erfindung gemäß einer weniger bevorzugten Ausführungsform aber auch möglich, die Analyse auf das Vorliegen einer Mutation ohne Referenzansatz durchzuführen, insbesondere bei bereits bekannten Schmelzpunkten bzw. -bereichen oder aber bei vorab erfolgter Standardisierung (insbesondere wie zuvor beschrieben).

Wie zuvor angeführt, kann es erfindungsgemäß vorgesehen sein, dass das Mutationsgen bzw. die Mutationsgene einerseits und das Wildtypgen bzw. die Wildtypgene andererseits in einer Probe und/oder einem Ensemble vorliegen, insbesondere wobei die bereitgestellte Probe und/oder das Ensemble von einem Patienten stammt und/oder insbesondere wobei die Probe und/oder das Ensemble von einem Körpermaterial, insbesondere einer Körperflüssigkeit, vorzugsweise Blut und/oder Gewebsflüssigkeit und/oder Lymphflüssigkeit und/oder Urin mit insbesondere zellulären Bestandteilen, stammt bzw. hieraus gewonnen werden kann. Zudem kann auch bereitgestelltes Zellmaterial bzw. Tumormaterial eingesetzt werden, beispielsweise auf Basis einer Biopsie.

Im Allgemeinen kann die dem erfindungsgemäßen Verfahren zugrundeliegende Probe derart sein, dass sie sowohl gesunde bzw. nichtmaligne Zellen mit intaktem EGF-Rezeptor sowie den diesbezüglich kodierenden Wildtypgenen, als auch Tumorzellen mit gegebenenfalls mutiertem EGF-Rezeptor und den diesbezüglich zugehörigen Mutationsgenen bzw. Mutationsallelen aufweist. Beispielsweise kann es sich bei der Probe um eine solche handeln, welche neben intakten Zellen auch zirkulierende Tumorzellen aufweist.

Die Probe bzw. das Ausgangsmaterial kann zudem vor Durchführung des erfindungsgemäßen Verfahrens weiter aufgereinigt werden bzw. die DNA aus der Probe zur weiteren Verwendung isoliert bzw. aufkonzentriert bzw. aufgereinigt werden. Die diesbezüglichen Methoden sind dem Fachmann hinlänglich bekannt, so dass es hierzu keiner weiteren Ausführung bedarf.

Im Rahmen der vorliegenden Erfindung ist es dabei möglich, dass Genveränderungen bzw. Mutationen auf Basis einer Probe bestimmt werden können, welche weniger als 1.000, insbesondere weniger als 500, vorzugsweise weniger als 200, besonders bevorzugt weniger als 100 Tumorzellen pro ml Probe aufweisen. Das erfindungsgemäße Verfahren kann auf Basis von Proben bzw. Ausgangsmaterialien mit DNA durchgeführt werden, welche weniger als 1 %, insbesondere weniger als 0,1 %, vorzugsweise weniger als 0,01 %, bevorzugt weniger als 0,001 % Tumorzellen, bezogen auf den Gesamtzellgehalt der Probe, aufweisen.

Das erfindungsgemäße Verfahren kann in Bezug auf eine Probe bzw. ein Ausgangsmaterial angewendet werden kann, welche(s) weniger als 1 % mutierte DNA, insbesondere etwa 0,5 %, vorzugsweise etwa 0,05 %, bevorzugt etwa 0,005 %, besonders bevorzugt etwa 0,0005 % mutierte DNA, bezogen auf die Gesamt-DNA in der Probe bzw. in dem Ausgangsmaterial, aufweist. Somit handelt es sich bei dem erfindungsgemäßen Verfahren um ein hochsensitives und effizientes Verfahren, bei dem selbst geringste Spuren an DNA hinsichtlich des Vorliegens von Mutationen aussagekräftig analysiert werden können.

Nachfolgend wird eine Analyse bzw. Auswertung rein exemplarisch und nicht beschränkend auf Basis einer Versuchsdurchführung mit einem parallelen Wildtyp-Referenzansatz weiter veranschaulicht:
Was die Analyse der Schmelzkurven anbelangt, so wird im Allgemeinen die Schmelzkurve mit der niedrigsten spezifischen Schmelztemperatur bzw. dem höchsten Schmelztemperaturbereich dem Mutationsgen zugeordnet, d. h. das Vorliegen verschiedener Schmelzkurven mit unterschiedlichen Schmelzpunkten bzw. -bereichen kann als Beleg bzw. Indiz für das Vorliegen einer Mutation herangezogen werden, wobei die Kurve mit dem höheren Schmelzpunkt bzw. Schmelzbereich auf die festere Bindung der Sensorsonde an den Wildtyp-DNA-Einzelstrang und somit auf die nichtmutierte Form zurückzuführen ist. Zudem kann bei Vorliegen mehrerer Schmelzkurven die Schmelzkurve mit der niedrigeren spezifischen Schmelztemperatur bzw. dem niedrigeren Schmelztemperaturbereich dem Mutationsgen zugeordnet werden, wobei die diesbezügliche Schmelzkurve mit der niedrigeren Schmelztemperatur bzw. dem niedrigeren Schmelztemperaturbereich ursächlich durch die weniger stark ausgebildete und unspezifische Bindung der Sensorsonde an den mutierten Abschnitt des wildtypischen DNA-Einzelstranges hervorgerufen wird. Sofern in der zu analysierenden Probe auch wildtypische DNA vorhanden ist, kann sich für die Schmelzkurve der genveränderten bzw. mutierten Form gleichermaßen ein bei höheren Temperaturen vorliegender Schmelzpunkt bzw. -bereich ausbilden, welcher aufgrund der erfindungsgemäß durchgeführten Maßnahmen im Allgemeinen jedoch ein geringeres Signalmaximum aufweist (z. B. Unterdrückung der Anbindung der Sensorsonde und/oder Unterdrückung der Amplifizierung).

Zudem ist es im Rahmen der vorliegenden Erfindung neben der zuvor angeführten Ermittlung bzw. Analyse, ob eine Mutation in einer Probe vorliegt und um welche Mutation es sich hierbei handelt, gleichermaßen möglich, eine Aussage darüber zu treffen, ob es sich bei der Mutation um eine homozygote bzw. heterozygote Genveränderung handelt. Hierbei sollte in Bezug auf die Probe von einem zumindest im Wesentlichen nicht mit Wildtypgenen verunreinigten Ansatz ausgegangen werden, welcher das zu analysierende Mutationsgen enthält. So kann es im Rahmen des erfindungsgemäßen Verfahrens vorgesehen sein, dass die Schmelzkurve mit dem niedrigsten Schmelzpunkt und/oder dem niedrigsten Schmelzbereich einer homozygoten Genveränderung zugeordnet wird für den Fall, dass die Schmelzkurve einen einzigen Schmelzpunkt bzw. Schmelzbereich aufweist. Zudem kann im Rahmen des erfindungsgemäßen Verfahrens derart vorgegangen werden, dass die Schmelzkurve mit dem niedrigsten Schmelzpunkt bzw. dem niedrigsten Schmelzbereich einer heterozygoten Genveränderung zugeordnet wird für den Fall, dass die Schmelzkurve zwei voneinander verschiedene Schmelzpunkte bzw. Schmelzbereiche aufweist.

Zum Nachweis von homozygoten bzw. heterozygoten Genveränderungen in einer Probe, welche auch das Wildtypgen enthält, kann beispielsweise derart vorgegangen werden, dass ein erster PCR-Ansatz der Probe ohne Blockierungsmittel und ein zweiter paralleler PCR-Ansatz mit Blockierungsmittel, gegebenenfalls mit einem weiteren Referenzansatz auf Basis des Wildtypgens, durchgeführt und analysiert werden. Über einen Vergleich der Ausbildung der Schmelzpunkte bzw. -bereiche insbesondere hinsichtlich der entsprechenden Signalmaxima der Wildtypgene bzw. Wildtypallele in den Proben mit bzw. ohne Blockierungsmittel kann eine Analyse auf das Vorliegen einer homozygot bzw. heterozygot ausgebildeten Mutation erfolgen.

Insbesondere aufgrund der spezifischen Kombination aller erfindungsgemäßen Maßnahmen ist es im Rahmen der vorliegenden Erfindung möglich, dass eine mindestens 10fache, insbesondere mindestens 50fache, vorzugsweise mindestens 100fache, bevorzugt mindestens 500fache, besonders bevorzugt mindestens 1.000fache Verstärkung bzw. Amplifizierung der Detektion in Bezug auf den die Genveränderung aufweisenden Genabschnitt, insbesondere DNA-Abschnitt, des Mutationsgens bzw. des damit einhergehenden (Fluoreszenz-)Signals erfolgt. Insbesondere ist es im Rahmen der vorliegenden Erfindung möglich, dass eine mindestens 10fache, insbesondere mindestens 50fache, vorzugsweise mindestens 100fache, bevorzugt mindestens 500fache, besonders bevorzugt mindestens 1.000fache Verstärkung und/oder Amplifizierung des mit dem die Genveränderung aufweisenden Genabschnitt, insbesondere DNA-Abschnitt, des Mutationsgens im Zusammenhang stehenden Messsignals erfolgt.

Im Ergebnis ist es im Rahmen der vorliegenden Erfindung gelungen, auf Basis der Kombination der zuvor genannten Maßnahmen einen äußerst sensitiven Mutationsnachweis bereitzustellen. Im Rahmen der vorliegenden Erfindung kann eine Mutation analysiert bzw. nachgewiesen werden, selbst wenn in der Probe nur sehr geringe Mengen an mutierter DNA vorliegen.

Das erfindungsgemäße Verfahren eignet sich gleichermaßen dazu, Verlaufskontrollen auf Basis des der Probe zugrundeliegenden Körpermaterials, insbesondere vorzugsweise peripherem Blut, durchzuführen, was eine leichte Nachverfolgung des Erkrankungsverlaufes auf molekularer Ebene ermöglicht. Auf Basis des erfindungsgemäßen Verfahrens, gegebenenfalls in Kombination mit weiteren Aufreinigungsmethoden, welche insbesondere auf eine spezifische Aufreinigung bzw. Isolation der zu untersuchenden DNA aus der Probe abstellen und welche dem Fachmann als solche wohlbekannt sind, ist es im Rahmen der vorliegenden Erfindung möglich, zirkulierende Tumorzellen in Vollblut nachzuweisen.

Das erfindungsgemäße Verfahren eignet sich somit dazu, Aussagen zur Diagnose der Tumor- bzw. Krebserkrankung bzw. zur Ermittlung des Risikos, an einer Tumor- bzw. der Krebserkrankung zu erkranken, bzw. zur Prognose eines Krankheitsverlaufs der Tumor- bzw. Krebserkrankung bzw. zur Prognose von individuellen Arzneimittelwirkungen bei Behandlung der Tumor- und/oder Krebserkrankung zu treffen. So kann beispielsweise und in nicht beschränkender Weise einem Patienten bzw. Probanden ein erhöhtes Risiko zugeordnet werden, an einem Bronchialkarzinom zu erkranken, sofern dieser eine oder mehrere der vorgenannten Mutationen aufweist, welche auf Basis des erfindungsgemäßen Verfahrens nachgewiesen werden können. Gleichermaßen kann eine Krankheitsprognose bzw. ein Krankheitsverlauf aufgenommen bzw. analysiert werden, wobei diesbezüglich dem Patienten bzw. Probanden über einen bestimmten Zeitraum Proben entnommen und auf Basis des erfindungsgemäßen Verfahrens analysiert werden können und der Nachweis bzw. die Detektion der entsprechenden Mutationen für das Vorliegen von Tumorzellen in der Probe herangezogen werden können.

Gleichermaßen ist es im Rahmen der vorliegenden Erfindung möglich, auf Basis der konkreten Analyse der zugrundeliegenden Mutation den Therapieansatz, insbesondere in Bezug auf die spezifische Medikation, zu optimieren. So können auf Basis der vorgefundenen Mutationen die jeweils spezifischen bzw. diesbezüglich optimal wirkenden Medikamente eingesetzt werden, was zu einer weiterführenden Individualisierung und Spezifizierung der Medikation bzw. Therapie, einhergehend mit einer höheren therapeutischen Wirksamkeit, führt. So kann bei Vorliegen der ΔE746-A750-Mutation und/oder der T790M-Mutation, gegebenenfalls in Kombination mit der L858R-Mutation, die Applikation bzw. Verabreichung der zuvor beschriebenen Inhibitoren der zweiten Generation indiziert sein, während bei Vorliegen der L858R-Mutation die Gabe der zuvor beschriebenen Inhibitoren der ersten Generation indiziert sein kann.

Wie zuvor angeführt, kann das erfindungsgemäße Verfahren zur Mutationsanalyse bei Bronchialkarzinomen und insbesondere bei dem nichtkleinzelligen Lungenkarzinom bzw. NSCLC mit der diesbezüglichen Involvierung des EGF-Rezeptors eingesetzt werden.

Die vorliegende Erfindung betrifft somit - gemäß einem bevorzugten Aspekt der vorliegenden Erfindung - ein Verfahren zum Nachweis bzw. zur Detektion einer Genveränderung, insbesondere Mutation, in einem für den EGF-Rezeptor kodierenden Gen, wobei der EGF-Rezeptor insbesondere im Zusammenhang mit einer Tumor- bzw. Krebserkrankung, insbesondere einem Lungenkarzinom, wie dem nichtkleinzelligen Lungenkarzinom (NSCLC), steht, insbesondere wobei das die Genveränderung aufweisende Gen (Mutationsgen) zusammen mit weiteren, für das Protein kodierenden, jedoch keine Genveränderung aufweisenden Genen (Wildtypgenen) vorliegt. Das Verfahren gemäß diesem Aspekt der vorliegenden Erfindung zeichnet sich gleichermaßen dadurch aus, dass es mittels asymmetrischer Polymerase-Kettenreaktion (*Polymerase Chain Reaction* bzw. PCR) unter kombinierter Verwendung mindestens einer detektierbaren wildtypspezifischen und nur unspezifisch an das Mutationsgen bindenden Hybridisierungssonde (Sensorsonde) einerseits und mindestens eines die Anbindung der Sensorsonde an die Wildtypgene inhibierenden wildtypspezifischen Blockierungsmittels sowie einer Ankersonde andererseits durchgeführt wird, so dass eine selektive Verstärkung und/oder Amplifizierung der Detektion in Bezug auf einen die Genveränderung aufweisenden Genabschnitt, insbesondere DNA-Abschnitt, des Mutationsgens erfolgt.

Die erfindungsgemäßen Verfahren gemäß den obigen Aspekten können als solche auf Basis von dem Fachmann an sich bekannten Methoden unter Einbezug der diesbezüglichen Apparaturen und Messeinrichtungen durchgeführt werden. Beispielsweise kann die Polymerase-Kettenreaktion sowie die Aufnahme und Analyse der entsprechenden Schmelzkurven unter Verwendung einer LightCycler®-480-Apparatur der Firma F. Hoffmann-La Roche Ltd. durchgeführt werden.

Das erfindungsgemäße, zuvor beschriebene Verfahren auf Basis einer asymmetrischen Polymerase-Kettenreaktion (*Polymerase Chain Reaction* bzw. PCR) zur Detektion mindestens einer Mutation in einem Gen, vorzugsweise in einem Gen, welches für ein mit einer Tumor- und/oder Krebserkrankung in Verbindung stehendes Protein kodiert, insbesondere wobei das die Genveränderung aufweisende Gen (Mutationsgen) zusammen mit weiteren, für das Protein kodierenden, jedoch keine Genveränderung aufweisenden Genen (Wildtypgenen) vorliegt, kann mit einer nachfolgend beschriebenen Zusammensetzung durchgeführt werden, wobei die Zusammensetzung in Kombination enthält:
(a) eine detektierbare wildtypspezifische Hybridisierungssonde (Sensorsonde), wie zuvor definiert;
(b) einen ersten Primer, welcher spezifisch an dem DNA-Einzelstrang des Mutationsgens (mt-Sondenstrang), mit welchem die Sensorsonde zu interagieren imstande ist, bindet;
(c) einen zweiten Primer, welcher spezifisch mit dem zum Sondenstrang komplementären DNA-Einzelstrang des Mutationsgens (mt-Komplementärstrang) zu interagieren imstande ist;
(d) ein die Anbindung der Sensorsonde an die Wildtypgene inhibierendes wildtypspezifisches Blockierungsmittel in Form einer *Locked Nucleic Acid* (LNA);
wobei der Gehalt und/oder die Menge des ersten Primers (b) in der Zusammensetzung größer ist als der Gehalt und/oder die Menge des zweiten Primers (c).

Was die Zusammensetzung anbelangt, so kann diese (a) die Hybridisierungssonde bzw. Sensorsonde in einer Menge von 0,01 bis 5 pmo/µl, insbesondere 0,05 bis 3 pmo/µl, vorzugsweise 0,1 bis 1 pmo/µl, bezogen auf die Zusammensetzung, enthalten.

Zudem kann die Zusammensetzung (b) den ersten Primer in einer Konzentration von 0,05 bis 10 pmo/µl, insbesondere 0,1 bis 5 pmo/µl, vorzugsweise 0,2 bis 2 pmo/µl, bezogen auf die Zusammensetzung, enthalten.

Weiterhin kann die Zusammensetzung (c) den zweiten Primer in einer Konzentration von 0,005 bis 5 pmo/µl, insbesondere 0,01 bis 2 pmo/µl, vorzugsweise 0,03 bis 0,5 pmol/µl, bezogen auf die Zusammensetzung, enthalten.

Die Zusammensetzung sollte (a) den ersten Primer und (b) den zweiten Primer in einem mengenmäßigen Verhältnis des (a) ersten Primers zu (b) dem zweiten Primer ((a) : (b)), bezogen auf die Zusammensetzung, im Bereich von 1.000 : 1 bis 1,05 : 1, insbesondere 100 : 1 bis 1,5 : 1, vorzugsweise 10 : 1 bis 2 : 1, enthalten.

Die Zusammensetzung kann zudem (d) das Blockierungsmittel in einer Konzentration von 0,005 bis 4 pmo/µl, insbesondere 0,01 bis 1 pmo/µl, vorzugsweise 0,015 bis 0,1 pmo/µl, bezogen auf das Gesamtvolumen der Zusammensetzung, enthalten.

Zudem kann die Zusammensetzung insbesondere eine (e) Ankersonde, insbesondere wie zuvor definiert, enthalten, insbesondere in einer Menge von 0,01 bis 5 pmo/µl, insbesondere 0,05 bis 3 pmo/µl, vorzugsweise 0,1 bis 1 pmo/µl, bezogen auf die Zusammensetzung.

Bei der Zusammensetzung handelt es sich insbesondere um eine wässrige Lösung bzw. Dispersion. In diesem Zusammenhang kann die Zusammensetzung insbesondere PCR-reines Wasser enthalten. Zudem kann die Zusammensetzung so genanntes *480-probes-Master* enthalten.

Die vorliegende Zusammensetzung kann anwendungsfertig zubereitet bzw. portioniert sein sowie zur Aufbewahrung bzw. Lagerung gekühlt bzw. eingefroren werden.

Gleichermaßen ist es auch möglich, dass die Zusammensetzung zumindest teilweise als räumlich voneinander getrennte Komponenten, insbesondere als *Kit-of-Parts,* vorliegt, wobei die Komponenten (a) bis (d) und gegebenenfalls (e) zumindest teilweise voneinander getrennt vorliegen können. Diesbezüglich können die Komponenten bzw. Bestandteile beispielsweise unmittelbar vor Versuchsdurchführung zum Erhalt einer anwendungsfertigen Zusammensetzung zusammengeführt werden.

Mit anderen Worten ist die zuvor beschriebene Zusammensetzung zur Verwendung in dem erfindungsgemäßen Verfahren zur Detektion mindestens einer Mutation in einem Gen, vorzugsweise in einem für ein mit einer Tumor- und/oder Krebserkrankung in Verbindung stehenden Protein kodierenden Gen, insbesondere wobei das die Genveränderung aufweisende Gen (Mutationsgen) zusammen mit weiteren, für das Protein kodierenden, jedoch keine Genveränderung aufweisenden Genen (Wildtypgenen) vorliegt, auf Basis einer asymmetrischen Polymerase-Kettenreaktion geeignet.

Die vorliegende Erfindung wird anhand der Figurendarstellung weiter und rein exemplarisch veranschaulicht, ohne die Erfindung jedoch hierauf zu beschränken. Es zeigen:
- Fig. 1: eine schematische Darstellung der Verteilung bzw. Anordnung der Exons des humanen EGF-Rezeptor-Gens in dem hiervon kodierten Protein sowie eine Übersicht der mit einer Arzneimittel-Resistenz bzw. -Sensitivität in Verbindung stehen Mutationen im EGF-Rezeptor;
- Fig. 2: das erfindungsgemäße Nachweisprinzip von Mutationen unter Verwendung von Hybridisierungssonden auf Basis von Sensorsonden, welche spezifisch für die Wildtyp-Sequenz des korrespondierenden Mutationsgens sind, einerseits und Ankersonden andererseits, sowie die Ausbildung eines FRET-Signals;
- Fig. 3: das Prinzip der erfindungsgemäß realisierten asymmetrischen PCR;
- Fig. 4: das Prinzip der Inhibierung des Wildtyps bzw. der Wildtyp-DNA durch das erfindungsgemäß eingesetzte Blockierungsmittel;
- Fig. 5: spezifische Schmelzkurven zur Detektion bzw. zum Nachweis einer Exon 19-Deletion ohne Zugabe eines Blockierungsmittels;
- Fig. 6: spezifische Schmelzkurven zur Detektion bzw. zum Nachweis einer Exon 19-Deletion unter erfindungsgemäßer Verwendung eines Blockierungsmittels;
- Fig. 7: eine Übersicht von erfindungsgemäß einsetzbaren *Primer,* Sonden sowie des wildtypspezifischen Blockierungsmittels und eine schematische Darstellung der Bindestellen der eingesetzten bzw. getesteten Primer-Paare sowie der eingesetzten Sonden.

Fig. 1 zeigt eine schematische Darstellung der Exon-Verteilung des humanen EGF-Rezeptor-Gens auf die funktionellen Domänen im korrespondierenden Protein sowie eine Übersicht der mit der Ausbildung bzw. dem Vorhandensein einer Arzneimittel-Resistenz bzw. -Sensitivität in Verbindung stehenden Mutationen (vgl. Sharma et al. "Epidermal growth factor rezeptor mutations in lung"; Nature Reviews, 2007, 7: 169-181). Dabei bezieht sich die Bezeichnung *"EGF binding"* auf den Bereich der Ligandenbindungstasche im korrespondierenden Protein, "*TM*" auf die Transmembrandomäne, "*nucleotidebinding-loop"* auf den Nukleotid-Bindungsbereich bzw. die Nukleotid-Bindungsschleife und *"activation loop"* auf den Aktivierungsbereich bzw. die Aktivierungsschleife. Ein Teil der dargestellten Mutationen stehen mit einer Arzneimittel-Resistenz hinsichtlich der Behandlung von Lungenkrebs in Verbindung, während ein anderer Teil der dargestellten Mutationen mit einer Veränderung der Arzneimittel-Sensitivität assoziiert ist. Etwa 45 % aller bekannten Mutationen des EGF-Rezeptors, die in Verbindung mit dem nichtkleinzelligen Lungenkarzinom stehen, sind im Bereich von Exon 19 lokalisiert. Die in Fig. 1 zu Exon 19 des EGF-Rezeptors angeführten Mutationen stellen Deletionen - mit Schwerpunkt ab der Aminosäure 746 oder 747 - dar.

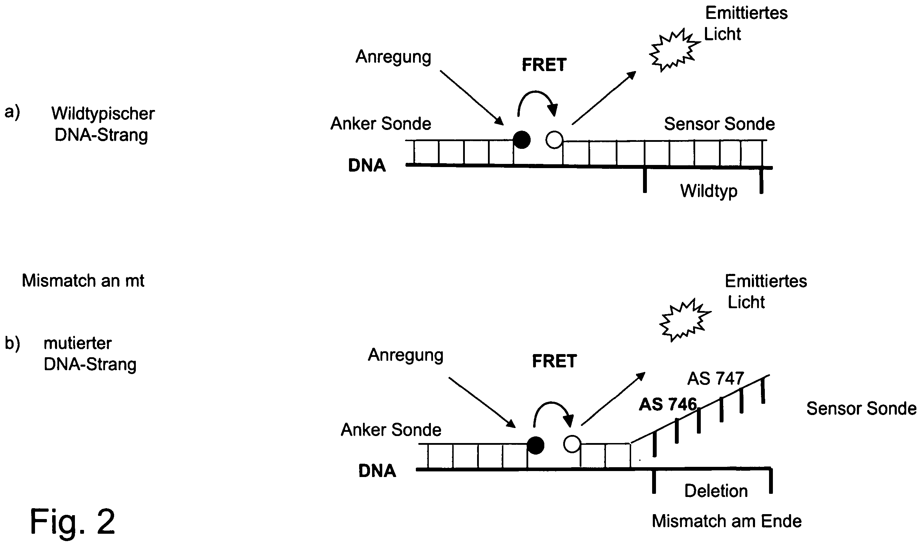

Weiterhin verdeutlicht Fig. 2 das erfindungsgemäße Nachweisprinzip unter Verwendung spezieller Hybridisierungssonden. In diesem Zusammenhang zeigt Fig. 2 a) die Bindung der jeweiligen Hybridisierungssonden - nämlich Sensorsonde einerseits und Ankersonde andererseits - an einen DNA-Einzelstrang ohne Genveränderung und somit an den Wildtyp. Hier bindet die Sensorsonde spezifisch in dem Bereich, dessen korrespondierender Bereich im Mutationsgen die Mutation, insbesondere die Deletion, enthält. Durch Anregung des Fluoreszenzfarbstoffs der Sensorsonde und Übertragung der Energie mittels FRET auf den Farbstoff der Ankersonde wird ein Signal emittiert, welches erfasst bzw. gemessen werden kann. Damit der Energietransfer stattfinden kann, ist es erfindungsgemäß von besonderem Vorteil, wenn der Basenabstand zwischen den Sonden nicht mehr als 5 bp beträgt. Der Fluoreszenzfarbstoff der Sensorsonde wird durch Licht angeregt, die Energie mittels FRET (Fluoreszenz Resonanz-Energie-Transfer) auf den Fluoreszenzfarbstoff der Ankersonde übertragen, und das emittierte Licht kann gemessen werden. Fig. 2 b) zeigt die Bindung der beiden Hybridisierungssonden an einen DNA-Einzelstrang mit Genveränderung bzw. Mutation, insbesondere in Form einer Deletion. Da die Sensorsonde spezifisch an den Wildtyp-Strang bindet bzw. komplementär zu dem Wildtyp-Strang ist, kann sie in diesem Fall an den mutierten Strang bzw. Mutationsstrang nur unvollständig bzw. nur abschnittsweise bzw. unspezifisch hybridisieren. Auch in diesem Fall kann bei vorhandener Bindung zunächst ein FRET stattfinden. Aufgrund der unvollständigen bzw. nur abschnittsweisen bzw. unspezifischen Bindung der Sensorsonde an den Wildtyp-Strang erfolgt im Vergleich zu dem Wildtyp-Strang bei Erwärmung eine frühzeitigere Ablösung der Sensorsonde von dem Mutationsstrang, so dass das FRET-Signal in Bezug auf den Mutationsstrang abnimmt bzw. verschwindet (nicht dargestellt).

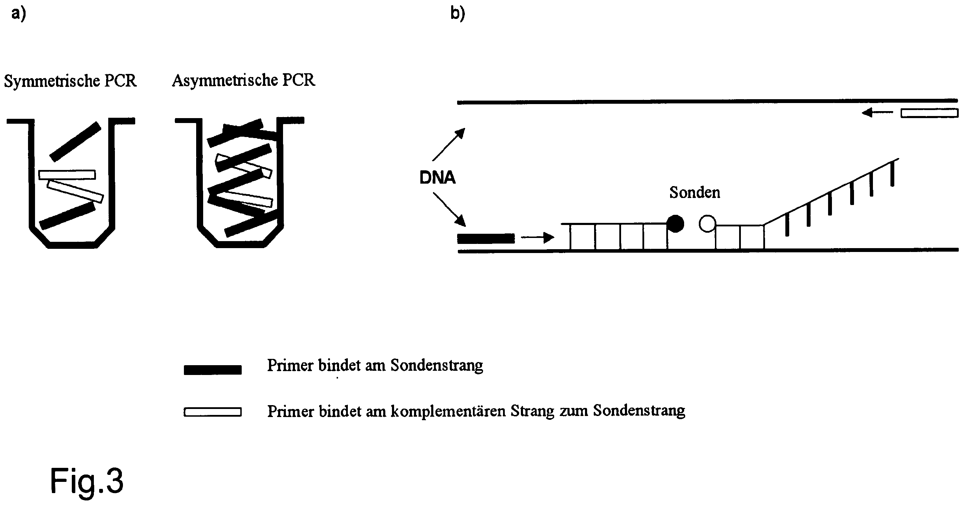

Weiterhin verdeutlicht Fig. 3 das Prinzip der erfindungsgemäß realisierten asymmetrischen PCR. In diesem Zusammenhang zeigt Fig. 3 a) dass - im Gegensatz zur symmetrischen PCR - das Primerverhältnis bei der asymmetrischen PCR nicht gleich ist. Die Konzentration des Primers, der am Sondenstrang bindet, wird erhöht (hier schwarz dargestellt). Fig. 3 b) zeigt eine Übersicht, wonach der schwarz markierte Primer an den Sondenstrang bindet, während der weiß dargestellte Primer an den komplementären DNA-Einzelstrang bindet.

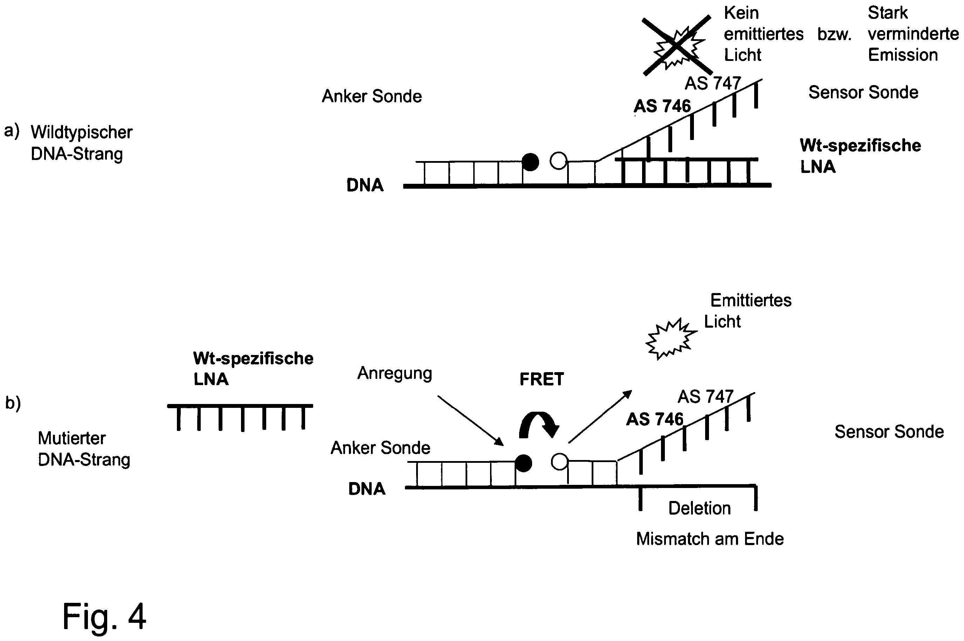

Fig. 4 veranschaulicht das Prinzip der Inhibierung des Wildtyp-DNA-Einzelstrangs durch das erfindungsgemäß eingesetzte Blockierungsmittel. Fig. 4 a) zeigt die Bindung des für den Wildtyp (wt) spezifischen Blockierungsmittels an den Wildtyp-DNA-Einzelstrang. Somit kann die Sensorsonde an dieser Stelle zumindest im Wesentlichen nicht mehr hybridisieren. Die wt-DNA wird zudem nicht mehr amplifiziert und emittiert durch das fehlende FRET auch kein Signal. Fig. 4 b) zeigt die Situation, wonach an dem mutierten DNA-Einzelstrang das Blockierungsmittel nicht binden kann, jedoch die Sensorsonde. Die mutierte DNA wird amplifiziert und emittiert ein Signal, insbesondere in Form von Licht, welches gemessen werden kann.

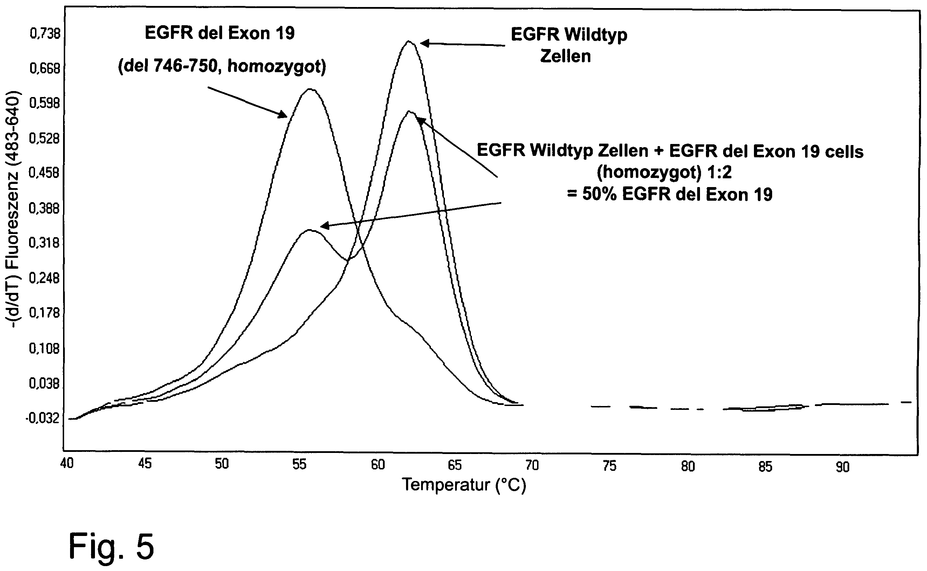

Fig. 5 zeigt eine Darstellung spezifischer Schmelzkurven auf Basis einer homozygoten Exon 19_del_746-750-Mutation, einer 1:2-Mischung der homozygoten Exon 1_del_746-750-Mutation und des Wildtyps unter Verwendung einer wildtypspezifischen Sensorsonde und ohne Zugabe eines Blockierungsmittels. Nach der eigentlichen PCR findet eine Schmelzkurvenanalyse statt. Dabei wird die Abnahme der Fluoreszenz, bedingt durch die Ablösung der FRET-Sonden vom DNA-Strang aufgrund der schrittweisen Temperaturerhöhung, in Abhängigkeit von der Temperatur gemessen. Zur besseren visuellen Darstellung wird die erste mathematische Ableitung der Schnittkurve berechnet, so dass der Wendepunkt der gemessenen Fluoreszenz als Kurve dargestellt wird. Der höchste Punkt bzw. das Maximum der Kurve entspricht dabei der spezifischen Schmelztemperatur. Die Kurve mit dem Maximum bei etwa 65 °C zeigt eine wildtypische Probe, deren Schmelzpunkt aufgrund der wildtypspezifischen Sensorsonde gegenüber der Mutante erhöht ist. Die Kurve mit dem Maximum bei etwa 55 °C zeigt die Exon 19_del_746-750-Mutation-Probe an, die im Vergleich zu Wildtypzellen einen niedrigeren Schmelzpunkt aufweist. Die 1:2-Mischung von Wildtyp und Exon 19_del_746-750-Mutation zeigt - ähnlich einer heterozygoten Mutante - einen "Doppelpeak" bzw. zwei Schmelzpunkte, da sie sowohl den Schmelzpunkt bzw. den Schmelzpunktbereich des Wildtyps als auch den der Exon 19_del_746-750-Mutation aufweist.

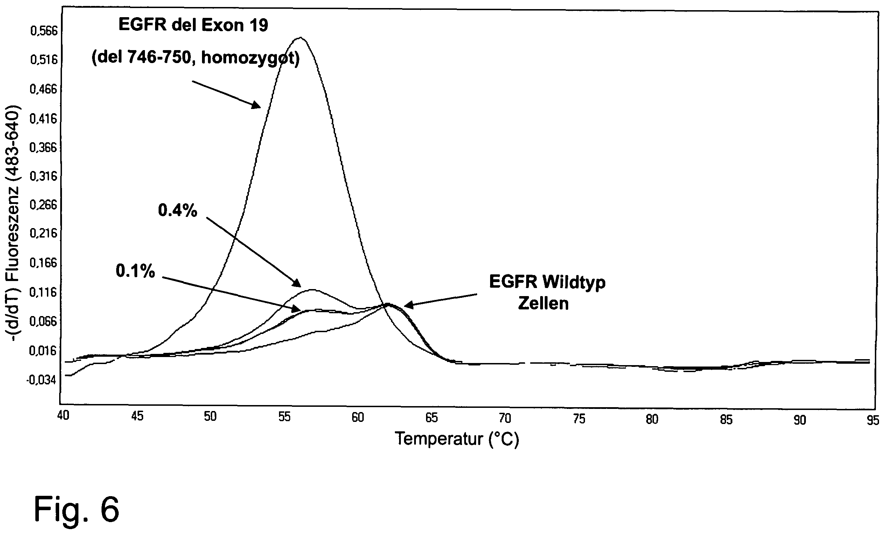

Fig. 6 zeigt eine Darstellung der spezifischen Schmelzkurven einer homozygoten Exon 19_del_746-750-Mutation und ihrer 0,1%eigen und 0,4%igen Verdünnungen sowie des Wildtyps, jeweils unter Zugabe eines wildtypspezifischen Blockierungsmittels, welches die Bindung der Sensorsonde an den Wildtyp-Sondenstrang des EGF-Rezeptor-Gens verhindert bzw. zumindest vermindert. Nach der eigentlichen PCR findet eine Schmelzkurvenanalyse statt. Dabei wird die Abnahme der Fluoreszenz, bedingt durch die Ablösung der FRET-Sonden vom DNA-Strang aufgrund der schrittweisen Temperaturerhöhung, in Abhängigkeit von der Temperatur gemessen. Zur besseren visuellen Darstellung wird die erste mathematische Ableitung der Schnittkurve berechnet, so dass der Wendepunkt der gemessenen Fluoreszenz als Kurve dargestellt wird. Der höchste Punkt bzw. das Maximum der Kurve entspricht dabei der spezifischen Schmelztemperatur. Die Kurven mit dem Maximum bei etwa 55 °C zeigen die Exon 19_del_746-750-Mutation-Probe sowie deren Verdünnungen. Durch die Durchführung der PCR unter Zugabe eines Blockierungsmittels geht das Wildtyp-Signal weitgehend zurück. Hierdurch ist ein effektiver Nachweis von Mutationen auch bei starken Verdünnungen, wie sie beispielsweise in einer biologischen Probe bzw. einer Probandenprobe vorkommen, möglich.

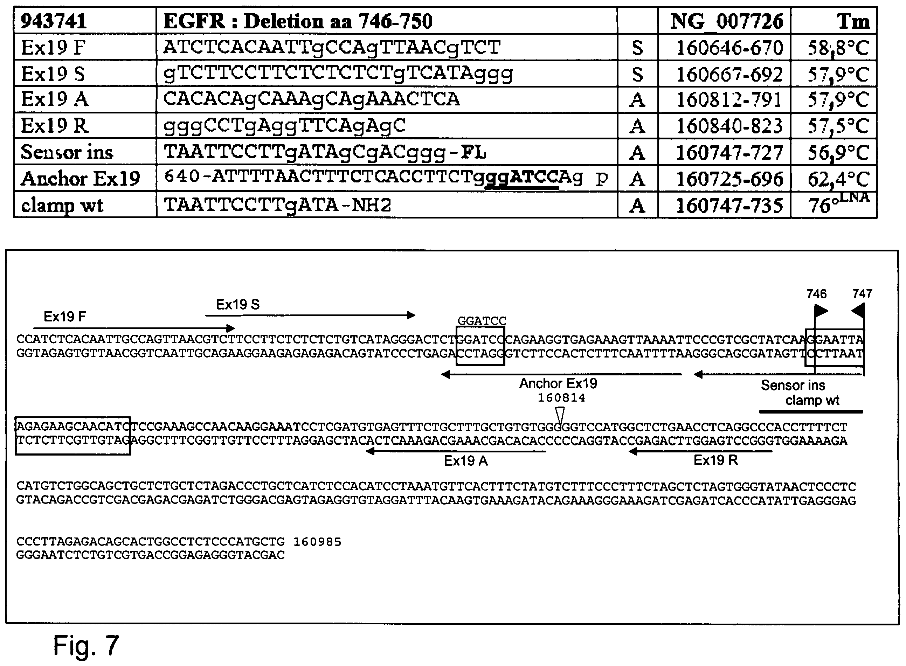

Schließlich zeigt Fig. 7 eine Übersicht von erfindungsgemäß einsetzbaren Primern (*Ex19F, Ex19S, Ex19A, Ex19R*), Sonden (*Sensor ins, Anchor Ex19*) sowie des wildtypspezifischen Blockierungsmittels (*clamp wt*) und eine schematische Darstellung der Bindestellen der eingesetzten bzw. getesteten Primerpaare sowie der eingesetzten Sonden. Derartige Primer und Hybridisierungssonden können beispielsweise von der TIB MOLBIOL GmbH, Berlin, Deutschland, bezogen werden. Gekennzeichnet sind die Bindestellen der Primer *Ex19F, Ex19S, Ex19A* und *Ex19R,* von denen eine Kombination aus *Ex19 S* und *Ex19 R* erfindungsgemäß bevorzugt ist, sowie der Sensorsonde *Sensor ins* und der Ankersonde *Anchor Ex19,* an der DNA-Sequenz des EGF-Gens.

Die vorliegende Erfindung wird nachfolgend weiterführend und nicht beschränkend veranschaulicht:
Im Zusammenhang mit der vorliegenden Erfindung konnte gezeigt werden, dass durch EGFR-Inhibitoren die Aktivierung des EGF-Rezeptors (EGFR) gehemmt werden kann. Über 80 % der Mutationen des EGF-Rezeptors bei NSCLC-Patienten beruhen auf verschiedenen Deletionen in Exon 19, wie der Deletion ΔE746-A750, sowie auf einer Punktmutation in Exon 20, nämlich L858R (Austausch der Aminosäure Leucin L an Position 858 durch Arginin R, vgl. Fig. 1). Patienten mit einem Lungentumor, welche eine dieser Veränderungen aufweisen, sind somit besonders geeignet für eine Therapie mit EGFR-Inhibitoren.

Obwohl die Therapie im Allgemeinen gut vertragen wird und sehr spezifisch wirksam ist, entwickeln die meisten Patienten nach einer gewissen Zeit eine so genannte Sekundärmutation, die zusätzlich zu der bereits vorhandenen Mutation auftreten kann und zur Resistenz gegenüber Erlotinib und Gefitinib führt. In etwa 50 % bis 65 % dieser Fälle findet man die Mutation T790M (Austausch der Aminosäure Threonin T durch Methionin M an Position 790, vgl. Fig. 1). Für diese Patienten stehen Medikamente zur Verfügung, deren Wirkmechanismus sich von denen der ersten Generation (Erlotinib, Gefitinib) unterscheidet. Diese Inhibitoren der so genannten zweiten Generation binden irreversibel an den Rezeptor und nicht reversibel, wie z.B. Tarceva® oder Iressa™. NSCLC-Patienten, die aufgrund der T790M-Mutation nicht mehr auf Medikamente der ersten Generation ansprechen, können folglich mit einem EGFR-Inhibitor der zweiten Generation (z.B. BIBW2992/Tovok von Boehringer Ingelheim) weiterbehandelt werden. Wie klinische Studien zeigen, sind diese Inhibitoren ebenfalls hochspezifisch und wirksam, so dass das Wachstum und Überleben der Tumorzellen verlangsamt oder verhindert werden kann.

Im Sinne einer individualisierten Medizin, also um jedem einzelnen Patienten mit NSCLC die für ihn am besten geeignete Therapie bieten zu können, ist es notwendig und sinnvoll, das Tumorgewebe auf den EGFR-Status zu testen. Somit kann denjenigen Patienten, die eine aktivierende Mutation im EGF-Rezeptor aufweisen, eine Behandlung mit den entsprechenden EGFR-Inhibitoren angeboten werden.

Um einen hohen Grad an Sensitivität zu erreichen, werden im Rahmen der vorliegenden Erfindung verschiedene Optimierungsschritte realisiert, auf die am Beispiel der Deletion ΔE746-A750 im Nachfolgenden veranschaulichend eingegangen wird.

Zu Beginn der Planung bzw. Durchführung des Mutationsnachweises werden zunächst spezifische Primer und Hybridisierungssonden generiert bzw. bereitgestellt. Derartige Primer und Hybridisierungssonden können beispielsweise von der TIB MOLBIOL GmbH, Berlin, bezogen werden.

Die spezifischen Primer amplifizieren bevorzugt den Abschnitt der DNA, in dem die nachzuweisende Mutation lokalisiert ist. Zusätzlich werden in der PCR-Reaktion gemäß einer bevorzugten Ausführungsform zwei voneinander verschiedene Hybridisierungssonden (Sensorsonde einerseits und Ankersonde andererseits) eingesetzt. Diese umfassen, wie die Primer auch, mehrere Nukleotide, die innerhalb des entstehenden PCR-Produktes hybridisieren. Dabei binden beide Sonden in räumlicher Nähe zueinander. An den einander zugewandten Enden sind die Sonden jeweils mit einem Fluoreszenzfarbstoff markiert und können über FRET (Fluoreszenz-Resonanz-Energie-Transfer) miteinander interagieren (vgl. Fig. 2). Die Sonden binden sowohl in bzw. an der Wildtypsequenz (wildtypischer DNA-Einzelstrang bzw. wildtypischer DNA-Strang) als auch an der Mutationssequenz (mutierter DNA-Einzelstrang bzw. mutierter DNA-Strang), wobei eine der Sonden (Ankersonde) im nicht veränderten Sequenzbereich und die andere Sonde (Sensorsonde) in Bezug auf die Wildtyp-DNA in dem zu der vermuteten Mutation korrespondierenden Bereich bindet, während in Bezug auf die Mutation als solche an der mutierten DNA zumindest im Wesentlichen keine Bindung erfolgt, so dass hinsichtlich der mutierten DNA eine nur abschnittsweise Bindung der Sensorsonde erfolgt, nämlich an einem unmittelbar an die Mutationsstelle angrenzenden Bereich der mutierten DNA. Es resultiert somit eine nur abschnittsweise Bindung der Sensorsonde an die mutierte DNA. Wie zuvor angeführt, bindet die Sensorsonde an der nichtmutierten und somit wildtypischen DNA spezifisch.

In beiden Fällen überträgt die Sensorsonde unter bzw. nach energetischer Anregung mit elektromagnetischer Strahlung einer entsprechenden Wellenlänge, wie sie durch die Xenon-Lampe des LightCyclers® erzeugt werden kann, ihre Energie durch FRET auf die Ankersonde. Diese wird hierdurch ebenfalls angeregt und emittiert ein Fluoreszenzsignal, welches vom Gerät detektiert werden kann. Die Messung kann am Ende der *Annealing*-Phase jedes PCR-Zyklus, der Phase, in welcher die Primer und Sonden an den DNA-Strang binden, durchgeführt werden.

Im Anschluss an die eigentliche PCR schließt sich eine Schmelzkurvenanalyse an. Hier werden die Reaktionsansätze langsam beispielsweise auf 95 °C erhitzt. An dem Punkt, an dem sich 50 % der Sensorsonden von den entstandenen PCR-Produkten gelöst haben, fällt die Fluoreszenz drastisch ab. Im Falle der spezifischen Bindung der Sonde an den nichtmutierten Strang erfolgt die Dehybridisierung bzw. Ablösung der Sensorsonde bei einer höheren Temperatur im Vergleich zu einer Bindung an Mutationssequenzen. Zusätzlich zur Abnahme des Fluoreszenzsignals mit steigender Temperatur wird insbesondere unter Verwendung des LightCycler® 480 die erste mathematische Ableitung der Schnittkurve berechnet, so dass der Wendepunkt der gemessenen Fluoreszenzabnahme als Kurve dargestellt wird. Der höchste Punkt bzw. das Maximum der Kurve auf Basis der ersten Ableitung entspricht der spezifischen Schmelztemperatur. Diese Darstellung dient der besseren visuellen Auswertung. Proben, die sowohl ein wildtypisches als auch ein mutiertes Allel besitzen, zeigen folglich zwei verschiedene Schmelzprofile, die entsprechend als "Doppelpeak" bzw. Doppelmaximum (Ausbildung zweier unterschiedlicher Maxima) zu sehen sind (vgl. Fig. 5).

Nach etwaiger Optimierung der eingesetzten DNA-Menge, der *Annealing-*Temperatur, der Primerkompatibilität und/oder -konzentration sowie der Sondenkonzentration zeigt der Nachweis der Mutation unter Bedingungen des Standes der Technik im Rahmen einer üblichen und nichtasymmetrischen PCR genauso, wie im Falle einer Sequenzierung, eine Sensitivität von lediglich 20 bis 25 %.

Um die Nachweisgrenze zu steigern, wird im Rahmen der vorliegenden Erfindung in zweckgerichteter Weise eine so genannte asymmetrische PCR angewendet bzw. durchgeführt. Im Gegensatz zur symmetrischen PCR ist hierbei das Verhältnis beider Primer zueinander nicht gleich. Es wird die Konzentration desjenigen Primers in dem PCR-Ansatz erhöht, welcher an denjenigen DNA-Strang bindet, an dem auch die Sonden hybridisieren (Sondenstrang). Somit wird dieser Strang bevorzugt vervielfältigt und die eingesetzten Sonden haben damit eine höhere Wahrscheinlichkeit, an diesen Strang zu binden (vgl. Fig. 3). Auf diese Weise kann auch die Sensitivität des Mutationsnachweises erhöht werden.

Zur weiteren Steigerung der Sensitivität ist es erfindungsgemäß zudem vorgesehen, die Amplifikation des Wildtyp-DNA-Strangs und/oder die Bindung der Sensorsonde an den Wildtyp-DNA-Strang zu verringern bzw. zu inhibieren, so dass nur bzw. vorrangig der mutierte DNA-Strang vervielfältigt wird und die eingesetzten Sonden somit gewissermaßen konkurrenzlos mit der mutierten DNA interagieren können. Mit Hilfe dieser erfindungsgemäßen Technik können in völlig überraschender Weise selbst kleinste Mengen mutierten Materials nachgewiesen werden. Diese spezifische Hemmung des Wildtyps kann z. B. mit einem Blockierungsmittel, insbesondere in Form einer so genannten *Locked Nucleic Acid* (LNA™ von der Firma Exiqon), welche auch *"Clamp"* genannt wird, durchgeführt werden (vgl. Fig. 4). Diese *"Clamps"* sind biochemisch so modifiziert, dass sie eine sehr hohe Bindungsstärke gegenüber dem Wildtypstrang aufweisen und erst bei sehr hohen Temperaturen abschmelzen.

Während der Schmelzkurvenanalyse wird die Abnahme der Fluoreszenz gemessen. Ist in der Probe eine Mutation nachzuweisen, ist die entsprechende Kurve sichtbar; sofern kein mutiertes Material vorhanden ist, ist keine bzw. eine minimale Wildtyp-Kurve zu sehen, da aufgrund der fehlenden Amplifikation und Sondenbindung des Wildtyps kein Signal gemessen werden kann (vgl. Fig. 6).

Um bestätigen zu können, dass die PCR-Reaktion erfolgreich durchgeführt wurde und um Heterozygotien nachweisen zu können, sollte auch ein Vergleichsansatz ohne Blockierungsmittel mitgeführt bzw. durchgeführt werden. In dieser Reaktion sollte zumindest der Wildtyp sichtbar sein.

Die erfindungsgemäße Kombination der oben genannten Maßnahmen ermöglicht Mutationsnachweise mit äußerst hoher Sensitivität. Im genannten Beispiel des Nachweises der ΔE746-A750-Mutation kann eine Sensitivität von 0,0005 % mutierter DNA in einem Wildtyp-Gemisch realisiert werden. Somit können kleinste Anteile mutierter DNA, beispielsweise eines Tumorgewebes, in einer Probe nachgewiesen werden. Auch eignet sich das erfindungsgemäße Verfahren, um Verlaufskontrollen im peripheren Blut oder anderen Körperflüssigkeiten durchzuführen. Bei vielen Patienten mit soliden Tumoren, wie z. B. NSCLC-Patienten, wird meist nur einmal Tumormaterial gewonnen, z. B. bei der Erstdiagnose oder einer Operation. Somit ist das *Monitoring* bzw. die Beobachtung des Erkrankungsverlaufs auf molekularer Ebene meist nicht möglich. Mit dem erfindungsgemäßen hochsensitiven Nachweisverfahren, welches auch in Kombination mit sensitiven Aufreinigungsmethoden durchgeführt werden kann, können sogar zirkulierende Tumorzellen in einer Vollblutprobe nachgewiesen werden. Es wurde beispielsweise beschrieben, dass Patienten mit NSCLC etwa 100 bis 200 zirkulierende Tumorzellen pro 1 ml Blut aufweisen. Geht man von etwa 10.000 kernhaltigen Zellen pro µl aus, bedeutet das einen Gehalt von etwa 0,001 % Tumorzellen. Mit der erfindungsgemäßen *Real-Time*-PCR-Methode, die auch ein sensitives Aufreinigungsverfahren der Tumorzellen umfassen kann, ist dieser Nachweis und damit eine Verlaufskontrolle bei Patienten vor bzw. unter Therapie möglich. Die vorliegende Erfindung stellt somit einen wichtigen Schritt in Richtung einer individualisierten und damit hochspezifischen Therapie dar.

Weitere Ausgestaltungen, Abwandlungen und Variationen der vorliegenden Erfindung sind für den Fachmann beim Lesen der Beschreibung ohne weiteres erkennbar und realisierbar, ohne dass er dabei den Rahmen der vorliegenden Erfindung verlässt.

Das nachfolgende Ausführungsbeispiel dient lediglich zur Veranschaulichung der vorliegenden Erfindung, ohne die vorliegende Erfindung jedoch hierauf zu beschränken.

### AUSFÜHRUNGSBEISPIEL:

### EGFR Deletion Exon 19-Mutationsnachweis am LC480:

Nachweis verschiedener Deletionen in Exon 19 des EGF Rezeptors in genomischer DNA mittels Schmelzkurvenanalyse am Light Cycler 480.

### Zusätzliche Referenzunterlagen:

- Datenblatt bzw. Data Sheet "LightCycler 480 Probes Master" von F. Hoffmann-La Roche Ltd.;
- Datenblatt bzw. Data Sheet "PureLink Genomic DNA Mini Kit" von Invitrogen Corp.

### Material:

- *LightCycler*® *480 Probes Master Kit* von F. Hoffmann-La Roche Ltd. (Best.-Nr. 04887301001);
- Primer und Sonden von TIB MOLBIOL GmbH, Berlin:
   Primer: Ex 19 S (Prod.-Nr. 1126164, vlg. Sequenzprotokoll II, Sequence Listing, SEQ ID NO. 2) und Ex 19 R (Prod.-Nr. 1126166, vlg. Sequenzprotokoll II, Sequence Listing, SEQ ID NO. 3);
   Sonden: Anchor Ex 19 (Prod.-Nr. 1131500, vlg. Sequenzprotokoll II, Sequence Listing, SEQ ID NO. 5) und Sensor ins (wildtypspezifische Sensorsonde, vlg. Sequenzprotokoll II, Sequence Listing, SEQ ID NO. 4) und LNA: clamp wt (Prod.-Nr. 1137307, vlg. Sequenzprotokoll II, Sequence Listing, SEQ ID NO. 6);
   - genomische DNA , z. B. aus peripherem Blut (Citrat);
      (20 - 90 µg/ml Ausgangskonzentration);
      isoliert mit "PureLink Genomic DNA Mini Kit" von Invitrogen Corp. (Best.-Nr. K1820-01);
   - Kontroll-DNA: z. B. A431 (EGFR Wildtyp) und HCC-827 (EGFR Deletion in Exon 19);
   - DNA-Vektoren: Plasmid MS658 (pcDNA3.1/V5-His-TOPO-EGFR_Exon19_Del. 746-750),
   - 96-Well-Platten von F. Hoffmann-La Roche Ltd., weiß (*LightCycler 480-Multiwell-Plate 96,* Best.-Nr. 04729692001)

### Geräte:

- Zentrifuge mit Einsatz für 96-well-Platten (z.B. Multifuge 3SR+ von Thermo Fisher Scientific Inc.);
- LightCycler 480 von F. Hoffmann-La Roche Ltd. mit 96-well-Block.

### Beschreibung des Ablaufs:

### Auftauen der benötigten Kit-Komponenten, der Primer und der Sonden auf Eis (Sonden und 480-Probes-Master im Dunkeln auftauen)

### Pipettieren des Primer/Sonden-Mixes auf Eis (ohne LAN):

| | | |
|---|---|---|
| Ex 19 S: | 0,10 µl (2 pmol) | 20 pmol/µl |
| + Ex 19 R: | 0,10 µl (2 pmol) | 20 pmol/µl |
| + Anchor Ex 19: | 0,15 µl (3 pmol) | 20 pmol/µl |
| + Sensor ins: | 0,15 µl (3 pmol) | 20 pmol/µl |
| + PCR-reines Wasser: | 1,50 µl | |
| Gesamtvolumen: 2 µl | | |

### Pipettieren des Primer/Sonden-Mixes auf Eis (mit LNA):

| | | |
|---|---|---|
| Ex 19 S: | 0,10 µl (2 pmol) | 20 pmol/µl |
| + Ex 19 R: | 0,10 µl (2 pmol) | 20 pmol/µl |
| + Anchor Ex 19: | 0,15 µl (3 pmol) | 20 pmol/µl |
| + Sensor ins: | 0,15 µl (3 pmol) | 20 pmol/µl |
| + Clamp wt: | 0,30 µl (6 pmol) | 20 pmol/µl |
| + PCR-reines Wasser: | 1,20 µl | |
| Gesamtvolumen: 2 µl | | |

### Pipettieren des Mastermixes:

| | |
|---|---|
| Primer/Sonden-Mix gemäß obigen Ausführungen: | 2 µl |
| + 480-Probes-Master: | 10 µl |
| + PCR-reines Wasser: | 7,5 µl |
| Gesamtvolumen: | 19,5 µl |

In eine weiße 96-Well-Platte werden 19,5 µl Mastermix und 0,5 ng DNA in 0,5 - 8 µl Gesamtvolumen pipettiert. Liegt die DNA in mehr als 0,5 µl gelöst vor, wird das PCR-reine Wasser aus Punkt 4 entsprechend reduziert. Als Einzelwerte werden immer folgende Kontrollen jeweils einmal mit LNA und einmal ohne LNA mitgeführt: Wasserkontrolle ohne DNA, Negativkontrolle (Wildtyp, z. B. A431), Positivkontrolle (Mutante, z. B. HCC-827) und eine zweite Positivkontrolle (Plasmid MS658).

Patientenproben werden als Einzelwert ohne LNA, als Doppelwert mit LNA mitgeführt.
Abdichten der Platte mit Folie und Zentrifugation: 1500 g, 2 min bei RT

### Platte in LightCycler® 480 einlegen und Programm starten:

| Filter-Kombinationen: | Schmelzfaktor: | Quantfaktor: | Max. Integrationszeit: |
|---|---|---|---|
| 483-640 | 1,2 | 5 | 2 Sekunden |

| Ablaufprotokoll: | Temperatur | Zeit | Zyklen |
|---|---|---|---|
| 1. Präinkubation: | 95 °C | 00:10:00 | 1 |
| 2. Amplifkation: | 95 °C | 00:00:10 | 40 |
| | 60 °C | 00:00:10 | |
| | 72 °C | 00:00:10 | |
| 3. Schmelzkurve: | 95 °C | 00:01:00 | 1 |
| | 40 °C | 00:02:00 | |
| | 95 °C | kontinuierlich | |
| 4. Abkühlung | 4 °C | kontinuierlich | |

### Erwartetes Ergebnis der Schmelzkurvenanalyse:

| Ergebnis: | Schmelzkurventemperaturen |
|---|---|
| Sonden binden spezifisch an Wildtyp | |
| homozygote Mutante (HCC 827): | eine Schmelzkurve zwischen 45 und 60 °C |
| homozygote Mutante (MS 658) | eine Schmelzkurve zwischen 47 und 55 °C |
| homozygoter Wildtyp: | eine Schmelzkurve zwischen 55 und 70 °C |
| Heterozygotie: | zwei Schmelzkurven; s.o. |

Hinweise und Anmerkungen: Der fertige PCR-Mix ist zumindest 24 Stunden bei RT im Dunkeln stabil.

| | |
|---|---|
| Abkürzung: | RT = Raumtemperatur |

## Patentansprüche

1. Verfahren zur Detektion mindestens einer Mutation in einem Gen, vorzugsweise in einem Gen, welches für ein mit einer Tumor- und/oder Krebserkrankung in Verbindung stehendes Protein kodiert, wobei das die Mutation aufweisende Gen (Mutationsgen) zusammen mit weiteren, für das Protein kodierenden, jedoch keine Mutation aufweisenden Genen (Wildtypgenen) vorliegt,
wobei das Verfahren mittels einer asymmetrischen Polymerase-Kettenreaktion (*Polymerase Chain Reaction* bzw. PCR) unter Verwendung mindestens einer detektierbaren Hybridisierungssonde (Sensorsonde), welche an dem keine Mutation aufweisenden Gen (Wildtypgen) und dem die Mutation aufweisenden Gen (Mutationsgen) zu binden imstande ist, mindestens einer hiervon verschiedenen zweiten Hybridisierungssonde (Ankersonde) und mindestens eines die Anbindung der Sensorsonde an die Wildtypgene inhibierenden wildtypspezifischen Blockierungsmittels durchgeführt wird und wobei zum Nachweis und/oder zur Detektion der Mutation im Anschluss an die Polymerase-Kettenreaktion eine Schmelzkurve aufgenommen wird und/oder eine Schmelzkurvenanalyse durchgeführt wird, wobei die Abspaltung und/oder Dehybridisierung der Sensorsonde von dem jeweiligen DNA-Einzelstrang des Mutationsgens und/oder des Wildtypgens erfasst wird und wobei über den oder die Schmelzpunkte und/oder Schmelzpunktbereiche der Schmelzkurve auf das Vorliegen einer Mutation geschlossen wird und im Falle des Vorliegens einer Mutation die Art der Mutation bestimmt wird,
wobei die Hybridisierungssonde (Sensorsonde) eine höhere Spezifität und/oder Bindungsaffinität und/oder Selektivität zu dem keine Mutation aufweisenden Gen (Wildtypgen) als zu dem die Mutation aufweisenden Gen (Mutationsgen) aufweist, wobei die Hybridisierungssonde (Sensorsonde) vollständig an dem DNA-Einzelstrang (wt-Sondenstrang) des Wildtypgens zu binden imstande ist und wobei die Hybridisierungssonde (Sensorsonde) an der Position der Mutation an dem die Mutation aufweisenden DNA-Einzelstrang (mt-Sondenstrang) des Mutationsgens nicht zu binden imstande ist,
wobei die zweite Hybridisierungssonde (Ankersonde) auf demselben DNA-Einzelstrang wie die Hybridisierungssonde (Sensorsonde) zu binden imstande ist und wobei die Hybridisierungssonde (Sensorsonde) einerseits und die zweite Hybridisierungssonde (Ankersonde) andererseits ein FRET-Paar auszubilden imstande sind,
wobei als Blockierungsmittel ein Nukleinsäure-Analogon in Form einer *Locked Nucleic Acid* (LNA) eingesetzt wird und wobei das Blockierungsmittel ausgewählt wird derart, dass das Blockierungsmittel eine höhere Spezifität und/oder Bindungsaffinität und/oder Selektivität in Bezug auf den Bereich des DNA-Einzelstrang des Wildtypgens, welcher zu dem die Mutation aufweisenden Genabschnitt des Mutationsgens korrespondiert, gegenüber dem entsprechenden Mutationsgen aufweist, und
wobei die asymmetrische Polymerase-Kettenreaktion in Gegenwart von Primern durchgeführt wird, wobei ein erster Primer spezifisch an dem DNA-Einzelstrang des Mutationsgens (mt-Sondenstrang) bindet, an welchen die Sensorsonde bindet, und wobei ein zweiter Primer spezifisch an dem zum Sondenstrang komplementären DNA-Einzelstrang des Mutationsgens (mt-Komplementärstrang) bindet, wobei die Konzentration des ersten Primers größer ist als die Konzentration des zweiten Primers.

2. Verfahren nach Anspruch 1, **dadurch gekennzeichnet, dass** es sich bei der Mutation um eine Leserastermutation, insbesondere um eine Deletion und/oder Insertion, und/oder um eine Punktmutation, handelt und/oder dass es sich bei der Mutation um eine Deletion und/oder Insertion, handelt.

3. Verfahren nach Anspruch 1 oder 2, **dadurch gekennzeichnet,**
**dass** die Sensorsonde derart ausgewählt wird, dass die Sensorsonde nur abschnittsweise mit dem DNA-Einzelstrang (mt-Sondenstrang) des Mutationsgens zu interagieren und/oder wechselzuwirken, insbesondere hieran zu binden, imstande ist, insbesondere wobei es sich bei dem Abschnitt um einen Randabschnitt und/oder randständigen Bereich und/oder um einen Endabschnitt und/oder endständigen Bereich der Nukleotidsequenz der Sensorsonde handelt; und/oder
**dass** die Sensorsonde mit dem sich an die Position und/oder Stelle der Mutation anschließenden Abschnitt und/oder Bereich des die Mutation aufweisenden DNA-Einzelstrangs (mt-Sondenstrang) des Mutationsgens zu interagieren und/oder wechselzuwirken, insbesondere hieran zu binden, imstande ist.

4. Verfahren nach einem der vorangehenden Ansprüche, **dadurch gekennzeichnet, dass** die Sensorsonde derart ausgewählt wird, dass die Sensorsonde, bezogen auf den die Mutation aufweisenden DNA-Einzelstrang (mt-Sondenstrang) des Mutationsgens, mindestens einen Bindungsbereich und/oder -abschnitt, vorzugsweise einen einzigen Bindungsbereich und/oder -abschnitt, und mindestens einen Nichtbindungsbereich- und/oder -abschnitt, vorzugsweise einen einzigen Nichtbindungsbereich- und/oder -abschnitt, aufweist, insbesondere wobei die Anzahl der Nukleotide des Nichtbindungsbereichs zumindest im Wesentlichen der Anzahl der die Mutation ausbildenden Nukleotide entspricht, und/oder insbesondere wobei die Anzahl der Nukleotide des Bindungsbereichs im Bereich von 1 bis 30, insbesondere 3 bis 21, vorzugsweise 6 bis 12, beträgt.

5. Verfahren nach einem der vorangehenden Ansprüche, **dadurch gekennzeichnet,**
**dass** die Sensorsonde ausgewählt wird derart, dass die Sensorsonde mindestens 3, insbesondere mindestens 6, vorzugsweise mindestens 9 Nukleotide aufweist und/oder dass die Anzahl der Nukleotide der Sensorsonde im Bereich von 3 bis 60, insbesondere 6 bis 30, vorzugsweise 9 bis 21, beträgt; und/oder
**dass** die Sensorsonde derart ausgewählt wird, dass das Verhältnis von bindenden Nukleotiden zu nichtbindenden Nukleotiden der Sensorsonde, bezogen auf den die Mutation aufweisenden DNA-Einzelstrang (mt-Sondenstrang) des Mutationsgens, im Bereich von 10 : 1 bis 1 : 10, insbesondere 6 : 1 bis 1 : 6, vorzugsweise 4 : 1 bis 1 : 4, liegt; und/oder dass die Sensorsonde derart ausgewählt wird, dass die Sensorsonde mit höchstens 60 %, insbesondere höchstens 50 %, vorzugsweise höchstens 40 %, der die Sensorsonde ausbildenden Nukleotide mit dem die Mutation aufweisenden DNA-Einzelstrang (mt-Sondenstrang) des Mutationsgens nicht zu interagieren und/oder nicht wechselzuwirken, insbesondere nicht hieran zu binden, imstande ist, bezogen auf die Gesamtzahl der Nukleotide der Sensorsonde; und/oder
**dass** die Sensorsonde derart ausgewählt wird, dass die Anzahl der Nukleotide der Sensorsonde, welche nicht mit dem die Mutation aufweisenden DNA-Einzelstrang (mt-Sondenstrang) des Mutationsgens zu interagieren und/oder wechselzuwirken, insbesondere nicht hieran zu binden, imstande sind, im Bereich von 1 bis 15, insbesondere 2 bis 12, vorzugsweise 3 bis 9, bevorzugt 3 bis 6, liegt.

6. Verfahren nach einem der vorangehenden Ansprüche, **dadurch gekennzeichnet,**
**dass** die Sensorsonde derart ausgewählt wird, dass eine wärmeinduzierte Ablösung der Sensorsonde von dem DNA-Einzelstrang des Mutationsgens (mt-Sondenstrang) bei niedrigeren Temperaturen erfolgt als von dem entsprechenden DNA-Einzelstrang des Wildtypgens (wt-Sondenstrang); und/oder
**dass** die Sensorsonde derart ausgewählt wird, dass die Sensorsonde im Falle der Wechselwirkung und/oder Interaktion und/oder Bindung an den DNA-Einzelstrang des Mutationsgens (mt-Sondenstrang) und/oder an den entsprechenden DNA-Einzelstrang des Wildtypgens (wt-Sondenstrang) ein detektierbares und/oder messbares Signal, insbesondere ein Fluoreszenzsignal, abzugeben imstande ist; und/oder
**dass** die Sensorsonde derart ausgewählt wird, dass die Sensorsonde im Falle einer insbesondere wärmeinduzierten Ablösung von dem DNA-Einzelstrang des Mutationsgens (mt-Sondenstrang) und/oder von dem korrespondierenden DNA-Einzelstrang des Wildtypgens (wt-Sondenstrang) kein oder aber zumindest ein vermindertes detektierbares und/oder messbares Signal, insbesondere kein oder aber zumindest ein vermindertes Fluoreszenzsignal, abzugeben imstande ist.

7. Verfahren nach einem der vorangehenden Ansprüche, **dadurch gekennzeichnet, dass** die Ankersonde ausgewählt wird derart, dass die Ankersonde in einem Abstand von 1 bis 5 bp (Basenpaare) zu der Sensorsonde zu binden imstande ist.

8. Verfahren nach einem der vorangehenden Ansprüche, **dadurch gekennzeichnet,**
**dass** es sich bei der Tumor- und/oder Krebserkrankung um ein Lungenkarzinom, insbesondere um ein nichtkleinzelliges Lungenkarzinom (*Non Small Cell Lung Cancer* bzw. NSCLC) und/oder um ein kleinzelliges Lungenkarzinom (*Small Cell Lung Cancer* bzw. SCLC), vorzugsweise um ein nichtkleinzelliges Lungenkarzinom (NSCLC), handelt und/oder dass es sich bei dem Protein um ein insbesondere humanes und/oder ein das Zellwachstum und/oder die Proliferation von Zellen regulierendes und/oder induzierendes Protein handelt und/oder dass es sich bei dem Protein um einen insbesondere transmembranen Rezeptor für Wachstumsfaktoren, insbesondere mit intrinsischer Tyrosinkinase-Aktivität, handelt und/oder dass es sich bei dem Protein um einen Epidermalen-Wachstumsfaktor-Rezeptor (*Epidermal-Growth-Fact*o*r*-Rezeptor bzw. EGF-Rezeptor) handelt; und/oder
**dass** die Mutation in Exon 18, Exon 19, Exon 20 oder Exon 21, vorzugsweise in Exon 19, des Epidermalen-Wachstumsfaktor-Rezeptors (*Epidermal-Growth-Fact*o*r*-Rezeptor bzw. EGF-Rezeptor) lokalisiert ist; und/oder
**dass** es sich bei der Mutation um eine Mutation, insbesondere Deletion, in Exon 19 und/oder im Bereich der Aminosäureposition 746 und/oder 747 des Epidermalen-Wachstumsfaktor-Rezeptors (*Epidermal-Growth-Fact*o*r*-Rezeptor bzw. EGF-Rezeptor) handelt.

9. Verfahren nach einem der vorangehenden Ansprüche, **dadurch gekennzeichnet, dass** es sich bei der Mutation um eine Deletion, insbesondere in Exon 19 des Epidermalen-Wachstumsfaktor-Rezeptors (*Epidermal-Growth-Fact*o*r*-Rezeptor bzw. EGF-Rezeptor), handelt, wobei die Deletion ausgewählt wird aus der Gruppe der Deletionen ΔE746-A750, ΔE746-T751, ΔE746-A750 (ins RP), ΔE746-T751 (ins A/I), ΔE746-T751 (ins VA), E746-S752 (ins A/V), ΔL747-E749 (A750P), ΔL747-A750 (ins P), ΔL747-T751, ΔL747-T751 (ins P/S), ΔL747-S752, ΔL747-S752 (E746V), ΔL747-S752 (P746V), ΔL747-S752 (ins Q), ΔL747-P753, ΔL747-P753 (ins S) und ΔS752-1759.

10. Verfahren nach Anspruch 9, **dadurch gekennzeichnet, dass** die Tumor- und/oder Krebserkrankung im Zusammenhang mit einer Mutation, insbesondere Translokation, in dem für das Fusionsprotein EML4-ALK kodierenden Gen steht und/oder dass die Mutation eine Mutation, insbesondere Translokation, in dem für das Fusionsprotein EML4-ALK kodierenden Gen darstellt.

11. Verfahren nach Anspruch 9, **dadurch gekennzeichnet, dass** es sich bei der Tumor- und/oder Krebserkrankung um eine Leukämie, insbesondere um eine akute myeloische Leukämie (AML), handelt und/oder dass die Tumor- und/oder Krebserkrankung im Zusammenhang mit einer Mutation, insbesondere Insertion, in dem für die Rezeptortyrosinkinase FLT3 kodierenden Gen steht und/oder dass die Mutation eine Mutation, insbesondere Insertion, in dem für die Rezeptortyrosinkinase FLT3 kodierenden Gen darstellt.

12. Verfahren nach einem der vorangehenden Ansprüche, **dadurch gekennzeichnet, dass** die asymmetrische Polymerase-Kettenreaktion (*Polymerase Chain Reaction* bzw. PCR) derart ausgeführt wird, dass eine insbesondere selektive Vermehrung und/oder Amplifizierung derjenigen DNA-Einzelstränge des Mutationsgens (mt-Sondenstränge) und des Wildtypgens (wt-Sondenstränge), an welchen die Sensorsonde zu interagieren und/oder wechselzuwirken, insbesondere hieran zu binden, imstande ist, stattfindet,
insbesondere wobei die asymmetrische PCR in Gegenwart von Primern, insbesondere in Form von Oligonukleotiden, durchgeführt wird und/oder insbesondere wobei eine Amplifizierung des die Mutation aufweisenden Genabschnitts des Mutationsgens erfolgt und/oder insbesondere wobei eine Amplifizierung des zu dem die Mutation aufweisenden Genabschnitt des Mutationsgens korrespondierenden Genabschnitts der Wildtypgene erfolgt und/oder insbesondere wobei der erste Primer zumindest im Wesentlichen spezifisch an dem DNA-Einzelstrang des Mutationsgens (mt-Sondenstrang) bindet, mit welchem die Sensorsonde zu interagieren imstande ist, und/oder dass der zweite Primer zumindest im Wesentlichen spezifisch an dem zum Sondenstrang komplementären DNA-Einzelstrang des Mutationsgens (mt-Komplementärstrang) bindet und/oder insbesondere wobei der erste Primer und der zweite Primer ausgewählt werden derart, dass die Menge und/oder die Konzentration des ersten Primers, insbesondere bezogen auf den PCR-Ansatz, größer ist als die Menge und/oder Konzentration des zweiten Primers, insbesondere so dass eine erhöhte und/oder verstärkte Amplifikation des mt-Sondenstrangs gegenüber dem Komplementärstrang erfolgt.

13. Verfahren nach einem oder mehreren der vorangehenden Ansprüche, **dadurch gekennzeichnet, dass** die Erfassung der Abspaltung und/oder Dehybridisierung, insbesondere der Sensorsonde von dem jeweiligen DNA-Einzelstrang des Mutationsgens und/oder des Wildtypgens photometrisch, insbesondere durch Messung der Fluoreszenz, erfolgt.

## Claims

1. A method for detecting at least one mutation in a gene, preferably in a gene that codes for a protein associated with a tumour- and/or cancerous disease, wherein the gene having the mutation (mutation gene) is present together with further genes (wild type genes) coding for the protein but not having a mutation,
wherein the method is carried out by means of an asymmetric polymerase chain reaction (PCR) with the use of at least one detectable hybridization probe (sensor probe), which is capable of binding to the gene not having a mutation (wild type gene) and to the gene having the mutation (mutation gene), of at least one second hybridization probe (anchor probe) different herefrom and of at least one wild-type-specific blocking agent which inhibits the binding of the sensor probe to the wild type genes, and wherein for evidence and/or for detection of the mutation following the polymerase chain reaction a melting curve is recorded and/or a melting curve analysis is carried out, wherein the cleavage and/or dehybridization of the sensor probe from the respective DNA single strand of the mutation gene and/or of the wild type gene is detected and wherein via the melting point or the melting points and/or melting point ranges of the melting curve a conclusion is drawn as to the presence of a mutation and in the case of the presence of a mutation, the nature of the mutation is determined,
wherein the hybridization probe (sensor probe) has a higher specificity and/or binding affinity and/or selectivity to the gene not having a mutation (wild type gene) than to the gene having the mutation (mutation gene), wherein the hybridization probe (sensor probe) is capable of binding completely to the DNA single strand (wt-probe strand) of the wild type gene and wherein the hybridization probe (sensor probe) is not capable of binding to the DNA single strand (mt-probe strand) having the mutation at the position of the mutation,
wherein the second hybridization probe (anchor probe) is capable of binding to the same DNA single strand as the hybridization probe (sensor probe) and wherein the hybridization probe (sensor probe) on the one hand and the second hybridization probe (anchor probe) on the other hand are capable of forming a FRET pair,
wherein a nucleic acid analogue in the form of a *Locked Nucleic Acid* (LNA) is used as blocking agent, and wherein the blocking agent is selected such that the blocking agent has a higher specificity and/or binding affinity and/or selectivity with respect to the region of the DNA single strand of the wild type gene, which corresponds to the gene segment of the mutation gene having the mutation, compared to the corresponding mutation gene, and
wherein the asymmetric polymerase chain reaction is carried out in the presence of primers, wherein a first primer binds specifically to the DNA single strand of the mutation gene (mt-probe strand), to which the sensor probe binds, and wherein a second primer binds specifically to the DNA single strand, complementary to the probe strand, of the mutation gene (mt-complementary strand), wherein the concentration of the first primer is greater than the concentration of the second primer.

2. The method according to claim 1,
**characterized in that** the mutation concerns a frameshift mutation, in particular a deletion and/or insertion, and/or a point mutation, and/or that the mutation concerns a deletion and/or insertion.

3. The method according to claim 1 or 2, **characterized in that** the sensor probe is selected such that the sensor probe is capable of only partially interacting and/or interplaying with the DNA single strand (mt-probe strand) of the mutation gene, in particular of binding hereto, in particular wherein the segment concerns an edge segment and/or marginal region and/or an end segment and/or terminal region of the nucleotide sequence of the sensor probe; and/or
that the sensor probe is capable of interacting and/or interplaying with the segment and/or region of the DNA single strand having the mutation (mt-probe strand) of the mutation gene following the position and/or site of the mutation, in particular of binding hereto.

4. The method according to one of the preceding claims,
**characterized in that** the sensor probe is selected such that the sensor probe, in relation to the DNA single strand having the mutation (mt-probe strand) of the mutation gene, has at least one binding region and/or -section, preferably a single binding region and/or -section, and at least one non-binding region and/or -section, preferably a single non-binding region and/or -section, in particular wherein the number of nucleotides of the non-binding region corresponds at least substantially to the number of nucleotides forming the mutation, and/or in particular wherein the number of nucleotides of the binding region is in the range of 1 to 30, in particular 3 to 21, preferably 6 to 12.

5. The method according to one of the preceding claims,
**characterized in**
**that** the sensor probe is selected such that the sensor probe has at least 3, in particular at least 6, preferably at least 9 nucleotides and/or that the number of nucleotides of the sensor probe is in the range of 3 to 60, in particular 6 to 30, preferably 9 to 21; and/or
**that** the sensor probe is selected such that the ratio of binding nucleotides to non-binding nucleotides of the sensor probe, in relation to the DNA single strand having the mutation (mt-probe strand) of the mutation gene lies in the range of 10:1 to 1:10, in particular 6:1 to 1:6, preferably 4:1 to 1:4; and/or
**that** the sensor probe is selected such that the sensor probe with at most 60%, in particular at most 50%, preferably at most 40%, of the nucleotides forming the sensor probe is not capable of interacting and/or interplaying with the DNA single strand having the mutation (mt-probe strand) of the mutation gene, in particular of binding hereto, in relation to the total number of nucleotides of the sensor probe; and/or
**that** the sensor probe is selected such that the number of nucleotides of the sensor probe which are not capable of interacting and/or interplaying with the DNA single strand having the mutation (mt-probe strand) of the mutation gene, in particular of binding hereto, lies in the range of 1 to 15, in particular 2 to 12, preferably 3 to 9, particularly 3 to 6.

6. The method according to one of the preceding claims,
**characterized in**
**that** the sensor probe is selected such that a heat-induced dissociation of the sensor probe from the DNA single strand of the mutation gene (mt-probe strand) takes place at lower temperatures than from the corresponding DNA single strand of the wild type gene (wt-probe strand); and/or
**that** the sensor probe is selected such that the sensor probe, in the case of the interplay and/or interaction and/or binding to the DNA single strand of the mutation gene (mt-probe strand) and/or to the corresponding DNA single strand of the wild type gene (wt-probe strand), is capable of emitting a detectable and/or measurable signal, in particular a fluorescence signal; and/or
**that** the sensor probe is selected such that the sensor probe, in the case of an in particular heat-induced dissociation from the DNA single strand of the mutation gene (mt-probe strand) and/or from the corresponding DNA single strand of the wild type gene (wt-probe strand) is capable of emitting no, or else at least a reduced, detectable and/or measurable signal, in particular no, or else at least a reduced, fluorescence signal.

7. The method according to one of the preceding claims,
**characterized in that** the anchor probe is selected such that the anchor probe is capable of binding at a distance of 1 to 5 bp (base pairs) to the sensor probe.

8. The method according to one of the preceding claims,
**characterized in**
**that** the tumour- and/or cancerous disease concerns a lung cancer, in particular a non-small cell lung cancer (*Non Small Cell Lung Cancer* or respectively NSCLC) and/or a small cell lung cancer (*Small Cell Lung Cancer* or respectively SCLC), preferably a non-small cell lung cancer (NSCLC), and/or that the protein concerns an in particular human protein and/or a protein regulating and/or inducing cell growth and/or the proliferation of cells, and/or that the protein concerns an in particular transmembrane receptor for growth factors, in particular with intrinsic tyrosine kinase activity, and/or that the protein concerns an epidermal growth factor receptor (*Epidermal-Growth-Factor* receptor or respectively EGF receptor); and/or
**that** the mutation is localized in exon 18, exon 19, exon 20 or exon 21, preferably in exon 19, of the epidermal growth factor receptor (*Epidermal-Growth-Factor* receptor or respectively EFG receptor); and/or that the mutation concerns a mutation, in particular deletion, in exon 19 and/or in the region of the amino acid position 746 and/or 747 of the epidermal growth factor receptor (*Epidermal-Growth-Factor* receptor or respectively EGF receptor).

9. The method according to one of the preceding claims,
**characterized in that** the mutation concerns a deletion, in particular in exon 19 of the epidermal growth factor receptor (*Epidermal-Growth-Factor* receptor or respectively EGF receptor), wherein the deletion is selected from the group of deletions ΔE746-A750, ΔE746-T751, ΔE746-A750 (ins RP), ΔE746-T751 (ins A/I), ΔE746-T751 (ins VA), ΔE746-S752 (ins A/V), ΔL747-E749 (A750P), ΔL747-A750 (ins P), ΔL747-T751, ΔL747-T751 (ins P/S), ΔL747-5752, ΔL747-S752 (E746V), ΔL747-S752 (P746V), ΔL747-S752 (ins Q), ΔL747-P753, ΔL747-P753 (ins S) and ΔS752-1759.

10. The method according to claim 9,
**characterized in that** the tumour- and/or cancerous disease is associated with a mutation, in particular translocation, in the gene coding for the fusion protein EML4-ALK and/or that the mutation represents a mutation, in particular translocation, in the gene coding for the fusion protein EML4-ALK.

11. The method according to claim 9,
**characterized in that** the tumour- and/or cancerous disease concerns a leukaemia, in particular an acute myeloid leukaemia (AML), and/or that the tumour- and/or cancerous disease is associated with a mutation, in particular insertion, in the gene coding for the receptor tyrosine kinase FLT3 and/or that the mutation represents a mutation, in particular insertion, in the gene coding for the receptor tyrosine kinase FLT3.

12. The method according to one of the preceding claims,
**characterized in that** the asymmetric polymerase chain reaction (*Polymerase Chain Reaction* or respectively PCR) is carried out such that an in particular selective increase and/or amplification occurs of those DNA single strands of the mutation gene (mt-probe strands) and of the wild type gene (wt-probe strands) with which the sensor probe is capable of interacting and/or interplaying, in particular of binding hereto,
in particular wherein the asymmetric PCR is carried out in the presence of primers, in particular in the form of oligonucleotides, and/or in particular wherein an amplification of the gene segment of the mutation gene, having the mutation, takes place, and/or in particular wherein an amplification takes place of the gene segment of the wild type gene, corresponding to the gene segment of the mutation gene, having the mutation, and/or in particular wherein the first primer binds at least substantially specifically to the DNA single strand of the mutation gene (mt-probe strand), with which the sensor probe is capable of interacting, and/or that the second primer binds at least substantially specifically to the DNA single strand of the mutation gene complementary to the probe strand (mt-complementary strand), and/or in particular wherein the first primer and the second primer are selected such that the amount and/or the concentration of the first primer, in particular in relation to the PCR mixture, is greater than the amount and/or concentration of the second primer, in particular so that an increased and/or intensified amplification of the mt-probe strand takes place compared to the complementary strand.

13. The method according to one or more of the preceding claims,
**characterized in that** the detection of the cleavage and/or dehybridization, in particular of the sensor probe from the respective DNA single strand of the mutation gene and/or of the wild type gene takes place photometrically, in particular by measurement of the fluorescence.

## Revendications

1. Procédé de détection d'au moins une mutation dans un gène, de préférence dans un gène qui code pour une protéine en liaison avec une pathologie tumorale et/ou cancéreuse, le gène présentant la mutation (gène mutant) étant présent avec d'autres gène (gène de type sauvage) codant pour la protéine mais ne présentant pas de mutation,
ce procédé étant réalisé au moyen d'une réaction en chaîne asymétrique de polymérase (PCR) en utilisant au moins une sonde d'hybridation détectable (sonde de détection) qui est en mesure de se lier au gène ne présentant pas de mutation (gène de type sauvage) et au gène présentant la mutation (gène mutant), d'au moins une seconde sonde d'hybridation (sonde d'ancrage) différente de celle-ci et d'au moins un agent de blocage (agent bloquant) spécifique au type sauvage inhibant la liaison de la sonde de détection au gène de type sauvage et dans lequel procédé, pour mettre en évidence et/ou pour détecter la mutation après la réaction en chaîne de polymérase, une courbe de fusion est relevée et/ou une analyse de la courbe de fusion est effectuée, la scission et/ou la déshybridation de la sonde de détection du brin individuel d'ADN respectif du gène mutant et/ou du gène de type sauvage est enregistrée et, par le biais du ou des points de fusion et/ou des plages de points de fusion de la courbe de fusion, on déduit la présence d'une mutation et, dans le cas de la présence d'une mutation, le type de mutation est défini,
la sonde d'hybridation (sonde de détection) présentant une spécificité et/ou affinité de liaison et/ou sélectivité par rapport au gène ne présentant pas de mutation (gène de type sauvage) plus élevée que par rapport au gène présentant la mutation (gène mutant), la sonde d'hybridation (sonde de détection) étant en mesure de se lier complètement au brin individuel d'ADN (brin de sonde wt) du gène de type sauvage et la sonde d'hybridation (sonde de détection) n'étant pas en mesure de se lier au niveau de la position de la mutation au brin individuel d'ADN présentant la mutation (brin de sonde mt) du gène mutant,
la seconde sonde d'hybridation (sonde d'ancrage) étant en mesure de se lier au même brin individuel d'ADN que la sonde d'hybridation (sonde de détection) et la sonde d'hybridation (sonde de détection) d'une part et la seconde sonde d'hybridation (sonde d'ancrage) étant d'autre part en mesure de constituer une paire FRET, sachant qu'on utilise comme agent de blocage un analogue d'acide nucléique sous forme d'un acide nucléique verrouillé (LNA) et que l'agent de blocage est sélectionné de manière à ce que l'agent de blocage présente une spécificité et/ou affinité de liaison et/ou sélectivité plus élevée au niveau de la plage du brin individuel d'ADN du gène de type sauvage qui correspond à la section génique présentant la mutation du gène mutant que le gène mutant correspondant, et
la réaction asymétrique en chaîne de polymérase étant réalisée en présence de primaires, un premier primaire se liant spécifiquement au brin individuel d'ADN du gène mutant (brin de sonde mt), auquel la sonde de détection se lie, et un second primaire se liant spécifiquement au brin individuel du gène mutant complémentaire du brin de sonde (brin complémentaires mt), la concentration du premier primaire étant supérieure à la concentration du second primaire.

2. Procédé selon la revendication 1,
**caractérisée en ce que** la mutation est une mutation à cadre de lecture, en particulier une délétion et/ou insertion et/ou une mutation ponctuelle, et/ou que la mutation est une délétion et/ou insertion.

3. Procédé selon la revendication 1 ou 2, **caractérisé**
**en ce que** la sonde de détection est sélectionnée de manière à ce que la sonde de détection ne soit en mesure d'interagir et/ou d' avoir une action conjointe que par endroits avec le brin individuel d'ADN (brin de sonde mt) du gène mutant, en particulier de s'y lier, sachant que la section est une section périphérique et/ou une zone située au bord et/ou une section terminale et/ou une section située au bout de la séquence de nucléotide de la sonde de détection; et/ou
**en ce que** la sonde de détection est en mesure d'interagir et/ou d'avoir une action conjointe avec la section et/ou zone suivant la position et/ou l'emplacement de la mutation du le brin individuel d'ADN présentant la mutation (brin de sonde mt) du gène mutant, en particulier de s'y lier.

4. Procédé selon une des revendications précédentes, **caractérisé**
**en ce que** la sonde de détection est sélectionnée de manière à ce que la sonde de détection, au niveau du brin individuel d'ADN présentant la mutation (brin de sonde mt) du gène mutant, présente au moins une zone et/ou section de liaison, de préférence une seule zone et/ou section de liaison et au moins une zone et/ou section de non-liaison, de préférence une seule zone et/ou section de non-liaison, le nombre de nucléotides de la section de non-liaison coïncidant en particulier du moins sensiblement au nombre de nucléotides constituant la mutation et/ou le nombre de nucléotides de la zone de liaison étant en particulier de l'ordre de 1 à 30, en particulier 3 à 21, de préférence 6 à 12.

5. Procédé selon une des revendications précédentes, **caractérisé**
**en ce que** la sonde de détection est sélectionnée de manière à ce que la sonde de détection présente au moins 3, en particulier au moins 6, de préférence au moins 9 nucléotides et/ou que le nombre de nucléotides de la sonde de détection soit de l'ordre de 3 à 60, en particulier 6 à 30, de préférence 9 à 21; et/ou en ce que la sonde de détection est sélectionnée de manière à ce que le rapport entre les nucléotides se liant et les nucléotides ne se liant pas de la sonde détection, au niveau du brin individuel d'ADN présentant la mutation (brin de sonde mt) du gène mutant soit de l'ordre de 10 : 1 à 1 : 10, en particulier 6 : 1 à 1 : 6, de préférence 4 : 1 à 1 : 4; et/ou
**en ce que** la sonde de détection est sélectionnée de manière à ce que la sonde de détection soit en mesure, avec au plus 60 %, en particulier au plus 50 %, de préférence au plus 40 % des nucléotides formant la sonde de détection, de ne pas interagir et/ou de ne pas agir conjointement avec le brin individuel d'ADN présentant la mutation (brin de sonde mt) du gène mutant, en particulier de s'y lier, en relation avec le nombre total de nucléotides de la sonde de détection; et/ou
**en ce que** la sonde de détection est sélectionnée de manière à ce que le nombre de nucléotides de la sonde de détection qui ne sont pas en mesure d'interagir et/ou d'agir conjointement avec le brin individuel d'ADN présentant la mutation (brin de sonde mt) du gène mutant, en particulier de ne pas s'y lier, soit de l'ordre de 1 à 15, en particulier 2 à 12, de préférence 3 à 9, très préférentiellement 3 à 6.

6. Procédé selon une des revendications précédentes, **caractérisé**
**en ce que** la sonde de détection est sélectionnée de manière à ce qu'un détachement induit thermiquement de la sonde de détection du brin individuel d'ADN du gène mutant (brin de sonde mt) ait lieu à des températures plus faibles que celles du brin individuel d'ADN correspondant du gène de type sauvage (brin de sonde wt); et/ou
**en ce que** la sonde de détection est sélectionnée de manière à ce que la sonde de détection, en cas d'interaction et/ou d'action conjointe et/ou de liaison au brin individuel d'ADN du gène mutant (brin de sonde mt) et/ou au brin individuel d'ADN correspondant du gène de type sauvage (brin de sonde wt) soit en mesure d'envoyer un signal détectable et/ou mesurable, en particulier un signal de fluorescence; et/ou
**en ce que** la sonde de détection est sélectionnée de manière à ce que la sonde de détection, en cas de détachement en particulier induit par la chaleur du brin individuel d'ADN du gène mutant (brin de sonde mt) et/ou du brin individuel d'ADN correspondant du gène de type sauvage (brin de sonde wt), soit en mesure d'envoyer pas de signal ou alors d'envoyer un signal détectable et/ou mesurable réduit, en particulier pas de signal de fluorescence ou alors au moins un signal réduit.

7. Procédé selon une des revendications précédentes,
**caractérisé en ce que** la sonde d'ancrage est sélectionnée de manière à ce que la sonde d'ancrage soit en mesure de se lier à une distance de 1 à 5 bp (paires de bases) à la sonde de détection.

8. Procédé selon une des revendications précédentes, **caractérisé**
**en ce que** la pathologie tumorale et/ou cancéreuse est un carcinome pulmonaire, en particulier un carcinome pulmonaire à cellules non petites (NSCLC) ou un carcinome pulmonaire à cellules petites (SCLC), de préférence un carcinome pulmonaire à cellules non petites (NSCLC), et/ou que la protéine est une protéine en particulier humaine et/ou régulant et/ou induisant la croissance cellulaire et/ou la prolifération de cellules et/ou que la protéine est un récepteur en particulier transmembranaire pour les facteurs de croissance, en particulier à activité intrinsèque de tyrosine kinase et/ou que la protéine est un récepteur de facteur de croissance épidermique (récepteur EGF); et/ou
**en ce que** la mutation en Exon 18, Exon 19, Exon 20 ou Exon 21, de préférence en Exon 19, du récepteur de facteur de croissance épidermique (récepteur EGF) est localisée; et/ou
**en ce que** la mutation est une mutation, en particulier une délétion, en Exon 19 et/ou au niveau de la position d'acide aminé 746 et/ou 747 du récepteur de facteur de croissance épidermique (récepteur EGF).

9. Procédé selon une des revendications précédentes,
**caractérisé en ce que** la mutation est une délétion, en particulier en Exon 19, du récepteur de facteur de croissance épidermique (récepteur EGF), la délétion étant sélectionnée dans le groupe composé des délétions dont ΔE746-A750, ΔE746-T751, ΔE746-A750 (dans RP), ΔE746-T751 (dans A/I), ΔE746-T751 (dans VA), ΔE746-S752 (dans A/V), ΔL747-E749 (A750P), ΔL747-A750 (dans P), ΔL747-T751, ΔL747- T751 (dans P/S), ΔL747-5752, ΔL747-S752 (E746V), ΔL747-S752 (P746V), ΔL747-S752 (dans Q), ΔL747-P753, ΔL747-P753 (dans S) et ΔS752-I759.

10. Procédé selon la revendication 9,
**caractérisé en ce que** la pathologie tumorale et/ou cancéreuse est en corrélation avec une mutation, en particulier une translocation, dans le gène codant pour la protéine de fusion EML4-ALK et/ou que la mutation constitue une mutation, en particulier une translocation, dans le gène codant pour la protéine de fusion EML4-ALK.

11. Procédé selon la revendication 9,
**caractérisé en ce que** la pathologie tumorale et/ou cancéreuse est une leucémie, en particulier une leucémie myélinique aiguë (AML), et/ou que la pathologie tumorale et/ou cancéreuse est en corrélation avec une mutation, en particulier une insertion, dans le gène codant pour la tyrosine kinase de récepteur FLT3 et/ou que la mutation représente une mutation, en particulier une insertion, dans le gène codant pour la tyrosine kinase de récepteur FLT3.

12. Procédé selon une des revendications précédentes,
**caractérisé en ce que** la réaction en chaîne asymétrique de polymérase (PCR) est réalisée de manière à ce qu'une multiplication et/ou amplification en particulier sélective des brins individuels d'ADN du gène mutant (brin de sonde mt) et du gène de type sauvage (brins de sonde wt) au niveau desquels la sonde de détection est en mesure d'interagir et/ou d'agir conjointement, en particulier de s'y lier, a lieu,
la PCR asymétrique étant réalisée en particulier en présence de primaires, en particulier sous forme de oligonucléotides et/ou en particulier une amplification de la section génique présentant la mutation du gène mutant se produisant et/ou une amplification de la section génique correspondant à la section génique du gène mutant du gène de type sauvage se produisant en particulier et/ou en particulier le premier primaire se liant du moins sensiblement spécifiquement au brin individuel d'ADN du gène mutant (brin de sonde mt) avec lequel la sonde de détection est en mesure d'interagir et/ou que le second primaire se lie du moins sensiblement spécifiquement au brin individuel d'ADN complémentaire au brin de sonde du gène mutant (brin complémentaire mt) et/ou le premier primaire et le second primaire étant en particulier sélectionnés de manière à ce que la quantité et/ou la concentration du premier primaire, en particulier relativement à l'approche PCR, soit supérieure à la quantité et/ou concentration du second primaire, en particulier de manière à ce qu'une amplification accrue et/ou intensifiée du brin de sonde mt par rapport au brin complémentaire se produise.

13. Procédé selon une ou plusieurs des revendications précédentes,
**caractérisé en ce que** l'enregistrement de la scission et/ou de la déshybridation, en particulier de la sonde de détection du brin individuel d'ADN respectif du gène mutant et/ou du gène de type sauvage a lieu par photométrie, en particulier par mesure de la fluorescence.


### SEQUENCE LISTING

<110> Universitaet Duisburg-Essen
<120> Verfahren zur Detektion von Mutationen, insbesondere Deletionen oder Insertionen
<130> 11.6105.6.do
<160> 6
<170> PatentIn version 3.3
<210> 1
   <211> 1210
   <212> PRT
   <213> Homo sapiens
<400> 1
<210> 2
   <211> 26
   <212> DNA
   <213> Artificial Sequence
<220>
   <223> PCR-Oligonukleotid, spezifisch fuer das korrespondierende Gen des humanen epidermalen Wachstumsfaktor-Rezeptors (EGFR)
<400> 2
   gtcttccttc tctctctgtc ataggg 26
<210> 3
   <211> 18
   <212> DNA
   <213> Artificial Sequence
<220>
   <223> PCR-Oligonukleotid, spezifisch fuer das korrespondierende Gen des humanen epidermalen Wachstumsfaktor-Rezeptors (EGFR)
<400> 3
   gggcctgagg ttcagagc 18
<210> 4
   <211> 21
   <212> DNA
   <213> Artificial Sequence
<220>
   <223> Real-Time PCR-Sonde, spezifisch fuer einen Abschnitt der wildtypischen DNA-Sequenz des EGFR-Gens im Bereich von Exon 19 (einsetzbar als Sensorsonde zur Detektion von Deletionen im Bereich von Exon 19).
<220>
   <221> misc_binding
   <222> (21)..(21)
   <223> FRET-Komponente, bevorzugterweise Fluoreszenzfarbstoff
<400> 4
   taattccttg atagcgacgg g 21
<210> 5
   <211> 30
   <212> DNA
   <213> Artificial Sequence
<220>
   <223> Real-Time PCR-Sonde, spezifisch fuer einen Abschnitt der wildtypischen DNA-Sequenz des EGFR-Gens im Bereich von Exon 19 (einsetzbar als Ankersonde zur Detektion von Deletionen im Bereich von Exon 19).
<220>
   <221> misc_binding
   <222> (1)..(1)
   <223> FRET-Komponente, bevorzugterweise Fluoreszenzfarbstoff
<400> 5
   attttaactt tctcaccttc tgggatccag 30
<210> 6
   <211> 13
   <212> DNA
   <213> Artificial Sequence
<220>
   <223> Blockierungsmittel nach dem Prinzip der locked nucleic acid, einsetzbar zur Real-Time PCR, spezifisch fuer einen Abschnitt auf dem wildtypischen EGFR-Gen
<220>
   <221> modified_base
   <222> (1)..(13)
   <223> Modifizierung zur Herstellung von Blockierungsmitteln nach dem Prinzip der locked nucleic acid, dazu werden das C2- und C4-Atom der Ribose/Desoxyribose ueber eine Sauerstoff-Methylengruppe miteinander verbunden.
<400> 6
   taattccttg ata 13